# EUROPEAN PATENT APPLICATION

(11) **EP 4 510 280 A1**
(43) Date of publication of application: **19.02.2025**
(21) Application number: 22939275.8
(22) Date of filing: 29.04.2022
(51) Int. Cl.: H01M 10/0567

(54) **SECONDARY BATTERY, BATTERY MODULE COMPRISING SAME, BATTERY PACK, AND ELECTRIC DEVICE**

(71) Applicant: Contemporary Amperex Technology (Hong Kong) Limited, Central, Central And Western Hong Kong (HK)
(72) Inventor: ZHANG, Limei, Ningde, Fujian 352100 (CN); ZOU, Hailin, Ningde, Fujian 352100 (CN); CHEN, Peipei, Ningde, Fujian 352100 (CN); LIU, Jiao, Ningde, Fujian 352100 (CN)
(74) Representative: Gong, Jinping
(86) International application number: PCT/CN2022/090476
(87) International publication number: WO 2023/206427

(57) **Abstract**

The present application provides a secondary battery, and providing a battery module, a battery pack, and a powder device comprising the same. The secondary battery includes a positive electrode plate and a non-aqueous electrolytic solution, in which the positive electrode plate includes a positive electrode active material having a core-shell structure, the positive electrode active material includes an core and a shell covering the core, the core includes Li₁₊ₓMn_{1-y}A_{y}P_{1-z}R_{z}O₄, the shell includes a first coating layer covering the core and a second coating layer covering the first coating layer, the non-aqueous electrolytic solution includes a first additive one or more selected from the compounds shown in formulae 1-A to 1-D. The secondary battery of the present application can have a relatively high energy density, and also a good rate performance, cycling performance, storage performance and safety performance.

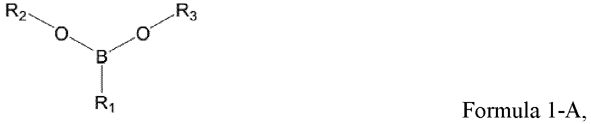

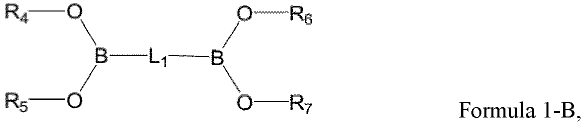

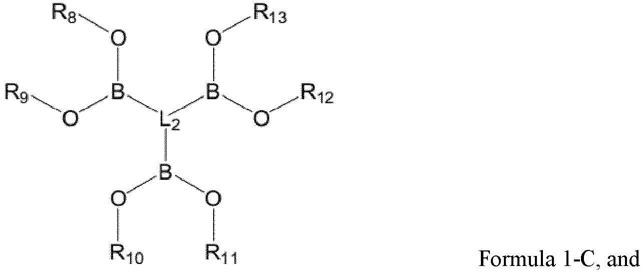

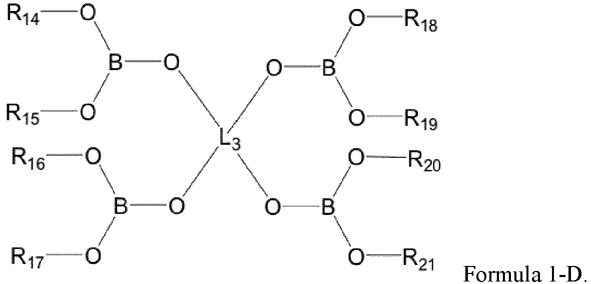

## Description

### TECHNICAL FIELD

The present application belongs to the technical field of battery, in particular to a secondary battery and a battery module, a battery pack and an electrical device comprising the same.

### BACKGROUND

In recent years, secondary batteries have been widely used in energy storage power systems such as hydro, thermal, wind and solar power plants, as well as electric tools, electric bicycles, electric motorcycles, electric vehicles, military equipment, aerospace and many other fields. With the application and promotion of secondary batteries, their safety performances are receiving more and more attention. Lithium manganese phosphate has become one of the most popular positive electrode active materials due to its high capacity, good safety performance and abundant raw material sources, however, lithium manganese phosphate is prone to manganese ion leaching-out during charging, resulting in rapid capacity decay, thereby constraining its commercialization.

### SUMMARY

An object of the present application is to provide a secondary battery and a battery module, a battery pack and an electrical device containing the same, aiming to enable the secondary battery to have a relatively high energy density, and a good rate performance, cycling performance, storage performance and safety performance.

A first aspect of the present application provides a secondary battery, comprising a positive electrode plate and a non-aqueous electrolytic solution, wherein
the positive electrode plate comprises a positive electrode active material having a core-shell structure, the positive electrode active material comprising a core and a shell covering the core, wherein
the core comprises Li₁₊ₓMn_{1-y}A_{y}P_{1-z}R_{z}O₄, wherein
   x is from -0.100 to 0.100,
   y is from 0.001 to 0.500,
   z is from 0.001 to 0.100,
   A is one or more selected from the group consisting of Zn, Al, Na, K, Mg, Mo, W, Ti, V, Zr, Fe, Ni, Co, Ga, Sn, Sb, Nb, and Ge, and optionally is one or more selected from the group consisting of Fe, Ti, V, Ni, Co, and Mg,
   R is one or more element(s) selected from the group consisting of B, Si, N, and S;
the shell comprises a first coating covering the core, and a second coating covering the first coating, wherein
   the first coating layer comprises pyrophosphate MP₂O₇ and phosphate XPO₄, wherein M and X are each independently one or more selected from Li, Fe, Ni, Mg, Co, Cu, Zn, Ti, Ag, Zr, Nb, and Al,
   the second coating comprises carbon;
the non-aqueous electrolytic solution comprises a first additive one or more selected from the compounds shown in formulae 1-A to 1-D, 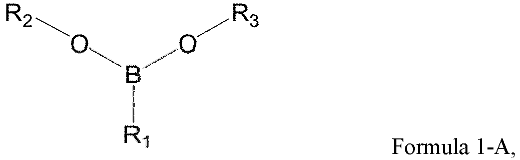 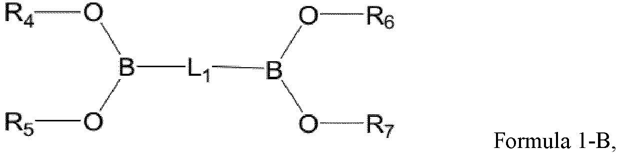 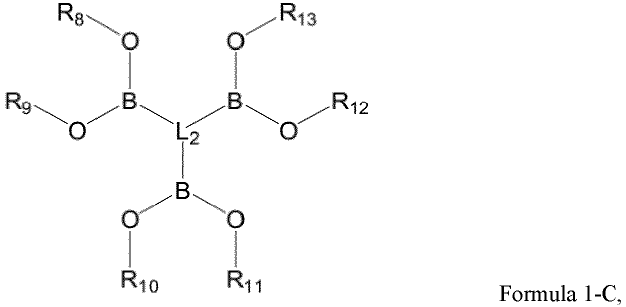 and 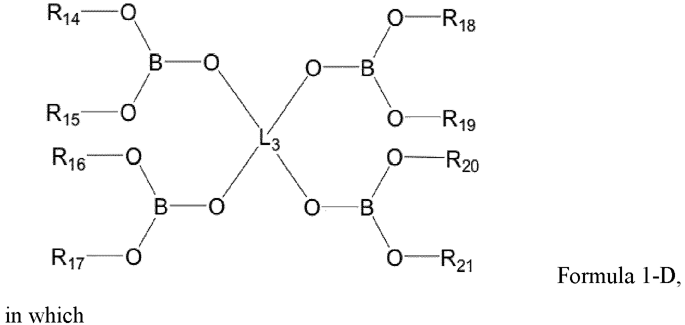
R₁ represents a hydrogen atom or at least one selected from the group consisting of hydroxyl, C1-C18 monovalent alkyl, C1-C18 monovalent alkoxy, C2-C18 monovalent alkoxy alkyl, C3-C18 monovalent cycloalkyl, C2-C18 monovalent heterocyclic alkyl, C6-C18 monovalent aryl, C7-C18 monovalent aryl alkyl, C7-C18 monovalent alkylaryl, C6-C18 monovalent aryloxy, C7-C18 monovalent aryloxy alkyl, C12-C18 monovalent aryl ether group, C2-C18 monovalent heteroaryl, C3-C18 monovalent heteroaryl alkyl, C3-C18 monovalent alkyl heteroaryl, and C1-C18 monovalent silyl;
R₂ to R₂₁ each independently represent a hydrogen atom or at least one selected from the group consisting of C1-C18 monovalent alkyl, C2-C18 monovalent alkoxyalkyl, C3-C18 monovalent cycloalkyl, C2-C18 monovalent oxocycloalkyl, C6-C18 monovalent aryl, C7-C18 monovalent aryl alkyl, C7-C18 monovalent alkylaryl, C7-C18 monovalent aryloxyalkyl, C12-C18 monovalent aryl ether group, C2-C18 monovalent heteroaryl, C3-C18 monovalent heteroaryl alkyl, C3-C18 monovalent alkyl heteroaryl, and C1-C18 monovalent silyl, and when R₂ and R₃ both are hydrogen atoms, R₁ is not hydroxyl;
optionally, R₂ and R₃ can further bond with each other to form a ring structure, R₄ and R₅ can further bond with each other to form a ring structure, R₆ and R₇ can further bond with each other to form a ring structure, R₈ and R₉ can further bond with each other to form a ring structure, R₁₀ and R₁₁ can further bond with each other to form a ring structure, R₁₂ and R₁₃ can further bond with each other to form a ring structure, R₁₄ and R₁₅ can further bond with each other to form a ring structure, R₁₆ and R₁₇ can further bond with each other to form a ring structure, R₁₈ and R₁₉ can further bond with each other to form a ring structure, and R₂₀ and R₂₁ can further bond with each other to form a ring structure;
L₁ represents an oxygen atom or at least one selected from the group consisting of C1-C18 divalent alkyl, C1-C18 oxodialkyl, C6-C18 divalent cycloalkyl, C6-C18 divalent oxo cycloalkyl, C6-C18 divalent aryl, C7-C18 divalent arylalkyl, C7-C18 divalent alkyl aryl, C6-C18 divalent aryloxy, C7-C18 divalent aryloxyalkyl, C12-C18 divalent aryl ether group, C2-C18 divalent heteroaryl, C3-C18 divalent heteroaryl alkyl, and C3-C18 divalent alkyl heteroaryl;
L₂ represents at least one selected from the group consisting of C1-C18 trivalent alkyl, C1-C18 oxo trivalent alkyl, C6-C18 trivalent cycloalkyl, C6-C18 trivalent oxocycloalkyl, C6-C18 trivalent aryl, C7-C18 trivalent arylalkyl, C7-C18 trivalent alkylaryl, C6-C18 trivalent aryloxy, C7-C18 trivalent aryloxyalkyl, C12-C18 trivalent aryl ether group, C3-C18 trivalent heteroaryl, C3-C18 trivalent heteroaryl alkyl, and C3-C18 trivalent alkyl heteroaryl; and
L₃ represents at least one selected from the group consisting of C1-C18 tetravalent alkyl, C1-C18 oxo tetravalent alkyl, C6-C18 tetravalent cycloalkyl, C6-C18 tetravalent oxo cycloalkyl, C6-C18 tetravalent aryl, C7-C18 tetravalent arylalkyl, C7-C18 tetravalent alkylaryl, C7-C18 tetravalent aryloxyalkyl, C12-C18 tetravalent aryl ether group, C4-C18 tetravalent heteroaryl, C4-C18 tetravalent heteroaryl, and C4-C18 tetravalent alkyl heteroaryl.

**In** the present application, by treating lithium manganese phosphate via doping specific element and surface coating, the leaching-out of manganese ions during the lithiation and delithiation of lithium can be can effectively reduced and the migration of lithium ions can be prompted; as a result, the rate performance, cycling performance, storage performance, and safety performance of the secondary battery can be improved. When the non-aqueous electrolytic solution contains the first additive represented by Formula 1-A to Formula 1-D, it can form a dense and stable interface film on the surface of the positive active material during the secondary battery charging process, reduce the leaching-out of the first coating layer, reduce the leaching-out of manganese ions, reduce the catalytic oxidation of the second coating layer under high voltage, and reduce the consumption of non-aqueous electrolytic solution and active lithium ions. Therefore, the secondary battery of the present application can simultaneously possess high energy density, good rate performance, cycling performance, storage performance, and safety performance.

In some embodiments of the present application, R₂ and R₃ bond with each other to form a ring structure.

In some embodiments of the present application, at least one of R₁ to R₃ represents C3-C18 monovalent cycloalkyl, C2-C18 monovalent oxocycloalkyl, C6-C18 monovalent aryl, C7-C18 monovalent arylalkyl, C7-C18 monovalent alkylaryl, C7-C18 monovalent aryloxyalkyl, C12-C18 monovalent aryl ether group, C2-C18 monovalent heteroaryl, C3-C18 monovalent heteroarylalkyl, or C3-C18 monovalent alkyl heteroaryl; and optionally, R₁ to R3 each independently represent C6-C18 monovalent aryl, C7-C18 monovalent arylalkyl, C7-C18 monovalent alkylaryl, C7-C18 monovalent aryloxyalkyl, C12-C18 monovalent aryl ether group, C2-C18 monovalent heteroaryl group, C3-C18 monovalent heteroaryl group, or C3-C18 monovalent alkyl heteroaryl group.

When R₁ to R₃ represent the above substituents, it is helpful to form an interface film containing cross-linked macromolecular polymer on the surface of the positive active material, further reduce the leaching-out of the first layer and reduce the catalytic oxidation of the second coating layer, reduce the leaching-out of manganese ions and the consumption of non-aqueous electrolytic solution and active lithium ions, so as to further enhance the improvement effect on the cycling performance and storage performance of the secondary battery.

In some embodiments of the present application, R₄ and R₅, and R₆ and R₇, at least one pair of them, bond with each other to form a ring structure.

In some embodiments of the present application, L₁ represents an oxygen atom, and R₄ to R₇ each independently represent C6-C18 monovalent aryl, C7-C18 monovalent arylalkyl, C7-C18 monovalent alkylaryl, C7-C18 monovalent aryloxyalkyl, C12-C18 monovalent aryl ether group, C2-C18 monovalent heteroaryl, C3-C18 monovalent heteroarylalkyl, or C3-C18 monovalent alkyl heteroaryl.

In some embodiments of the present application, R₈ and R₉, R₁₀ and R₁₁, and R₁₂ and R₁₃, at least one pair of them, bond to form a ring structure.

In some embodiments of the present application, L₂ represents C6-C18 trivalent aryl, C7-C18 trivalent arylalkyl, C7-C18 trivalent alkylaryl, C6-C18 trivalent aryloxy, C7-C18 trivalent aryloxyalkyl, C12-C18 trivalent aryl ether group, C3-C18 trivalent heteroaryl group, C3-C18 trivalent heteroaryl alkyl, or C3-C18 trivalent alkyl heteroaryl.

In some embodiments of the present application, R₁₄ and R₁₅, R₁₆ and R₁₇, R₁₈ and R₁₉, R₂₀ and R₂₁, at least on pair of them, bond to form a ring structure.

In some embodiments of the present application, L₃ represents C6-C18 tetravalent aryl, C7-C18 tetravalent arylalkyl, C7-C18 tetravalent alkylaryl, C7-C18 tetravalent aryloxyalkyl, C12-C18 tetravalent aryl ether group, C4-C18 tetravalent heteroaryl group, C4-C18 tetravalent heteroaryl, or C4-C18 tetravalent alkyl heteroaryl.

In some embodiments of the present application, the first additive comprises at least one of the following compounds:

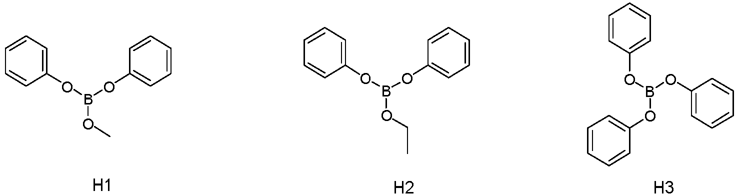

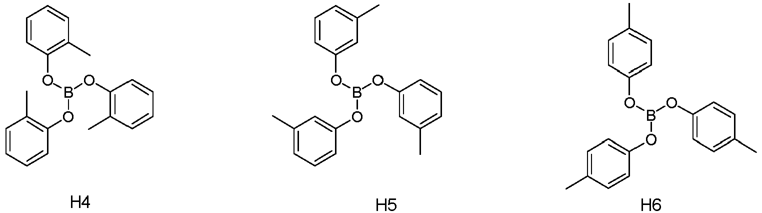

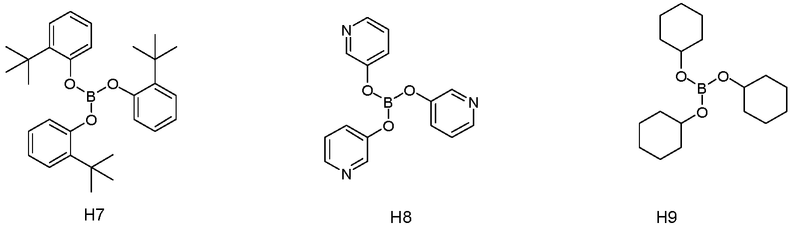

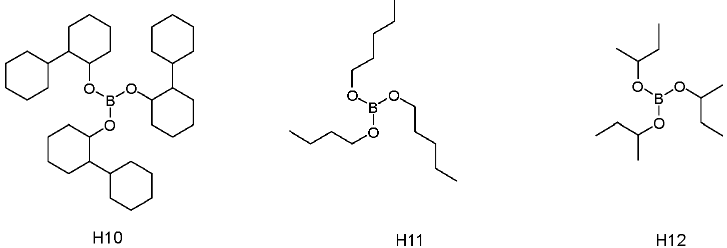

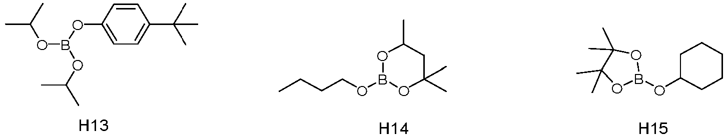

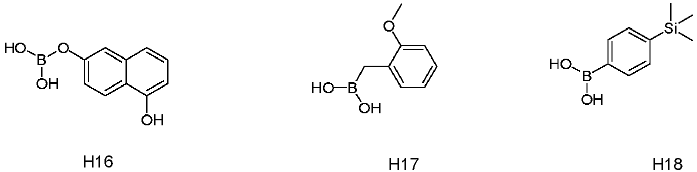

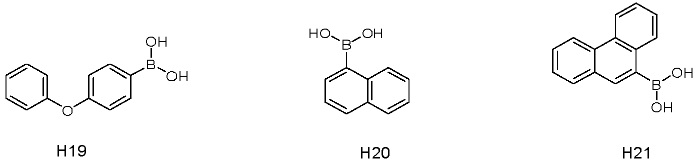

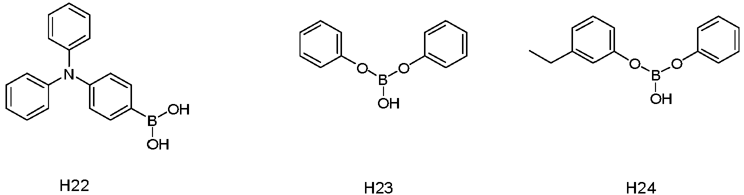

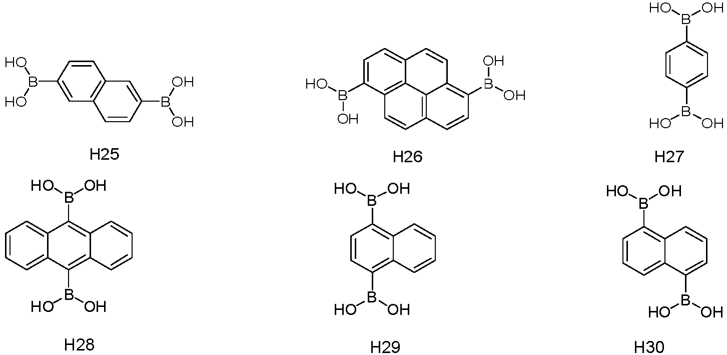

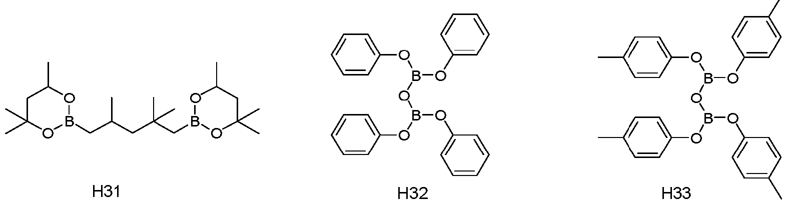

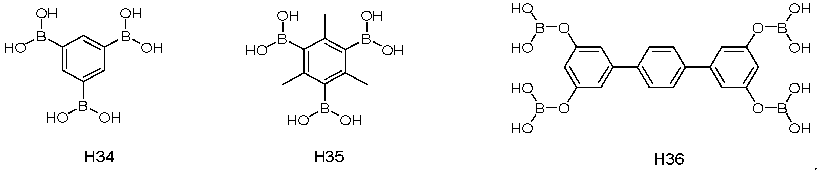

The inventors have found in the research process that taking at least one of the above compounds H1 to H36 as the first additive can form a more compact and stable interface film on the surface of the positive active material, which helps to further reduce the leaching-out of the first coating layer and reduce the catalytic oxidation of the second coating layer, and further reduce the leaching-out of manganese ions and the consumption of non-aqueous electrolytic solution and active lithium ions; this can further enhance the improvement effect on the cycling performance and storage performance of secondary batteries.

Optionally, the first additive comprises at least one selected from the group consisting of H1 to H10, H13 to H15, H31 to H33, and more optionally, the first additive comprises at least one selected from the group consisting of H1 to H8 and H32 to H33. These first additives help to form an interface film containing cross-linked macromolecular polymer on the surface of the positive active material, further reduce the leaching-out of the first coating layer and reduce the catalytic oxidation of the second coating layer, thus further reducing the leaching-out of manganese ions and the consumption of a non-aqueous electrolytic solution and active lithium ions, which can further enhance the improvement of the secondary battery cycling performance and storage performance.

In some embodiments of the present application, the non-aqueous electrolytic solution further comprises a second additive comprising one or more selected from the group consisting of sulfonic acid lactones, cyclic sulfate, lithium difluorophosphate, lithium difluorooxalate phosphate, and lithium difluorooxalate borate. When the non-aqueous electrolytic solution comprises both the first and second additives, it helps to significantly improve the cycling and storage performance of the secondary battery, while also enhancing the capacity exertion and rate performance of the secondary battery.

In some embodiments of the present application, the sulfonic acid lactones comprise at least one of the compounds as represented by Formula 2-A, 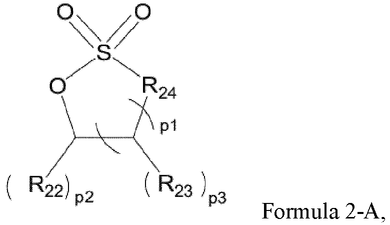 in which
p1 represents 1, 2, or 3, p2 represents 1 or 2, and p3 represents 1 or 2;
R₂₂, at each occurrence, independently represents a hydrogen atom, halogen atom, carboxylic ester group, sulfonic ester group, C1-C6 monovalent alkyl, C1-C6 monovalent haloalkyl, C1-C6 monovalent alkoxy, C1-C6 monovalent haloalkoxy, C2-C6 monovalent alkenyl, or a chain structure or a ring structure formed from more than one of the aforementioned groups linked together with single bonds, and when R₂₂ represents the ring structure, it is bonded to the ring of Formula 2-A via a single bond or shares a carbon atom with the ring of Formula 2-A to form a spiral-ring compound;
R₂₃, at each occurrence, independently represents a hydrogen atom, halogen atom, carboxylic ester group, sulfonic ester group, C1-C6 monovalent alkyl, C1-C6 monovalent haloalkyl, C1-C6 monovalent alkoxy, C1-C6 monovalent haloalkoxy, C2-C6 monovalent alkenyl, or a chain structure or a ring structure formed from more than one of the aforementioned groups linked together with single bonds, and when R₂₃ represents the ring structure, it is bonded to the ring of Formula 2-A via a single bond or shares a carbon atom with the ring of Formula 2-A to form a spiral-ring compound;
R₂₄ represents carbonyl or C(Y¹)₂, wherein Y₁ at each occurrence independently represents a hydrogen atom, halogen atom, carboxylic ester group, sulfonic ester group, C1-C6 monovalent alkyl group, C1-C6 monovalent haloalkyl group, C1-C6 monovalent alkoxy group, C1-C6 monovalent haloalkoxy group, C2-C6 monovalent alkenyl group, C6-C12 monovalent aryl group, or a combination thereof; and
optionally, R₂₂ and R₂₃ can further bond with each other to form a ring structure.

Optionally, the sulfonic acid lactone comprises at least one of the following compounds:

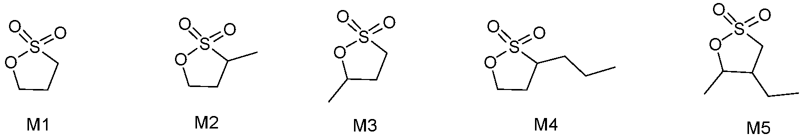

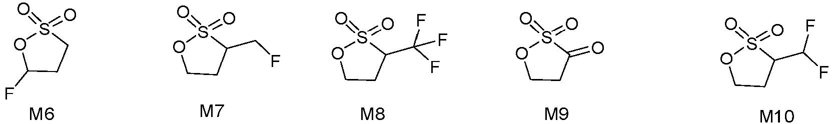

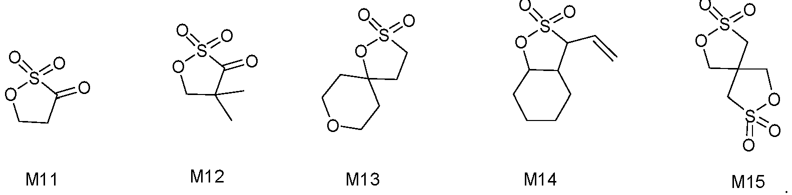

In some embodiments of the present application, the cyclic sulfate comprises at least one of the compounds represented by Formula 2-B, 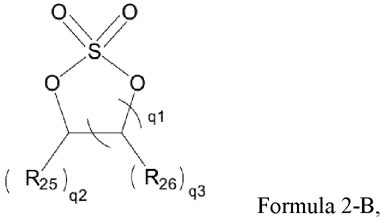 in which
q1 represents 1, 2, or 3, q2 represents 1 or 2, q3 represents 1 or 2.
R₂₅, at each occurrence, independently represents a hydrogen atom, halogen atom, carbonyl oxygen atom, carboxylate ester group, sulfate ester group, C1-C6 monovalent alkyl, C1-C6 monovalent haloalkyl, C1-C6 monovalent alkoxy, C1-C6 monovalent haloalkoxy, and C2-C6 monovalent alkenyl, or a chain structure or a ring structure formed from more than one of the aforementioned groups linked together with single bonds, and when R₂₅ represents the ring structure, it is bonded to the multi-ring of Formula 2-B via a single bond or shares a carbon atom with the multi-ring of Formula 2-B to form a spiral-ring compounds;
R₂₆, at each occurrence, independently represents a hydrogen atom, halogen atom, carboxylic ester group, sulfate group, C1-C6 monovalent alkyl group, C1-C6 monovalent haloalkyl, C1-C6 monovalent alkoxy, C1-C6 monovalent haloalkoxy, and C2-C6 monovalent alkenyl, or a chain structure or a ring structure formed from more than one of the aforementioned groups linked together with single bonds, and when R₂₆ represents the ring structure, it is bonded to the multi-ring of Formula 2-B via a single bond or shares a carbon atom with the multi-ring of Formula 2-B to form a spiral-ring compounds;
optionally, R₂₅ and R₂₆ can further bond with each other to form a ring structure.

Optionally, the cyclic sulfate comprises at least one of the following compounds:

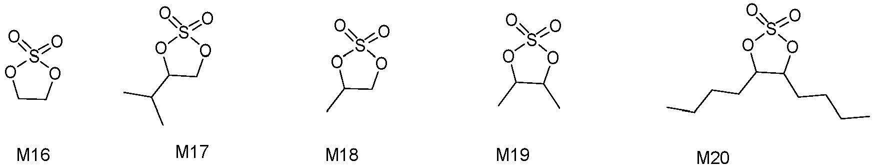

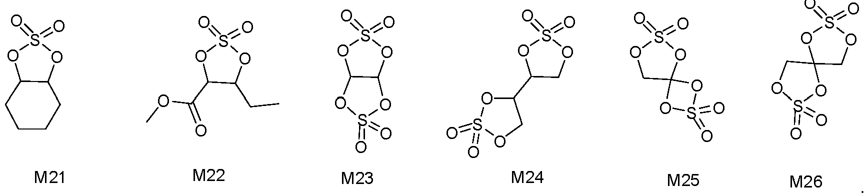

In some embodiments of the present application, the first additive has an amount of W1 % by weight of from 0.01 to 20, optionally from 0.1 to 10, and more optionally from 0.3 to 5, based on the total weight of the non-aqueous electrolytic solution. This can reduce the leaching-out of the first coating layer and reduce the catalytic oxidation effect of the second coating layer, and not deteriorate the positive electrode interface impedance, thereby significantly improving the cycling and storage performance of the secondary battery, without affecting the capacity exertion and rate performance of the secondary battery.

In some embodiments of the present application, the second additive has an amount of W2 % by weight of from 0.01 to 20, optionally from 0.1 to 10, and more optionally from 0.3 to 5, based on the total weight of the non-aqueous electrolytic solution. This can effectively improve the capacity exertion and rate performance of secondary batteries.

In some embodiments of the present application, the first coating has a coating amount of C1 % by weight of from greater than 0 to less than or equal to 7, and optionally from 4 to 5.6, based on the weight of the core. Thus, the function of the first coating layer can be effectively utilized without affecting the dynamic performance of the secondary battery due to the thickness of the coating layer.

In some embodiments of the present application, the second coating has a coating amount of C2 % by weight of from greater than 0 to less than or equal to 6, and optionally from 3 to 5, based on the weight of the core. Thus, the existence of the second coating layer can avoid direct contact between the positive electrode active material and the electrolyte, reduce the erosion of the electrolyte on the positive electrode active material, and improve the conductivity of the positive electrode active material. When the second layer of coating is within the above range, it can effectively enhance the specific capacity of the positive electrode active material.

In some embodiments of the present application, W1/W2 is from 0.01 to 20, and optionally from 0.01 to 10. This can further enhance the improvement effect on the cycling performance and storage performance of the secondary battery, while enhancing the capacity exertion and rate performance of the secondary battery.

In some embodiments of the present application, (W1+W2)/(C1+C2) is from 0.001 to 2, and optionally from 0.01 to 1. This can significantly enhance the improvement effect on the cycling performance and storage performance of the secondary battery, while enhancing the capacity exertion and rate performance of the secondary battery.

In some embodiments of the present application, the non-aqueous electrolytic solution further comprises a third additive comprising one or more selected from the group consisting of cyclic carbonate compounds having unsaturated bond(s), halogenated cyclic carbonate compounds, nitrile compounds, phosphoronitrile compounds, aromatic and halogenated aromatic compounds, isocyanate compounds, anhydride compounds, phosphite compounds, phosphate ester compounds, sulfite compounds or dimethyl disulfonate compounds. The third additive helps to form a more compact and stable interface film on the surface of the positive and/or negative active materials, thus helping to further improve at least one of the cycling performance, storage performance and magnification performance of the secondary battery.

**In** some embodiments of the present application, in the core, y and 1-y has a ratio of from 1:10 to 10:1, and optionally from 1:4 to 1:1. Under the circumstances, the energy density and cycling performance of the secondary battery can be further improved.

In some embodiments of the present application, in the core, z and 1-z has a ratio of from 1:9 to 1:999, and optionally from 1:499 to 1:249. Under the circumstances, the energy density and cycling performance of the secondary battery can be further improved.

**In** some embodiments of the present application, A is s at least two selected from Fe, Ti, V, Ni, Co, and Mg. Therefore, by using A from two or more above metals, doping at the manganese site is beneficial for enhancing the doping effect, thereby further reducing surface oxygen activity and inhibiting the leaching-out of manganese ions.

In some embodiments of the present application, the interplanar spacing of the phosphate in the first coating layer is 0.345-0.358nm, and the included angle in the crystal direction (111) is 24.25°-26.45°. Under the circumstances, the energy density and cycling performance of the secondary battery can be further improved.

In some embodiments of the present application, the interplanar spacing of the pyrophosphate of the first coating layer is 0.293-0.326 nm, and the included angle in the crystal directions (111) is 26.41°-32.57°. Under the circumstances, the energy density and cycling performance of the secondary battery can be further improved.

In some embodiments of the present application, the weight ratio of the pyrophosphate to phosphate in the first coating layer is from 1:3 to 3:1, and optionally from 1:3 to 1:1. Therefore, by using pyrophosphate and phosphate in an appropriate weight ratio range, the leaching-out of manganese ions can be effectively hinderer, the content of surface lithium impurities can be reduced, and interface side reactions can be reduced; as a result, the rate performance, cycling performance, storage performance, and safety performance of secondary batteries can be improved.

In some embodiments of the present application, the crystallinity of the pyrophosphate and phosphate are independently from 10% to 100%, and optionally from 50% to 100%. Therefore, by making the pyrophosphate and phosphate fall with the above crystallinity ranges, it is beneficial for the pyrophosphate to fully hinder the leaching-out of manganese ions and is beneficial for the phosphate to reduce surface lithium content and reduce interfacial side reactions.

In some embodiments of the present application, the Li/Mn antisite defect concentration of the positive electrode active material is 4% or less, and optionally may be 2% or less. Under the circumstances, the specific capacity and rate performance of the positive electrode active material can be improved.

In some embodiments of the present application, the lattice change rate of the positive electrode active material is 6% or less, and optionally may be 4% or less. Under the circumstances, the rate performance of the secondary battery can be improved.

In some embodiments of the present application, the surface oxygen valence of the positive electrode active material is -1.88 or less, and optionally may be from -1.98 to -1.88. Under the circumstances, the cycling performance and storage performance of the secondary battery can be improved.

In some embodiments of the present application, the compaction density under 3 tons of the positive electrode active material is 2.0g/cm³ or higher, and optionally may be 2.2 g/cm³ or more or even higher. Under the circumstances, the volume energy density of the secondary battery can be improved.

**In** some embodiments of the present application, the positive electrode active material has a specific surface area of B in m²/g, with B being in the range of from 7 to 18, optionally from 10 to 15. As a result, it is allowed to ensure that the positive electrode plate has a high lithium ion conductivity, while the secondary battery has good rate performance, cycling performance and storage performance.

A second aspect of the present application provides a battery module, comprising the secondary battery according to the first aspect of the present application.

A third aspect of the present application provides a battery pack, comprising the battery module according to the second aspect of the present application.

A fourth aspect of the present application provides an electrical device comprising at least one of the secondary battery of the first aspect of the present application, the battery module of the second aspect of the present application or the battery pack of the third aspect of the present application.

The battery module, battery pack and electrical device include the secondary battery of the present application, and thus have at least the same advantages as the secondary battery.

### DESCRIPTION OF THE DRAWINGS

In order to explain the technical solutions of the embodiments of the present application more clearly, the following will briefly introduce the drawings that need to be used in the embodiments of the present application. Apparently, the drawings described below are only some embodiments of the present application. A person skilled in the art can obtain other drawings based on the accompanying drawings without creative work.
FIG. 1 is a schematic diagram of a secondary battery according to an embodiment of the present application.
FIG. 2 is an exploded view of a secondary battery according to the embodiment of the present application as shown in FIG. 1.
FIG. 3 is a schematic diagram of a battery module according to an embodiment of the present application.
FIG. 4 is a schematic diagram of a battery pack according to an embodiment of the present application.
FIG. 5 is an exploded view of the battery pack according to the embodiment of the present application as shown in FIG. 4.
FIG. 6 is a schematic diagram of an electrical device according to an embodiment of the present application using the secondary battery of the present application as power.
Figure 7 is a comparison between the XRD pattern of the core of the positive active material prepared in Example 1-1 and the standard XRD pattern of lithium manganese phosphate (00-033-0804).

In the drawings, the accompanying drawings are not necessarily drawn to an actual scale. Among them, the reference sings in the accompanying drawings are illustrated as follows: 1 - battery pack, 2 - upper case, 3 - lower case, 4 - battery module, 5 - secondary battery, 51 - housing, 52 - electrode assembly, and 53- cover plate.

### DETAILED DESCRIPTION

Hereinafter, embodiments of the secondary battery and the battery module, the battery pack and the electrical device containing the same will be described in detail with reference to the accompanying drawings as appropriate. However, unnecessary detailed descriptions may be omitted in some cases, for example the detailed description of a well-known item or the repetitive description of an actually identical structure, so as to prevent the following description from becoming unnecessarily redundant and to facilitate understanding by those skilled in the art. In addition, the drawings and the following description are provided for those skilled in the art to fully understand the present application, and are not intended to limit the subject matter described in the claims.

The "range(s)" disclosed in the present application is/are defined in the form of lower and upper limits, and a given range is defined by selection of a lower limit and an upper limit that define boundary of the particular range. Ranges defined in this manner may or may not be inclusive of the endpoints, and may be arbitrarily combined. That is, any lower limit may be combined with any upper limit to form a range. For example, if the ranges of 60-120 and 80-110 are listed for a particular parameter, it is to be understood that the ranges of 60-110 and 80-120 are also contemplated. Additionally, if the minimum range values 1 and 2 are listed, and the maximum range values 3, 4, and 5 are listed, the following ranges are all expected: 1-3, 1-4, 1-5, 2- 3, 2-4 and 2-5. In the present application, unless stated otherwise, the numerical range "a-b" represents an abbreviated representation of any combination of real numbers between a and b, where both a and b are real numbers. For example, the numerical range "0-5" means that all real numbers between "0-5" have been listed herein, and the range "0-5" is just an abbreviated representation of the combination of these numerical values. In addition, when a parameter is expressed as an integer greater than or equal to 2, it is equivalent to disclose that the parameter is, for example, an integer of 2, 3, 4, 5, 6, 7, 8, 9, 10, 11, 12, and the like.

Unless stated otherwise, all the embodiments and the optional embodiments of the present application can be combined with each other to form a new technical solution, and such technical solution should be considered to be included in the disclosure of the present application.

Unless stated otherwise, all technical features and optional technical features of the present application can be combined with each other to form a new technical solution, and such technical solution should be considered to be included in the disclosure of the present application.

Unless stated otherwise, all steps of the present application can be carried out sequentially, and also can be carried out randomly, preferably they are carried out sequentially. For example, the method includes steps (a) and (b), indicating that the method may include steps (a) and (b) performed in sequence, or that the method may include steps (b) and (a) performed in sequence. For example, reference to the method further comprising step (c) indicates that step (c) may be added to the method in any order. As an example, the method may comprises steps (a), (b) and (c), steps (a), (c) and (b), or steps (c), (a) and (b), and the like.

Unless stated otherwise, the transition phases "comprise", "comprising", "contain" and "containing" mentioned in the present application mean that it is drafted in an open mode, or it may also mean a close mode. For example, the transition phases "comprise", "comprising", "contain" and "containing" may mean that other components not listed may also be included or contained, or only the listed components may be included or contained.

In the present application herein, unless otherwise stated, the term "or" is inclusive. For example, the phrase "A or B" means "A, B, or both A and B". More specifically, any of the following conditions meets "A or B": A is true (or present) and B is false (or absent); A is false (or absent) and B is true (or present); or both A and B are true (or present).

It shall note that as used herein, the term "median particle size Dv50" is a particle size at which a cumulative volume distribution percentage of a material reaches to 50%. In the present application, the median particle size Dv50 of a material may be determined using a laser diffraction particle size analysis. For example, with reference to standard GB/T 19077-2016, it is determined using a laser particle size analyzer (e.g., Malvern Master Size 3000).

It should be noted that in this article, the term "coating layer" refers to a material coated on the core. The use of "coating layer" is only for convenience of description and is not intended to limit the present invention. In addition, for each coating layer, the coating may be fully coating or partially coating.

Herein, the term "source" refers to a compound that is the source of an element, including, for example, but not limited to, carbonates, sulfates, nitrates, elemental substances, halides, oxides, and hydroxides.

Herein, the term "aryl" refers to a closed aromatic ring or ring system. Unless otherwise explicitly stated, the "aryl" structure can be single ring, multi ring, or fused ring. Examples of aryl groups include, but are not limited to, phenyl, naphthyl, anthracyl, phenyl, diphenyl, triphenyl, tetraphenylene, triphenylene, pyrenyl, perylene, indenyl, benzophenyl, fluorenyl, 9,9-dimethylfluorenyl, and spirodifluorenyl.

Herein, the term "heteroaryl" refers to one or more atoms in the ring of an aryl group being elements other than carbon. Examples of heteroaryl groups include, but are not limited to, pyrrolyl, furanyl, thienyl, indolyl, benzofuranyl, benzothienyl, dibenzofuran, dibenzothiophene, carbazolyl, indocarbazolyl, indolecarbazolyl, pyridinyl, pyrimidinyl, pyrazine, pyridazinyl, triazine, oxazole, isoxazolyl, thiazolyl, and isothiazolyl. The heteroatoms in the heteroaryl group can include N, O, S, Si, etc. The number of heteroatoms in a heteroaryl group can be one or multiple, and the multiple heteroatoms can be the same or different.

Herein, when substituents represent groups consisting of certain functional groups, they include groups formed by single bond bonding of these groups. For example, when a substituent represents at least one "selected from the group consisting of hydroxyl, C1-C18 monovalent alkyl, and C6-C18 monovalent aryl", the substituent separately discloses hydroxyl, C1-C18 monovalent alkyl, C6-C18 monovalent aryl, hydroxyl-substituted C1-C18 monovalent alkyl, hydroxyl-substituted C6-C18 monovalent aryl, and C1-C18 monovalent alkyl-substituted C1-C18 monovalent aryl.

Herein, when a substituent is "monovalent", it refers to a group formed by removing one H atom from the molecule. When a substituent is "divalent", it refers to a group formed by removing two H atoms from the molecule. When a substituent is "trivalent", it refers to a group formed by removing three H atoms from the molecule. When a substituent is "tetravalent", it refers to a group formed by removing four H atoms from the molecule.

Throughout this specification, the substituents of compounds are disclosed in groups or ranges. Clearly expect this description to include each individual sub combination of members of these groups and scopes. For example, it is explicitly expected that the term "C1-C6 alkyl" will separately disclose C1, C2, C3, C4, C5, C6, C1-C6, C1-C5, C1-C4, C1-C3, C1-C2, C2-C6, C2-C5, C2-C4, C2-C3, C3-C6, C3-C5, C3-C4, C4-C6, C4-C5, and C5-C6 alkyl groups.

The inventors of the present application found in an actual operation that leaching-out of manganese ions is serious during deep charging and discharging of lithium manganese phosphate positive electrode active material. Although the prior art has attempted to coat the lithium manganese phosphate with lithium iron phosphate to reduce interfacial side reactions, such a coating layer cannot prevent the leached-out manganese ions from migrating into a non-aqueous electrolytic solution. The disassociated manganese ions are reduced to metal manganese after migration to a negative electrode. The produced metal manganese is equivalent to a "catalyst", which can catalyze decomposition of SEI (solid electrolyte interface) film on the surface of the negative electrode. A part of the produced by-product is gas, which is prone to cause the secondary battery to expand and thus negatively affect safety performance of the secondary battery; and the other part deposits on the surface of the negative electrode and thus blocks channels for lithium ions to move in and out of the negative electrode, resulting in an increased impedance of the battery and negatively affecting dynamic performance of the battery. In addition, in order to supplement the decomposed SEI film, the non-aqueous electrolytic solution and active lithium ions in the battery are continuously consumed, which has an irreversible impact on capacity retention rate of the battery.

The inventors, after thorough consideration on a positive electrode and a non-aqueous electrolytic solution, have designed a secondary battery capable of simultaneously combining a high energy density with good rate performance, cycling performance, storage performance and safety performance.

Specifically, a first aspect of the present application provides a secondary battery.

### SECONDARY BATTERY

**A** secondary battery, also known as a rechargeable battery or accumulator, is a battery that can continue to be used by activating its active material by means of charging after the battery has been discharged. Typically, a secondary battery includes an electrode assembly and a non-aqueous electrolytic solution, and the electrode assembly includes a positive electrode plate, a negative electrode plate, and a separator. The separator is disposed between the positive electrode plate and the negative electrode plate, which mainly functions to prevent short circuit of the positive and negative electrodes and at the same time allow lithium ions to pass through. The non-aqueous electrolytic solution functions to conduct lithium ions between the positive electrode plate and the negative electrode plate.

### [Positive electrode plate]

The secondary battery of the present application adopts a positive electrode plate comprising a positive electrode active material with a core-shell structure wherein the positive electrode active material comprises a core and a shell covering the core. The inner core comprises Li₁₊ₓMn_{1-y}A_{y}P_{1-z}R_{z}O₄, in which x is from -0.100 to 0.100, y is from 0.001 to 0.500, z is from 0.001 to 0.100, A is one or more selected from the group consisting of Zn, Al, Na, K, Mg, Mo, W, Ti, V, Zr, Fe, Ni, Co, Ga, Sn, Sb, Nb, and Ge, and optionally is one or more selected from the group consisting of Fe, Ti, V, Ni, Co, and Mg, and R is one or more element(s) selected from the group consisting of B, Si, N, and S; the shell comprises a first coating covering the inner core and a second coating covering the first coating, wherein the first coating layer comprises pyrophosphate MP₂O₇ and phosphate XPO₄, wherein M and X are each independently one or more selected from Li, Fe, Ni, Mg, Co, Cu, Zn, Ti, Ag, Zr, Nb, and Al; the second coating comprises carbon.

Unless otherwise stated, in the above chemical formula, when A represents more than two elements, the above range for the x value is not only for the stoichiometric number of each element as A, but also for the sum of the stoichiometric numbers of the elements as A. For example, when A represents more than two elements A1, A2 ...... and An, each of the stoichiometric numbers y1, y2 ......and yn of respective A1, A2 ......and An must fall within the numerical range defined by the present application for x, and the sum of y1, y2...... and yn must also fall within such numerical range of the present application. Similarly, in the case where R is more than two elements, the limitations on numerical ranges for the stoichiometric numbers of R in the present application also have the above meaning.

After extensive research, the inventors found that for lithium manganese phosphate positive electrode active materials, the serious leaching-out of manganese ions and high surface reactivity may result from the Ginger Taylor effect and the changes in Li⁺ channel size after Mn³⁺ deintercalation. To this end, the inventors have obtained a positive electrode active material that have significantly reduced leaching-out of manganese ions and reduced lattice change rate by doping and modifying lithium manganese phosphate and coating it with multiple layers.

The positive electrode active material of the present application has a core-shell structure, wherein the core comprises Li₁₊ₓMn_{1-y}A_{y}P_{1-z}R_{z}O₄. The doping of element A in the manganese site of lithium manganese phosphate in the core helps to reduce the lattice change rate of lithium manganese phosphate during the intercalation and deintercalation of lithium, improve the structural stability of lithium manganese phosphate as the positive electrode active material, greatly reduce the leaching-out of manganese ions, and reduce the oxygen activity of the particle surface. Element R dopped at the phosphorus site helps to facilitate the changing of the Mn-O bond-length, thereby reducing the potential barrier for lithium ion migration, promoting lithium ion migration, and improving the rate performance of secondary batteries.

The first coating layer of the positive electrode active material of the present application includes pyrophosphate and phosphate. Due to the high migration potential barrier of transition metals in pyrophosphate (>1eV), the leaching-out of transition metal ions can be effectively reduced. Phosphates have excellent lithium-ion conductivity and thus can reduce the surface impurities.

The second coating layer of the positive electrode active material of the present application is a carbon containing layer, so the conductivity and desolvation ability of LiMnPO₄ can be effectively improved. In addition, the "barrier" effect of the second coating layer can further hinder the migration of manganese ions into non-aqueous electrolytic solution and reduce the erosion of positive electrode active materials by the non-aqueous electrolytic solution.

Therefore, in the present application, the leaching-out of manganese ions during the deintercalation of lithium phosphate can be effectively reduced by doping specific elements and surface coating, also the migration of lithium ions is promoted, thereby improving the rate performance, cycling performance, storage performance, and safety performance of the secondary battery.

It should be pointed out that the position of the main characteristic peak of the positive electrode active material core in the present application is basically consistent with that before LiMnPO₄ doping, indicating that the doped lithium manganese phosphate positive electrode active material core has no impurity phase; the improvement of secondary battery performance mainly results from the element doping, rather than impurity phase.

In the core, x ranges from -0.100 to 0.100, for example, x may be 0.006, 0.004, 0.003, 0.002, 0.001, 0, -0.001, -0.003, -0.004, -0.005, -0.006, -0.007, -0.008, -0.009, and -0.100.

In the core, y ranges from 0.001 to 0.500, for example, y may be 0.1, 0.2, 0.25, 0.3, 0.35, 0.4, and 0.45.

In the core, z ranges from 0.001 to 0.100, for example, z may be 0.001, 0.002, 0.003, 0.004, 0.005, 0.006, 0.007, 0.008, 0.009, and 0.100.

In some embodiments, optionally, in the core, the ratio of y to (1-y) is from 1:10 to 10:1, and optionally from 1:4 to 1:1. Herein y denotes the sum of the stoichiometric numbers of the Mn-site doping element A. Under the circumstances that the above condition is satisfied, the energy density and cycling performance of the secondary battery can be further improved when the above conditions are met.

In some embodiments, in the core, the ratio of z to (1-z) is from 1:9 to 1:999, and optionally from 1:499 to 1:249. Herein z denotes the sum of the stoichiometric numbers of the P-position doping element R. Under the circumstances that the above condition is satisfied, the energy density and cycling performance of a secondary battery can be further improved when the above conditions are met.

**In** some embodiments, optionally, A is at least two selected from Fe, Ti, V, Ni, Co, and Mg.

It is conductive to enhancing the doping effect of two or more than two of the above elements at the manganese site in the lithium manganese phosphate as the positive electrode active material. On the one hand, the lattice change rate is it further reduced, thereby reducing the leaching-out of manganese ions and reducing the consumption of non-aqueous electrolytic solution and active lithium ions. On the other hand, the surface oxygen activity is further reduced and the interface side reactions between the positive electrode active material and non-aqueous electrolytic solutions is reduced, thereby improving the cycling performance and high-temperature storage performance of the secondary battery.

**In** some embodiments, optionally, the interplanar spacing of the phosphate in the first coating layer is 0.345-0.358nm, and the included angle in the crystal direction (111) is 24.25°-26.45°.

**In** some embodiments, optionally, the interplanar spacing of the first coating layer of pyrophosphate is 0.293-0.326nm, and the angle between the crystal directions (111) is 26.41°- 32.57°.

Under the condition that, in the first coating layer, the interplanar spacing and crystal orientation (111) angle between the phosphate and the pyrophosphate are within the above ranges, the impurity phase in the coating layer can be effectively reduced, thereby improving the specific capacity, cycling performance, and rate performance of the positive electrode active material.

**In** some embodiments, optionally, the weight ratio of the pyrophosphate to the phosphate in the first coating layer may be 1:3 to 3:1, and optionally may be 1:3 to 1:1.

The appropriate ratio of the pyrophosphate to the phosphate is conducive to fully achieving the synergistic effect of them, which can effectively hinder the leaching-out of manganese ions, reduce the surface lithium impurities, and reduce interface side reactions, so as to improve the rate performance, cycling performance, and safety performance of secondary batteries. In addition, the following situations can be effectively avoid: if there is too much pyrophosphate but too little phosphate, battery impedance may increase; if there is too much phosphate but too little pyrophosphate, the effect of reducing the leaching-out of manganese ions is not significant.

In some embodiments, the crystallinity of the pyrophosphate and phosphate may be independently from 10% to 100%, and optionally may be from 50% to 100%.

**In** the first coating layer of the manganese phosphate lithium as the positive electrode active material of the present application, pyrophosphate and phosphate with a certain crystallinity are beneficial for maintaining the structural stability of the first coating layer and reducing lattice defects. On the one hand, it is beneficial for the pyrophosphate to fully hinder the leaching-out of manganese ions. On the other hand, it is also beneficial for the phosphate to reduce the surface lithium-impurity content and the surface oxygen valence. As a result, the interface side reactions between the positive electrode active material and non-aqueous electrolytic solution can be reduced, the consumption of non-aqueous electrolytic solution can be reduced, and the cycling and storage performance of secondary batteries can be improved.

It should be noted that in the present application, the crystallinity of the pyrophosphate and phosphate can be adjusted by adjusting sintering conditions, such as sintering temperature and sintering time. The crystallinity of the pyrophosphate and phosphate may be measured by methods well-known in the art, such as X-ray diffraction, density method, infrared spectroscopy, differential scanning calorimetry, and nuclear magnetic resonance absorption method.

In some embodiments, the coating amount of the first coating layer is C1 weight%, with C1 greater than 0 and less than or equal to 7, and optionally from 4 to 5.6, based on the weight of the core.

Under the condition that the coating amount of the first coating layer is within the above ranges, the leaching-out of manganese ions can be further reduced and the transport of lithium ions can be prompted. Also, the following situations can effectively avoided: if the coating amount of the first coating layer is too small, the pyrophosphate cannot fully inhibit the leaching-out of manganese ions, and the transport performance of is cannot be significantly improved. If the coating amount of the first coating layer is too large, the coating layer may be too thick, so the battery impedance is increased and the dynamic performance of the secondary battery is affected.

In some embodiments, the coating amount of the second coating layer is C2 weight%, with C2 greater than 0 and less than or equal to 6, and optionally from 3 to 5, based on the weight of the core.

As the second coating layer, the carbon-containing layer has a "barrier" function, so as to avoid direct contact between the positive electrode active material and non-aqueous electrolytic solution, thereby reducing the erosion of non-aqueous electrolytic solution on the positive electrode active material and improving the safety performance of secondary batteries at high temperatures. In addition, the carbon-containing layer has strong conductivity, which can reduce the internal resistance of the battery, thereby improving the dynamic performance of the secondary battery. However, due to the low specific capacity of carbon materials, excessive use of the second coating layer may reduce the overall specific capacity of the positive electrode active material. Therefore, under the condition that the coating amount of the second coating layer is within the above ranges, the dynamic performance and safety performance of the secondary battery can be further improved without sacrificing the capacity of the positive electrode active material.

In some embodiments, the Li/Mn antisite defect concentration of the positive electrode active material may be 4% or less, and optionally may be 2% or less.

Li/Mn antisite defect refers to the exchange of positions between Li⁺ and Mn₂₊ in the LiMnPO₄ lattice. Li/Mn antisite defect concentration refers to the percentage of Li⁺ exchanged with Mn²⁺ in the positive electrode active material in the total amount of Li⁺. Due to the one-dimensional nature of the Li⁺ transport channel, Mn²⁺ is difficult to migrate in the Li⁺ transport channel. Therefore, Mn²⁺ with antisite defects can hinder the transport of Li⁺. In the positive electrode active material of the present application, by controlling the Li/Mn antisite defect concentration at a low level, the specific capacity and rate performance of the positive electrode active material can be improved. In the present application, the antisite defects concentration may be determined, for example, according to JIS K 0131-1996.

In some embodiments, the lattice change rate of the positive electrode active material may be 6% or less, and optionally may be 4% or less.

The intercalation and deintercalation of lithium of LiMnPO₄ is a two-phase reaction. The interfacial stress of the two phases is determined by the lattice change rate. The smaller the lattice change rate, the smaller the interfacial stress, and thus the easier the Li⁺ transfer. Therefore, reducing the lattice change rate of the core will be beneficial for enhancing the transmission capacity of Li⁺, thereby improving the rate performance of the secondary battery.

**In** some embodiments, optionally, the average discharge voltage of the positive electrode active material is 3.5V or more, and the discharging specific capacity is 140mAh/g or more; and optionally, the average discharging voltage is 3.6V or more and the discharging specific capacity is 145mAh/g or more.

Although the undoped LiMnPO₄ has an average discharging voltage of 4.0V or more, its discharge capacity is relatively low, usually less than 120mAh/g, resulting in a lower energy density of secondary batteries. By adjusting the lattice change rate via doping, the discharge capacity of the secondary battery can be significantly increased. With a slight decrease in average discharge voltage, the overall energy density of the secondary battery has significantly increased.

In some embodiments, the surface oxygen valence of the positive electrode active material may be - 1.88 or less, and optionally from -1.98 to -1.88.

The higher the valence of oxygen in a compound, the stronger the ability to obtain electrons of the oxygen, i.e. the stronger the oxidability of the oxygen. In the lithium manganese phosphate as the positive electrode active material of the present application, by controlling the surface oxygen valence at a lower level, the reaction activity of the positive electrode active material surface can be reduced, and the interface side reactions between the positive electrode active material and non-aqueous electrolytic solution can be reduced, thereby improving the cycling performance and high-temperature storage performance of the secondary battery.

In some embodiments, optionally, the compaction density under 3 tons (T) of the positive electrode active material is 2.0g/cm³ or more, and optionally 2.2g/cm³ or more.

The higher the compaction density of the positive electrode active material, i.e. the larger the weight of the positive electrode active material per unit volume; thus, it will be more conducive to improving the volume energy density of the secondary battery. In the present application, the compaction density may be determined for example according to GB/T 24533-2009.

In some embodiments, the specific surface area of the positive electrode active material is B m²/g, with B being from 7 to 18, and optionally from 10 to 15.

If the specific surface area of the positive active material is high, lithium ion conductivity of the positive electrode plate will be increased, which in turn can improve capacity exertion and rate performance of secondary batteries. However, the specific surface area of the positive electrode active material should not be too high, which may increase contact area between the positive electrode active material and a non-aqueous electrolytic solution, thereby increasing their interfacial side reactions and affecting cycling performance and storage performance of secondary batteries. By controlling the specific surface area of the positive electrode active material in a suitable range, it can ensure that the positive electrode plate has a high lithium ions conductivity, and at the same time, the secondary battery has good rate performance, cycling performance and storage performance. The specific surface area of the positive electrode active material has the common meaning in the art and may be determined by equipment and method known in the art. For example, with reference to GB/T 19587-2017, the nitrogen adsorption specific surface area analysis method can be used for testing, and the BET (BrunauerEmmett Teller) method can be used for calculating. The test can be carried out by Micromeritics Tri-Star 3020 specific surface area aperture analysis tester.

The present application further provides a method for the preparation of a positive electrode active material, comprising the steps of providing a core material and coating layer the core material.

Step of providing a core material: the core comprises Li₁₊ₓMn_{1-y}A_{y}P_{1-z}R_{z}O₄, in which x is from - 0.100 to 0.100, y is from 0.001 to 0.500, and z is from 0.001 to 0.100; A is one or more selected from Zn, Al, Na, K, Mg, Mo, W, Ti, V, Zr, Fe, Ni, Co, Ga, Sn, Sb, Nb, and Ge, and optionally one or more selected from Fe, Ti, V, Ni, Co, and Mg; R is one or more selected from B, Si, N, and S.

Step of coating: Providing MP₂O₇ powder and XPO₄ suspension containing a carbon source, add the core material and MP₂O₇ powder to the XPO₄ suspension containing a carbon source, mixing them, after sintering, a positive electrode active material is obtained. M and X are independently one or more selected from Li, Fe, Ni, Mg, Co, Cu, Zn, Ti, Ag, Zr, Nb, and Al. The positive electrode active material has a core-shell structure, which includes a core and a shell covering the core. The shell includes a first coating layer covering the core and a second coating layer covering the first coating layer. The first coating layer includes pyrophosphate MP₂O₇ and phosphate XPO₄, and the second coating layer includes carbon.

The preparation method of the present application does not have special restrictions on the source of materials. Optionally, the core material in the preparation method of the present application may be commercially available or obtained through the method of the present application. Optionally, the core material is prepared by the method described below.

**In** some embodiments, optionally, the step of providing a core material includes the following Step (1) and Step (2).

Step (1), mixing a manganese source, a dopant of element A and an acid in a solvent and stirring to obtain manganese salt particles doped with element A.

Step (2): mixing the manganese salt particles doped with element A with a source of lithium, a source of phosphorus, and a source of element R in a solvent to obtain a slurry, sintering in an inert gas atmosphere to obtain lithium manganese phosphate doped with element A and R. The lithium manganese phosphate doped with element A and R is Li₁₊ₓMn_{1-y}A_{y}P_{1-z}R_{z}O₄, with x ranging from -0.100 to 0.100, y ranging from 0.001 to 0.500, and z ranging from 0.001 to 0.100. The A is selected from Zn, Al, Na, K, Mg, Mo, W, Ti, V, Zr One or more of Fe, Ni, Co, Ga, Sn, Sb, Nb, and Ge, optionally one or more of Fe, Ti, V, Ni, Co, and Mg, wherein R is one or more selected from B, Si, N, and S.

Optionally, step (1) is carried out at a temperature of 20-120°C, and optionally of25-80°C. The stirring in step (1) is carried out under 500-700 rpm for 60-420 minutes, and optionally for 120-360 minutes.

During doping, by controlling the reaction temperature, stirring rate, and mixing time, the uniform distribution of doped elements can be achieved, the lattice defects can be reduced, manganese ion leaching-out can be reduced, and the interface side reactions between the positive electrode active material and non-aqueous electrolytic solution can be reduced, thereby improving the specific capacity and rate performance of the positive electrode active material.

It should be noted that in the present application, the source of a certain element may include one or more of its elemental substances, sulfates, halides, nitrates, organic acids, oxides, or hydroxides, provided that the source can make the preparation method of the present application achieve. As an example, the source of element A is one or more selected from the elemental substances, sulfates, halides, nitrates, organic acids, oxides, or hydroxides of element A. As an example, the source of the element R is one or more selected from the elemental substances of element R, sulfates, halides, nitrates, organic salts, oxides or hydroxides, and inorganic acids of element R. Optionally, the inorganic acid of element R is one or more selected from phosphoric acid, nitric acid, boric acid, silicic acid, or orthosilicic acid.

Optionally, in step (1), the source of manganese is one or more selected from elemental manganese, manganese dioxide, manganese phosphate, manganese oxalate, and manganese carbonate.

Optionally, element A is iron, and optionally, in step (1), the source of iron is one or more selected from ferrous carbonate, ferric hydroxide, or ferrous sulfate.

Optionally, in step (1), the acid is one or more selected from hydrochloric acid, sulfuric acid, nitric acid, phosphoric acid, organic acids such as oxalic acid, and may be oxalic acid. In some embodiments, the acid is a dilute acid with a concentration of no more than 60% by weight.

Optionally, in step (2), the source of lithium is one or more selected from lithium carbonate, lithium hydroxide, lithium phosphate, or lithium dihydrogen phosphate.

Optionally, in step (2), the source of phosphorus is one or more selected from diammonium hydrogen phosphate, ammonium dihydrogen phosphate, ammonium phosphate, and phosphoric acid.

**In** some embodiments, optionally, the solvent as used in the preparation method described in the present application is a solvent commonly used in the art. For example, the solvents in the preparation method of the present application may independently be at least one selected from ethanol or water (such as deionized water).

In some embodiments, the pH of the solution is controlled to be 4-6 during the preparation of manganese salt particles doped with element A. It should be noted that in the present application, the pH of the obtained mixture may be adjusted by methods commonly used in the art, such as adding acids or bases.

In some embodiments, optionally, in step (2), the molar ratio of the manganese salt particles doped with element A, the source of lithium, and the source of phosphorus is 1: (0.5-2.1): (0.5-2.1).

In some embodiments, optionally, in step (2), the sintering condition is as follows: sintering at 600-800°C for 4-10 hours under the protection of an inert gas or a mixture of inert gas and hydrogen gas. As a result, the crystallinity of the sintered material is higher, thereby improving the specific capacity and rate performance of the positive electrode active material.

Optionally, the mixture of inert gas and hydrogen is nitrogen (70-90 volume %) + hydrogen (10-30 volume%).

In some embodiments, optionally, the MP₂O₇ powder is a commercially available product, or optionally, the MP₂O₇ powder is prepared as follows: adding a source of element M and a source of phosphorus to a solvent to give a mixture, adjusting the pH of the mixture to 4-6, stirring and fully reacting, and then drying and sintering, giving M; M is one or more selected from Li, Fe, Ni, Mg, Co, Cu, Zn, Ti, Ag, Zr, Nb, and Al.

Optionally, during the preparation process of MP₂O₇ powder, the drying step is conducted at 100-300°C and optionally at 150-200°C for 4-8 hours.

Optionally, in the preparation process of MP₂O₇ powder, the sintering step is conducted under an inert gas atmosphere for 4-10 hours at 500-800°C and optionally 650-800°C.

In some embodiments, the XPO₄ suspension containing a carbon source may be commercially available, or optionally, prepared as follows: mixing the lithium source, X source, phosphorus source, and carbon source in a solvent, and then heating the reaction mixture to 60-120°C for 2-8 hours to give the XPO₄ suspension containing a carbon source. Optionally, in the process of preparing XPO₄ suspension containing carbon source, the pH of the mixture is adjusted to 4-6.

Optionally, the source of carbon is an organic carbon source, and the organic carbon source is one or more selected from starch, sucrose, glucose, polyvinyl alcohol, polyethylene glycol, and citric acid.

In some embodiments, in the coating step, the mass ratio of element A and element R doped lithium manganese phosphate (core), MP₂O₇ powder, and XPO₄ suspension containing carbon source is 1: (0.001 0.05): (0.001 0.05).

In some embodiments, optionally, in the coating step, the sintering temperature is 500-800°C, and the sintering time is 4-10 hours.

In some embodiments, optionally, the median particle size Dv50 of the primary particle of the bilayer coated lithium manganese phosphate positive electrode active material of the present application is 50-2000nm.

The positive electrode plate of the present application comprises a positive electrode current collector and a positive electrode film layer provided on at least one surface of the positive electrode current collector. In particular, the positive electrode current collector has two surfaces opposite to each other in the direction of its own thickness, and the positive electrode film layer is provided on either or both of two opposite surfaces of the positive electrode current collector. The positive electrode film layer comprises the positive electrode active material of the present application described above.

In some embodiments, optionally, the positive electrode active material is present in the positive electrode film layer in a content of 10 wt% or more, based on the total weight of the positive electrode film layer. More optionally, the positive electrode active material is present in the positive electrode film layer in a content of from 90 wt% to 99.5wt%, based on the total weight of the positive electrode film layer.

Under the condition that the content of the positive electrode active material is within the above ranges, it is beneficial to fully utilize the advantages of the positive electrode active material of the present application.

The positive electrode film layer does not exclude other positive electrode active materials than the positive electrode material of the present application. For example, in some embodiments, the positive electrode film layer may further comprise at least one of a lithium transition metal oxide and a modified compound thereof. By way of example, the other positive electrode materials may include at least one of lithium cobalt oxide, lithium nickel oxide, lithium manganese oxide, lithium nickel cobalt oxide, lithium manganese cobalt oxide, lithium nickel manganese oxide, lithium nickel cobalt manganese oxide, lithium nickel cobalt aluminum oxide, and respective modified compounds thereof.

In some embodiments, the positive electrode film layer optionally further comprises a positive electrode conductive agent. The present application does not specifically limit the type of the positive electrode conductive agent, and as an example, the positive electrode conductive agent includes at least one of superconducting carbon, conductive graphite, acetylene black, carbon black, Ketjen black, carbon dots, carbon nanotubes, graphene, and carbon nanofibers.

In some embodiments, the positive electrode film layer optionally further comprises a binder. The present application does not specifically limit the type of the binder, and as an example, the positive electrode binder may include at least one of polyvinylidene fluoride (PVDF), polytetrafluoroethylene (PTFE), vinylidene fluoride-tetrafluoroethylene-propylene terpolymer, vinylidene fluoride-hexafluoropropylene-tetrafluoroethylene terpolymer, tetrafluoroethylene-hexafluoropropylene copolymer, and fluorine-containing acrylate-based resin.

In some embodiments, the positive electrode current collector may be a metal foil or a composite current collector. As an example of a metal foil, an aluminum foil may be used. The composite current collector may include a polymeric material matrix and a metallic material layer formed on at least one surface of the polymeric material matrix. As an example, the metallic material may be at least one selected from aluminum, aluminum alloy, nickel, nickel alloy, titanium, titanium alloy, silver, and silver alloy. As an example, the polymeric material matrix may be selected from polypropylene (PP), polyethylene terephthalate (PET), polybutylene terephthalate (PBT), polystyrene (PS), polyethylene (PE), etc.

The positive electrode film layer is usually made by applying a positive electrode slurry to the positive collector followed by drying and cold pressing. The positive electrode slurry is usually formed by dispersing the positive electrode active material, an optional conductive agent, an optional binder, and any other components in a solvent and stirring. The solvent may be N-methylpyrrolidone (NMP), but is not limited thereto.

### [Negative electrode plate]

In some embodiments, the negative electrode plate comprises a negative electrode current collector and a negative electrode film layer provided on at least one surface of the negative electrode current collector and comprising a negative electrode active material. For example, the negative electrode current collector has two surfaces opposite to each other in the direction of its own thickness, and the negative electrode film layer is provided on either or both of two opposite surfaces of the negative electrode current collector.

The negative electrode active material may be a negative electrode active material known in the art for use in secondary batteries. By way of example, the negative electrode active material includes, but is not limited to, at least one of natural graphite, artificial graphite, soft carbon, hard carbon, silicon-based material, tin-based material, and lithium titanate. The silicon-based material may include at least one of elemental silicon, a silicon oxide, a silicon carbon composite, a silicon nitrogen composite, a silicon alloy material. The tin-based materials may include at least one of elemental tin, a tin oxide, and a tin alloy material. The present application is not limited to these materials, and other conventionally known materials that can be used as negative electrode active materials for secondary batteries may also be used. These negative electrode active materials can be used alone, or in combination of two or more materials.

In some embodiments, the negative electrode film layer may also optionally comprise a negative electrode conductive agent. In the present application, the type of the negative conductive agent is not particularly limited and, as an example, the negative conductive agent may include at least one of superconducting carbon, conductive graphite, acetylene black, carbon black, Ketjen black, carbon dots, carbon nanotubes, graphene, and carbon nanofibers.

In some embodiments, the negative electrode film layer may further optionally comprise a negative electrode binder. In the present application, the type of the negative binder is not particularly limited and, as an example, the negative electrode binder may include at least one of styrene-butadiene rubber (SBR), water-soluble unsaturated resin SR-1B, aqueous acrylic resins (e.g., polyacrylic acid PAA, polymethacrylic acid PMAA, polyacrylic acid sodium salt PAAS), polyacrylamide (PAM), polyvinyl alcohol (PVA), sodium alginate (SA), and carboxymethyl chitosan (CMCS).

In some embodiments, the negative electrode film layer may optionally also include other additives. As an example, the other additives may include thickeners, e.g., sodium carboxymethyl cellulose (CMC), and PTC thermistor material, etc.

In some embodiments, the negative electrode current collector may be a metal foil or a composite collector. As an example of a metal foil, a copper foil may be used. The composite collector may include a polymeric material matrix and a metallic material layer formed on at least one surface of the polymeric material matrix. As an example, the metallic material may be selected from at least one of copper, copper alloy, nickel, nickel alloy, titanium, titanium alloy, silver, and silver alloy. As an example, the polymer material matrix may be selected from polypropylene (PP), polyethylene terephthalate (PET), polybutylene terephthalate (PBT), polystyrene (PS), polyethylene (PE), etc.

The negative electrode film layer is usually made by applying a negative electrode slurry to the negative electrode current collector followed by drying and cold pressing. The negative electrode slurry is usually formed by dispersing the negative active material, an optional conductive agent, an optional binder, and any other optional additives in a solvent and stirring. The solvent may be N-methylpyrrolidone (NMP) and deionized water, but is not limited thereto.

The negative electrode plate does not exclude other additional functional layers than the negative electrode film layer. For example, in some embodiments, the negative electrode plate described in the present application further comprises a conductive primer (e.g. being composed of a conductive agent and a binder) sandwiched between the negative electrode current collector and the negative electrode film layer and disposed on the surface of the negative electrode current collector. In some other embodiments, the negative electrode plate described in the present application further comprises a protective layer covering the surface of the negative electrode film layer.

### [Separator]

There is no particular limitation on the type of separator in the present application, and any well-known porous-structure separator with good chemical stability and mechanical stability may be used.

In some embodiments, materials of the separator may be at least one of glass fibers, non-woven fabrics, polyethylene, polypropylene and polyvinylidene fluoride. The separator may be a single-layer film or a multi-layer composite film. When the separator is a multi-layer composite film, materials of each layer may be the same or different.

In some embodiments, the positive electrode plate, the separator, and the negative electrode plate may be made into an electrode assembly by a winding process or a stacking process.

### [Non-aqueous electrolytic solution]

The secondary battery comprises a non-aqueous electrolytic solution, which is a bridge for the passage of lithium ions in the secondary battery, takes the burden of transporting lithium ions between the positive and negative electrodes in the secondary battery, and plays a crucial role in capacity exertion, cycling performance, storage performance, rate performance and safety performance of the secondary battery.

At present, the most widely commercialized non-aqueous electrolytic solution system is a mixed carbonate solution of lithium hexafluorophosphate. However, lithium hexafluorophosphate has poor thermal stability in high temperature environments and will decompose to form PF₅ at higher temperatures. PF₅ has strong Lewis acidity and can interact with lone pair electrons on oxygen atoms in organic solvent molecules, causing organic solvents to decompose; In addition, PF₅ has a high sensitivity to trace amounts of water in non-aqueous electrolytic solutions. When it encounters water, it generates HF, which increases the acidity of the non-aqueous electrolytic solution and can easily damage the coating layer on the surface of the positive electrode active material. In particular, it is easy to damage the first coating layer including pyrophosphate and phosphate, thereby accelerating the leaching-out of manganese ions, and affecting the cycling and storage performance of the secondary battery. In addition, the carbon in the second coating layer will catalyze the oxidation and decomposition of non-aqueous electrolytic solutions under high voltage, further increasing the irreversible consumption of active lithium ions.

The inventors further conducted extensive research by cleverly adding the first additive represented by Formulae 1-A to 1-D to the non-aqueous electrolytic solution, which can reduce the leaching-out of the first coating layer, reduce the catalytic oxidation effect of the second coating layer, and significantly improve the cycling and storage performance of the secondary battery.

In particular, the non-aqueous electrolytic solution of the present application comprises a first additive including one or more of the compounds shown in formulae 1-A to 1-D,

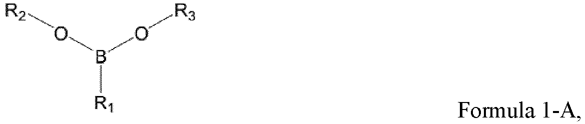

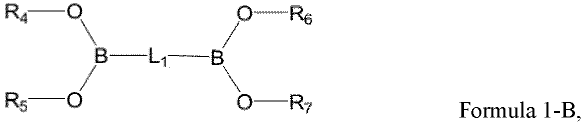

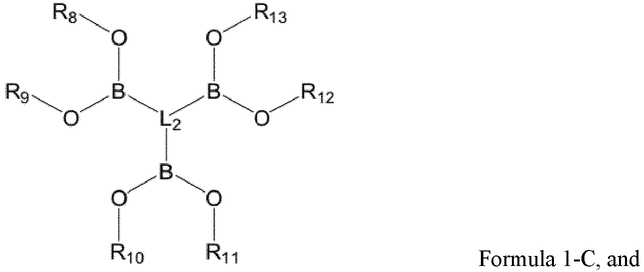

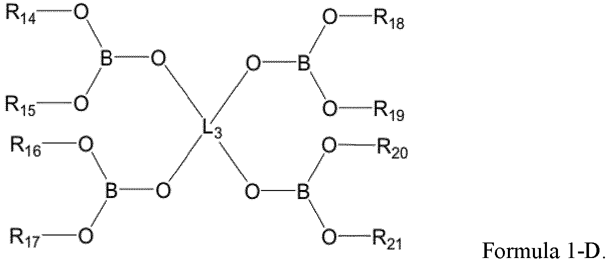

R₁ represents a hydrogen atom or at least one selected from the group consisting of hydroxyl, C1-C18 monovalent alkyl, C1-C18 monovalent alkoxy, C2-C18 monovalent alkoxy alkyl, C3-C18 monovalent cycloalkyl, C2-C18 monovalent heterocyclic alkyl, C6-C18 monovalent aryl, C7-C18 monovalent aryl alkyl, C7-C18 monovalent alkylaryl, C6-C18 monovalent aryloxy, C7-C18 monovalent aryloxy alkyl, C12-C18 monovalent aryl ether group, C2-C18 monovalent heteroaryl, C3-C18 monovalent heteroaryl alkyl, C3-C18 monovalent alkyl heteroaryl, and C1-C18 monovalent silyl.

R₂ to R₂₁ each independently represent a hydrogen atom or at least one selected from the group consisting of C1-C18 monovalent alkyl, C2-C18 monovalent alkoxyalkyl, C3-C18 monovalent cycloalkyl, C2-C18 monovalent oxocycloalkyl, C6-C18 monovalent aryl, C7-C18 monovalent aryl alkyl, C7-C18 monovalent alkylaryl, C7-C18 monovalent aryloxyalkyl, C12-C18 monovalent aryl ether group, C2-C18 monovalent heteroaryl, C3-C18 monovalent heteroaryl alkyl, C3-C18 monovalent alkyl heteroaryl, and C1-C18 monovalent silyl.

When R₂ and R₃ both are hydrogen atoms, R₁ is not hydroxyl.

R₂ and R₃ can further bond with each other to form a ring structure, R₄ and R₅ can further bond with each other to form a ring structure, R₆ and R₇ can further bond with each other to form a ring structure, R₈ and R₉ can further bond with each other to form a ring structure, R₁₀ and R₁₁ can further bond with each other to form a ring structure, R₁₂ and R₁₃ can further bond with each other to form a ring structure, R₁₄ and R₁₅ can further bond with each other to form a ring structure, R₁₆ and R₁₇ can further bond with each other to form a ring structure, R₁₈ and R₁₉ can further bond with each other to form a ring structure, and R₂₀ and R₂₁ can further bond with each other to form a ring structure.

L₁ represents an oxygen atom or at least one selected from the group consisting of C1-C18 divalent alkyl, C1-C18 oxodialkyl, C6-C18 divalent cycloalkyl, C6-C18 divalent oxo cycloalkyl, C6-C18 divalent aryl, C7-C18 divalent arylalkyl, C7-C18 divalent alkyl aryl, C6-C18 divalent aryloxy, C7-C18 divalent aryloxyalkyl, C12-C18 divalent aryl ether group, C2-C18 divalent heteroaryl, C3-C18 divalent heteroaryl alkyl, and C3-C18 divalent alkyl heteroaryl.

L₂ represents at least one selected from the group consisting of C1-C18 trivalent alkyl, C1-C18 oxo trivalent alkyl, C6-C18 trivalent cycloalkyl, C6-C18 trivalent oxocycloalkyl, C6-C18 trivalent aryl, C7-C18 trivalent arylalkyl, C7-C18 trivalent alkylaryl, C6-C18 trivalent aryloxy, C7-C18 trivalent aryloxyalkyl, C12-C18 trivalent aryl ether group, C3-C18 trivalent heteroaryl, C3-C18 trivalent heteroaryl alkyl, and C3-C18 trivalent alkyl heteroaryl.

L₃ represents at least one selected from the group consisting of C1-C18 tetravalent alkyl, C1-C18 oxo tetravalent alkyl, C6-C18 tetravalent cycloalkyl, C6-C18 tetravalent oxo cycloalkyl, C6-C18 tetravalent aryl, C7-C18 tetravalent arylalkyl, C7-C18 tetravalent alkylaryl, C7-C18 tetravalent aryloxyalkyl, C12-C18 tetravalent aryl ether group, C4-C18 tetravalent heteroaryl, C4-C18 tetravalent heteroaryl, and C4-C18 tetravalent alkyl heteroaryl.

Under the condition that the non-aqueous electrolytic solution contains the first additive represented by Formulae 1-A to 1-D, it can form a dense and stable interface film on the surface of the positive active material during the secondary battery charging process, reduce the leaching-out of the first coating layer, reduce the leaching-out of manganese ions, reduce the catalytic oxidation of the second coating layer under high voltage, and reduce the consumption of non-aqueous electrolytic solution and active lithium ions, This can significantly improve the cycling and storage performance of secondary batteries, especially under high temperature and high voltage conditions.

The first additive comprises one or more of the compounds represented by Formulae 1-A to 1-D. Specifically, in some embodiments, the first additive includes one or more of the compounds represented by formula 1-A; in some embodiments, the first additive includes one or more of the compounds represented by Formula 1-B; in some embodiments, the first additive includes one or more of the compounds represented by Formula 1-C; in some embodiments, the first additive includes one or more of the compounds represented by Formula 1-D; in some embodiments, the first additive includes any two, three, or four of the compounds represented by Formulae 1-A to 1-D.

In some embodiments of the present application, optionally, R₂ and R₃ bond with each other to form a ring structure. More optionally, R₂ and R₃ bond with each other to form a ring structure with five or more members.

In some embodiments of the present application, at least one of R₁ to R₃ represents C3-C18 monovalent cycloalkyl, C2-C18 monovalent oxocycloalkyl, C6-C18 monovalent aryl, C7-C18 monovalent arylalkyl, C7-C18 monovalent alkylaryl, C7-C18 monovalent aryloxyalkyl, C12-C18 monovalent aryl ether group, C2-C18 monovalent heteroaryl, C3-C18 monovalent heteroarylalkyl, or C3-C18 monovalent alkyl heteroaryl; and optionally, R₁ to R₃ independently represent C6-C18 monovalent aryl, C7-C18 monovalent arylalkyl, C7-C18 monovalent alkylaryl, C7-C18 monovalent aryloxyalkyl, C12-C18 monovalent aryl ether group, C2-C18 monovalent heteroaryl group, C3-C18 monovalent heteroaryl group, or C3-C18 monovalent alkyl heteroaryl group.

When R₁ to R₃ represent the above substituents, it is helpful to form an interface film containing cross-linked macromolecular polymer on the surface of the positive active material, further reduce the leaching-out of the first coating layer, further reduce the catalytic oxidation of the second coating layer, reduce the leaching-out of manganese ions and the consumption of non-aqueous electrolytic solution and active lithium ions, so as to further enhance the improvement effect on the cycling performance and storage performance of the secondary battery.

As an example, the first additive comprises at least one of the following compounds:

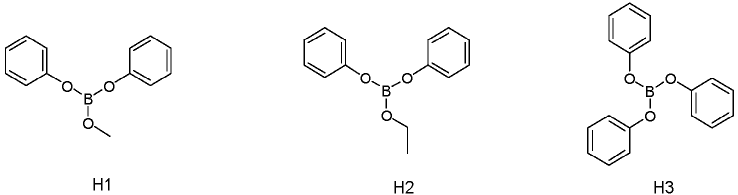

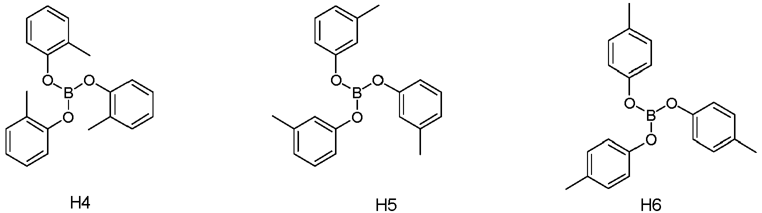

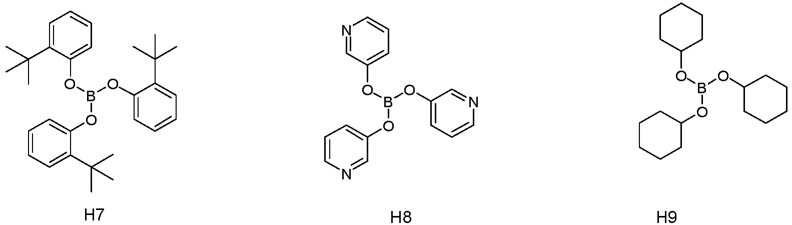

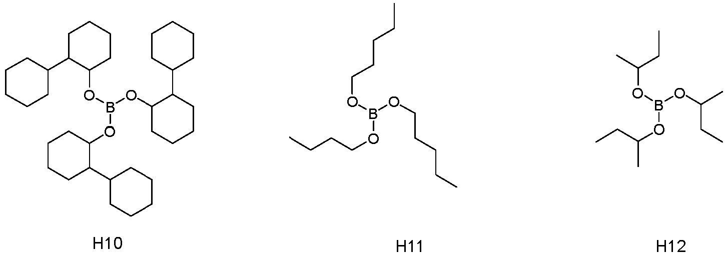

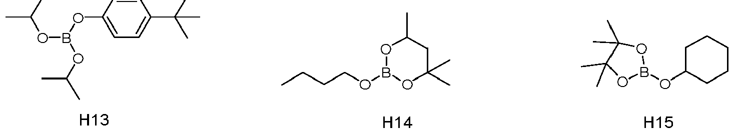

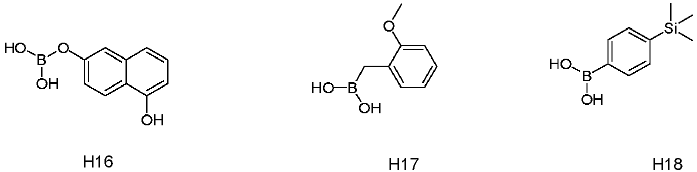

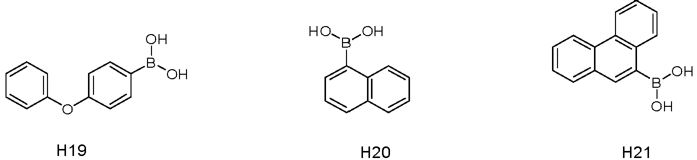

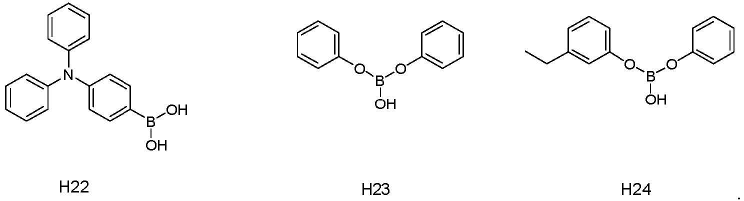

Optionally, the compound of Formula 1-A comprises at least one selected from the group consisting of H1 to H10, and H13 to H15, and more optionally, the compound of Formula 1-A comprises at least one selected from the group consisting of H1 to H8.

In some embodiments, optionally, R₄ to R₇ represent hydrogen atoms, and L₁ represents C6-C18 divalent cycloalkyl, C6-C18 divalent oxoalkyl, C6-C18 divalent aryl, C7-C18 divalent arylalkyl, C7-C18 divalent alkylaryl, C6-C18 divalent aryloxy, C7-C18 divalent aryloxyalkyl, C12-C18 divalent aryl ether gropu, C2-C18 divalent heteroaryl, C3-C18 divalent heteroarylalkyl, or C3-C18 divalent alkyl heteroaryl. Optionally, R₄ to R₇ represent hydrogen atoms, and L₁ represents C6-C18 divalent aryl, C7-C18 divalent arylalkyl, C7-C18 divalent alkylaryl, C6-C18 divalent aryloxy, C7-C18 divalent aryloxyalkyl, C12-C18 divalent aryl ether group, C2-C18 divalent heteroaryl group, C3-C18 divalent heteroaryl or C3-C18 divalent alkyl heteroaryl. Optionally, R₄ to R₇ represent hydrogen atoms, and L₁ represents divalent phenyl, divalent diphenyl, divalent triphenyl, divalent tetraphenyl, divalent naphthyl, divalent anthracene, divalent phenyl, divalent pyrene, or divalent fluorene.

In some embodiments, R₄ and R₅, and R₆ and R₇, at least one pair of them, bond to form a ring structure. For example, R₄ and R₅ bond to form a ring structure, or R₆ and R₇ bond to form a ring structure, or R₄ and R₅ bond to form a ring structure and R₆ and R₇ bond to form a ring structure.

In some embodiments, optionally, R₄ to R₇ are the same and are not hydrogen atoms.

In some embodiments, optionally, L1 represents an oxygen atom, and R₄ to R₇ each independently represent C3-C18 monovalent cycloalkyl, C2-C18 monovalent oxocyclic alkyl, C6-C18 monovalent aryl, C7-C18 monovalent arylalkyl, C7-C18 monovalent alkylaryl, C7-C18 monovalent aryloxyalkyl, C12-C18 monovalent aryl ether, C2-C18 monovalent heteroaryl, C3-C18 monovalent heteroaryl, or C3-C18 monovalent alkyl heteroaryl. Optionally, L₁ represents an oxygen atom, and R₄ to R7 each independently represent C6-C18 monovalent aryl, C7-C18 monovalent arylalkyl, C7-C18 monovalent alkylaryl, C7-C18 monovalent aryloxyalkyl, C12-C18 monovalent aryl ether group, C2-C18 monovalent heteroaryl, C3-C18 monovalent heteroaryl alkyl, or C3-C18 monovalent alkyl heteroaryl group.

As an example, the first additive comprises at least one of the following compounds:

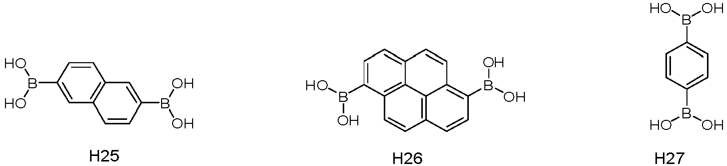

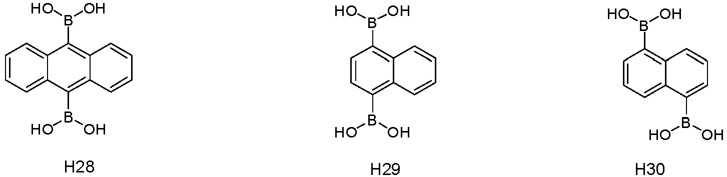

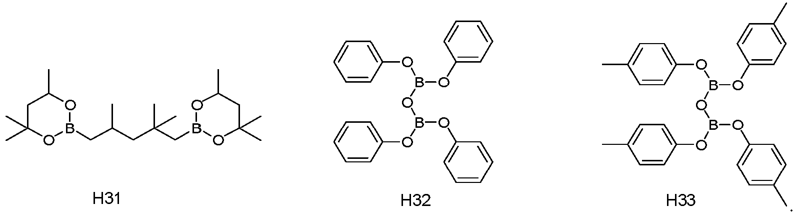

Optionally, the compounds represented by Formulas 1-B include at least one of H31 to H33. Optionally, the compounds represented by Formulas 1-B include at least one of H32 to H33.

In some embodiments, at least one group of R₈ and R₉, R₁₀ and R₁₁, R₁₂ and R₁₃ can optionally bond to form a ring structure. For example, R₈ and R₉ bond to form a ring structure, R₁₀ and R₁₁ bond to form a ring structure, R₁₂ and R₁₃ bond to form a ring structure, or any two or three groups of R₈ and R₉, R₁₀ and R₁₁, R₁₂ and R₁₃ bond to form a ring structure.

In some embodiments, optionally, R₈ to R₁₃ each independently represent C1-C18 monovalent alkyl, C2-C18 monovalent alkoxyalkyl, C3-C18 monovalent cycloalkyl, C2-C18 monovalent oxo cycloalkyl, C6-C18 monovalent aryl, C7-C18 monovalent arylalkyl C7-C18 monovalent alkylaryl, C7-C18 monovalent aryloxyalkyl, C12-C18 monovalent aryl ether, C2-C18 monovalent heteroaryl, C3-C18 monovalent heteroarylalkyl, or C3-C18 monovalent alkyl heteroaryl.

In some embodiments, optionally, L₂ represents C6-C18 trivalent cycloalkyl, C6-C18 trivalent oxoalkyl, C6-C18 trivalent aryl, C7-C18 trivalent arylalkyl, C7-C18 trivalent alkylaryl, C6-C18 trivalent aryloxy, C7-C18 trivalent aryloxyalkyl, C12-C18 trivalent aryl ether, C3-C18 trivalent heteroaryl, C3-C18 trivalent heteroarylalkyl, or C3-C18 trivalent alkyl heteroaryl. More optionally, L₂ represents C6-C18 trivalent aryl, C7-C18 trivalent aryl alkyl, C7-C18 trivalent alkylaryl, C6-C18 trivalent aryloxy, C7-C18 trivalent aryloxy alkyl, C12-C18 trivalent aryl ether, C3-C18 trivalent heteroaryl, C3-C18 trivalent heteroaryl alkyl, or C3-C18 trivalent alkyl heteroaryl. Optionally, L₂ represents trivalent phenyl, trivalent phenylalkyl, and trivalent alkylphenyl.

As an example, the compound of Formula 1-C comprises at least one of the following compounds:

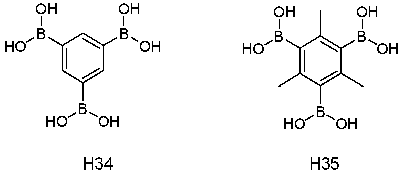

In some embodiments, optionally, R₁₄ and R₁₅, R₁₆ and R₁₇, R₁₈ and R₁₉, R₂₀ and R₂₁, at least one pair of them, bond to form a ring structure. For example, R₁₄ and R₁₅ bond to form a ring structure, or R₁₆ and R₁₇ bond to form a ring structure, or R₁₈ and R₁₉ bond to form a ring structure, or R₂₀ and R₂₁ bond to form a ring structure, or any two, three, or four groups of R₁₄ and R₁₅, R₁₆ and R₁₇, R₁₈ and R₁₉, R₂₀ and R₂₁ bond to form a ring structure.

In some embodiments, optionally, R₁₄ to R₂₁ each independently represent C1-C18 monovalent alkyl, C2-C18 monovalent alkoxyalkyl, C3-C18 monovalent cycloalkyl, C2-C18 monovalent oxoalkyl, C6-C18 monovalent aryl, C7-C18 monovalent arylalkyl, C7-C18 monovalent alkylaryl, C7-C18 monovalent aryloxyalkyl, C12-C18 monovalent aryl ether, C2-C18 monovalent heteroaryl, C3-C18 monovalent heteroarylalkyl, or C3-C18 monovalent alkyl heteroaryl.

In some embodiments, optionally, L₃ represents C6-C18 tetravalent cycloalkyl, C6-C18 tetravalent oxoalkyl, C6-C18 tetravalent aryl, C7-C18 tetravalent arylalkyl, C7-C18 tetravalent alkylaryl, C7-C18 tetravalent aryloxyalkyl, C12-C18 tetravalent aryl ether, C4-C18 tetravalent heteroaryl, C4-C18 tetravalent heteroarylalkyl, or C4-C18 tetravalent alkyl heteroaryl. More optionally, L₃ represents C6-C18 tetravalent aryl, C7-C18 tetravalent aryl alkyl, C7-C18 tetravalent alkylaryl, C7-C18 tetravalent aryloxyalkyl, C12-C18 tetravalent aryl ether, C4-C18 tetravalent heteroaryl, C4-C18 tetravalent heteroaryl alkyl, or C4-C18 tetravalent alkyl heteroaryl. Optionally, L₃ represents tetravalent phenyl, tetravalent biphenyl, tetravalent triphenyl, or tetravalent tetraphenyl.

As an example, the compound of Formula 1-D comprises at least one of the following compounds:

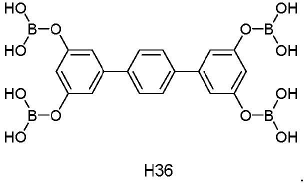

The inventors have found in the research process that taking at least one of the above compounds H1 to H36 as the first additive can form a more compact and stable interface film on the surface of the positive active material, which helps to further reduce the leaching-out of the first coating layer and reduce the catalytic oxidation of the second coating layer, and thus further reduce the leaching-out of manganese ions and the consumption of non-aqueous electrolytic solution and active lithium ions; this can further enhance the improvement effect on the cycling performance and storage performance of secondary batteries.

Optionally, the first additive comprises at least one selected from the group consisting of H1 to H10, H13 to H15, H31 to H33, and more optionally, the first additive comprises at least one selected from the group consisting of H1 to H8 and H32 to H33. These first additives help to form an interface film containing cross-linked macromolecular polymer on the surface of the positive active material, further reduce the leaching-out of the first coating layer and reduce the catalytic oxidation of the second coating layer, and thus further reduce the leaching-out of manganese ions and the consumption of non-aqueous electrolytic solution and active lithium ions, which can further enhance the improvement of the secondary battery cycling performance and storage performance.

During the research process, the inventors also found that when the non-aqueous electrolytic solution contains too much first additive, the positive electrode interface impedance significantly increases, which affects the capacity exertion and rate performance of the secondary battery. Therefore, the content of the first additive in non-aqueous electrolytic solution should not be too high. In some embodiments, the content of the first additive is W1% by weight, with W1 ranging from 0.01 to 20, optionally from 0.1 to 10, and more optionally from 0.3 to 5, based on the total weight of the non-aqueous electrolytic solution. When the content of the first additive is within an appropriate range, it can reduce the leaching-out of the first coating layer, reduce the catalytic oxidation effect of the second coating layer, and avoid the deterioration of the positive electrode interface impedance, thereby significantly improving the cycling and storage performance of the secondary battery, without affecting the capacity exertion and rate performance of the secondary battery.

In some embodiments, the non-aqueous electrolytic solution also includes a second additive, which includes one or more of sulfonic acid lactones, cyclic sulfates, lithium difluorophosphate (LiPO₂F₂), lithium difluorooxalate phosphate (LiDODFP), and lithium difluorooxalate borate (LiDFOB).

The second additive helps to form a low-impedance interface film on the surface of the positive active material. In addition, the second additive can also form a dense and stable interface film on the surface of the negative active material so as to reduce the direct contact between the negative active material and non-aqueous electrolytic solution, and reduce the negative interface side reaction. Therefore, when the non-aqueous electrolytic solution contains both the first and second additives, it helps to significantly improve the cycling and storage performance of the secondary battery, while also enhancing the capacity exertion and rate performance of the secondary battery.

In some embodiments of the present application, the sulfonic acid lactones comprise at least one of the compounds as represented by Formula 2-A,

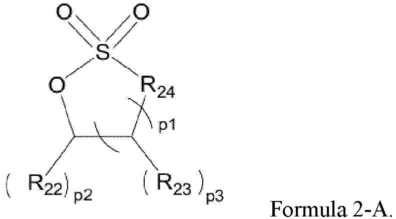

p1 represents 1, 2, or 3, p2 represents 1 or 2, and p3 represents 1 or 2.

R₂₂, at each occurrence, independently represents hydrogen atom, halogen atom, carboxylic ester group (-C(=O)-O-), sulfonic ester group(-S(=O)₂-O-), C1-C6 monovalent alkyl, C1-C6 monovalent haloalkyl, C1-C6 monovalent alkoxy, C1-C6 monovalent haloalkoxy, C2-C6 monovalent alkenyl, or a chain structure or a ring structure formed from more than one of the aforementioned groups linked together with single bonds. Optionally, the ring structure is a cylic sulfonic ester group, a cyclic carboxylic ester group, or oxacycloalkyl. More optionally, the ring structure is a four-membered ring structure, a five-membered ring structure, or a six-membered ring structure.

When R₂₂ represents the ring structure, it is bonded to the ring of Formula 2-A via a single bond or shares a carbon atom with the ring of Formula 2-A to form a spiral-ring compound.

R₂₃, at each occurrence, independently represents hydrogen atom, halogen atom, carboxylic ester group (-C(=O)-O-), sulfonic ester group (-S(=O)₂-O-), C1-C6 monovalent alkyl, C1-C6 monovalent haloalkyl, C1-C6 monovalent alkoxy, C1-C6 monovalent haloalkoxy, and C2-C6 monovalent alkenyl p, or a chain structure or a ring structure formed from more than one of the aforementioned groups linked together with single bonds. Optionally, the ring structure is a cyclic sulfonic ester group, a cyclic carboxylic ester group, or oxacycloalkyl. More optionally, the ring structure is a four-membered ring structure, a five-membered ring structure, or a six-membered ring structure.

When R₂₃ represents the ring structure, it is bonded to the ring of Formula 2-A via a single bond or shares a carbon atom with the ring of Formula 2-A to form a spiral-ring compound.

R₂₄ represents carbonyl (C=O) or C(Y¹)₂, wherein Y₁ at each occurrence independently represents hydrogen atom, halogen atom, carboxylic ester group (-C(=O)-O-), sulfonic ester group (-S(=O)₂-O-), C1-C6 monovalent alkyl group, C1-C6 monovalent haloalkyl group, C1-C6 monovalent alkoxy group, C1-C6 monovalent haloalkoxy group, C2-C6 monovalent alkenyl group, C6-C12 monovalent aryl group, or a combination thereof.

R₂₂ and R₂₃ can further bond with each other to form a ring structure.

Optionally, the sulfonic acid lactone comprises at least one of the following compounds:

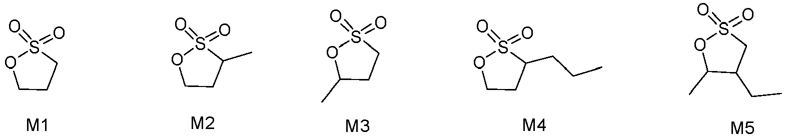

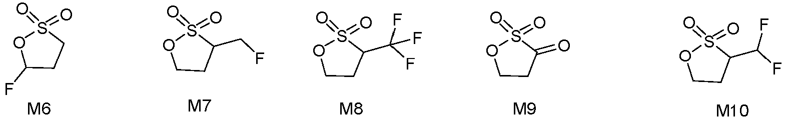

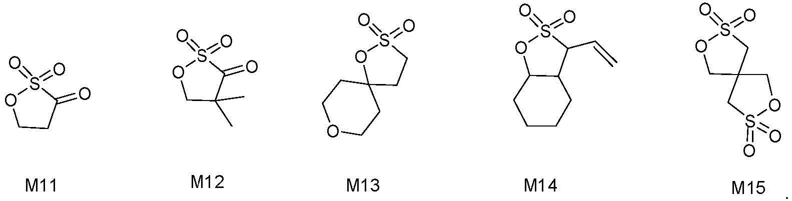

In some embodiments of the present application, the cyclic sulfate comprises at least one of the compounds represented by Formula 2-B,

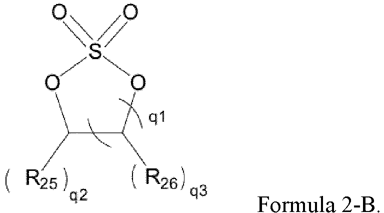

q1 represents 1, 2, or 3, q2 represents 1 or 2, q3 represents 1 or 2.

R₂₅, at each occurrence, independently represents a hydrogen atom, halogen atom, carbonyl oxygen atom, carboxylic ester group (-C(=O)-O-), sulfate ester group (-O-S(=O)₂-O-), C1-C6 monovalent alkyl, C1-C6 monovalent haloalkyl, C1-C6 monovalent alkoxy, C1-C6 monovalent haloalkoxy, C2-C6 monovalent alkenyl, or a chain structure or a ring structure formed from more than one of the aforementioned groups linked together with single bonds. Optionally, the ring structure is a cylic sulfonic ester group, a cyclic carboxylic ester group, or oxacycloalkyl. More optionally, the ring structure is a four-membered ring structure, a five-membered ring structure, or a six-membered ring structure.

When R₂₅ represents the ring structure, it is bonded to the ring of Formula 2-B via a single bond or shares a carbon atom with the ring of Formula 2-B to form a spiral-ring compound.

R₂₆, at each occurrence, independently represents a hydrogen atom, halogen atom, carboxylic ester group (-C(=O)-O-), sulfate group (-O-S(=O)₂-O-), C1-C6 monovalent alkyl group, C1-C6 monovalent haloalkyl, C1-C6 monovalent alkoxy, C1-C6 monovalent haloalkoxy, C2-C6 monovalent alkenyl, or a chain structure or a ring structure formed from more than one of the aforementioned groups linked together with single bonds. Optionally, the ring structure is a cylic sulfonic ester group, a cyclic carboxylic ester group, or oxacycloalkyl. More optionally, the ring structure is a four-membered ring structure, a five-membered ring structure, or a six-membered ring structure.

When R₂₆ represents the ring structure, it is bonded to the ring of Formula 2-B via a single bond or shares a carbon atom with the ring of Formula 2-B to form a spiral-ring compound.

R₂₅ and R₂₆ can further bond with each other to form a ring structure.

Optionally, the cyclic sulfate comprises at least one of the following compounds:

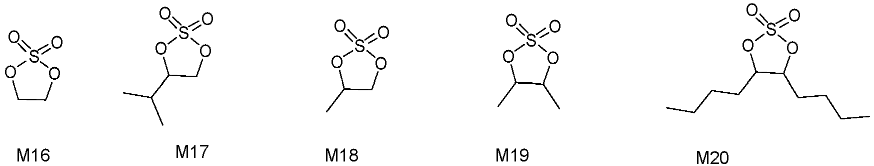

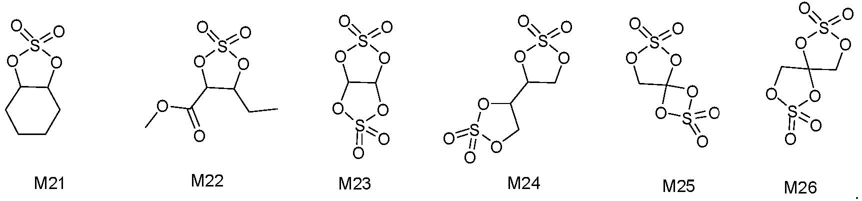

**During** the research process, the inventors have found that when the non-aqueous electrolytic solution contains too much second additive, the positive electrode interface impedance does not decrease but increases, thereby affecting the capacity exertion and rate performance of the secondary battery. Therefore, the content of the second additive in non-aqueous electrolytic solution should not be too high. In some embodiments, the content of the second additive is W2% by weight, with W2 ranging from 0.01 to 20, optionally from 0.1 to 10, and more optionally from 0.3 to 5, based on the total weight of the non-aqueous electrolytic solution. When the content of the second additive is within an appropriate range, it can effectively improve the capacity exertion and rate performance of the secondary battery.

During the research process, the inventors also found that the ratio of the content W1 wt% of the first additive to the content W2 wt% of the second additive affects the electrochemical performance of the secondary battery. In some embodiments, W1/W2 ranges from 0.01 to 20. Optionally, W1/W2 is 0.01 to 10, 0.1 to 10, 0.1 to 8, 0.1 to 5, 0.2 to 5, 0.5 to 5, or 0 to 5. When W1/W2 is within the appropriate range, it can better achieve the synergistic effect between the two, help to form a dense, stable and low-impedance interface film on the surface of the positive active material, further reduce the dissolution of the first coating layer and reduce the catalytic oxidation of the second coating layer, and further reduce the leaching-out of manganese ions and the consumption of non-aqueous electrolytic solution and active lithium ions. This can further enhance the improvement effect on the cycling performance and storage performance of the secondary battery, while enhancing the capacity exertion and rate performance of the secondary battery. And it can effectively avoid the following situations: when W1/W2 is large, the second additive cannot effectively reduce the positive electrode interface impedance, which may affect the capacity exertion and rate performance of the secondary battery; When W1/W2 is small, it cannot significantly reduce the dissolution of the first coating layer and reduce the catalytic oxidation effect of the second coating layer. Therefore, the improvement effect on the cycling performance and storage performance of the secondary battery may not be significant.

In some embodiments, the first coating layer has a coating amount C1% by weight, the second coating layer has a coating amount C2% by weight, the content W1% by weight of the first additive and the content W2 % by weight of the second additive satisfy (W1+W2)/(C1+C2) ranging from 0.001 to 2. Optionally, (W1+W2)/(C1+C2) is 0.01 to 2, 0.01 to 1.5, 0.01 to 1, 0.1 to 1, 0.14 to 1, 0.18 to 1, or 0.22 to 1. Under the condition that (W1+W2)/(C1+C2) is within an appropriate range, it can significantly enhance the improvement effect on the cycling and storage performance of the secondary battery, while also enhancing the capacity exertion and rate performance of the secondary battery. And it can effectively avoid the following situations: when (W1+W2)/(C1+C2) is too small, there is no enough first and second additives to form a film on the surface of the positive electrode active material, which cannot significantly reduce the dissolution of the first coating layer and reduce the catalytic oxidation effect of the second coating layer, and thus the improvement effect on the cycling performance and storage performance of the secondary battery is not significant; When (W1+W2)/(C1+C2) is large, the positive electrode interface impedance increases, which affects the capacity exertion and rate performance of the secondary battery.

In some embodiments, the electrolyte injection coefficient Q0 g/Ah of the secondary battery satisfies Q0 ranging from 2.5 to 4.0.

In some embodiments, the content of the first additive W1%, the content of the second additive W2%, the initial capacity D0 mAh/g of the secondary battery, the electrolyte injection coefficient Q0 g/Ah of the secondary battery, and the specific surface area B m²/g of the positive electrode active material satisfy that [(W1%+W2%) × D0 × Q0]/B is from 0.005 to 10, optionally from 0.01 to 5. When [(W1%+W2%) × D0 × Q0]/B is within the appropriate range, the improvement effect on the cycling and storage performance of the secondary battery can be significantly enhanced, while the capacity exertion and rate performance of the secondary battery are also improved. And the following situations can be avoided: when [(W1%+W2%) × D0 ×Q0]/B is too small, there is no enough first and second additives to form a film on the surface of the positive electrode active material, so the dissolution of the first coating layer cannot be significantly reduced and the catalytic oxidation effect of the second coating layer cannot be further reduced. Therefore, the effect on improving the cycling performance and storage performance of the secondary battery is not significant. When [(W1%+W2%) × D0 × Q0]/B is too large, the positive electrode interface impedance increases, affecting the capacity exertion and rate performance of the secondary battery.

An initial capacity D0 in mAh/g of a secondary battery can be obtained by the following method. A secondary battery is charged to a charge cut-off voltage at 0.33C at a constant temperature of 25°C and then is charged at the constant voltage until the current is less than or equal to 0.05mA. After that, the battery is allowed to stand for 5 minutes, and then is discharged to a discharge cut-off voltage at 0.33C. At this time, a ratio of the discharge capacity to the mass of the positive electrode active material is an initial capacity D0 in mAh/g of the secondary battery. In the test method of the present application, the charge and discharge cut-off voltage of the secondary battery may be 2.5V-4.3V.

In some embodiments, the non-aqueous electrolytic solution also includes a third additive, which includes cyclic carbonate compounds containing unsaturated bonds, halogenated cyclic carbonate compounds, nitrile compounds, phosphonitrile compounds, aromatic hydrocarbons and halogenated aromatic hydrocarbon compounds, isocyanate compounds, anhydride compounds, phosphite ester compounds, phosphate ester compounds, sulfite ester compounds One or more of the dimethyl disulfonate compounds. When the non-aqueous electrolytic solution contains the first additive and the third additive, or the first additive, the second additive and the third additive at the same time, the third additive helps to form a more compact and stable interface film on the surface of the positive and/or negative active materials, thus helping to further improve at least one of the cycling performance, storage performance and magnification performance of the secondary battery. In some embodiments, the content of the third additive is W3% by weight of, with W3 being 10 or less, optionally ranging from 0.1 to 10, and more optionally from 0.5 to 5, based on the total weight of the non-aqueous electrolytic solution.

There are no special restrictions on the types of third additives in the present application, as long as they do not undermine the main purpose of the present application. For example, third additives can be selected from the following specific substances at any ratio.

### Cyclic carbonate compound containing carbon-carbon unsaturated bonds

The cyclic carbonate compound containing carbon-carbon unsaturated bonds may comprise one or more of the compounds shown in Formula 3-1. R₂₇ represents C1-C6 alkylene group with alkenyl or alkynyl substituents on its branched chain, or a substituted or unsubstituted C2-C6 straight-chain alkenylene group, wherein the substituent is one or more selected from a halogen atom, C1-C6 alkyl group, and a C2-C6 alkenyl group.

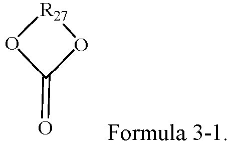

Optionally, the cyclic carbonate compound containing a carbon-carbon unsaturated bond may include, but is not limited to, one or more of the following compounds.

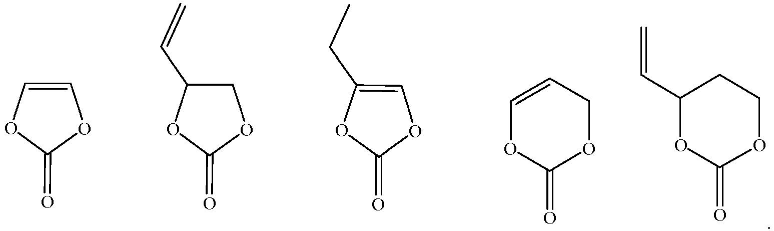

### (b) Halogen-substituted cyclic carbonate compound

The halogen-substituted cyclic carbonate compound may include one or more of the compounds shown in Formula 3-2. R₂₈ represents a halogen-substituted C1-C6 alkylene group, or a halogen-substituted C2-C6 alkenylene group.

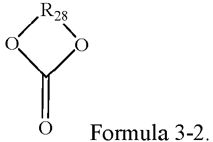

Optionally, the halogen-substituted cyclic carbonate compound may include, without being limited to, one or more of fluoro ethylene carbonate (FEC), fluoro propylene carbonate (FPC), trifluoro propylene carbonate (TFPC), and trans or cis-4,5-difluoro-1,3-dioxolan-2-one (hereinafter, both are collectively referred to as DFEC).

### (c) Nitrile compound

The nitrile compound may be a dinitrile or trinitrile compound. Optionally, the nitrile compound may comprise one or more of the compounds shown in Formula 3-3 and Formula 3-4. R₂₉ represents substituted or unsubstituted C1-C12 alkylene, substituted or unsubstituted C1-C12 oxa-alkylene, substituted or unsubstituted C2-C12 alkenylene, or substituted or unsubstituted C2-C12 alkynylene, and R₃₀ to R₃₂ each independently represent, substituted or unsubstituted C0-C12 alkylene, substituted or unsubstituted C1-C12 oxa-alkylene, substituted or unsubstituted C2-C12 alkenylene, or substituted or unsubstituted C2-C12 alkynylene, wherein the substituent is one or more selected from a halogen atom, a nitrile group, C1-C6 alkyl, C2-C6 alkenyl, and C1-C6 alkoxy.

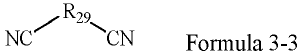

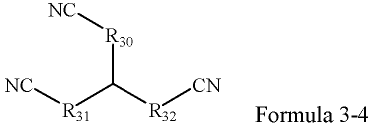

Optionally, the nitrile compound may include one or more of ethanedinitrile, propanedinitrile, butanedinitrile, pentanedinitrile, hexanedinitrile, heptanedinitrile, octanedinitrile, nonanedinitrile, decanedinitrile, undecanedinitrile, dodecanedinitrile, tetramethylsuccinonitrile, methylpentanedinitrile, butadienedinitrile, 2-pentenedinitrile, hex-2-enedinitrile, hex-3-enedinitrile, oct-4-enedinitrile, oct-4-ynedinitrile, 1,2,3-propane-tricarbonitrile, 1,3,5-pentanetricarbonitrile, 1,3,6-hexanetrinitrile.

### (d) Phosphonitrile compound

The phosphonitrile compound may be a cyclic phosphonitrile compound. The cyclic phosphonitrile compound may include one or more of methoxy pentafluorocyclotriphosphonitrile, ethoxy pentafluorocyclotriphosphonitrile, phenoxy pentafluorocyclotriphosphonitrile, and ethoxy heptafluorocyclotetraphosphonitrile. Optionally, the cyclic phosphonitrile compound may include one or more of methoxy pentafluorocyclotriphosphonitrile, ethoxy pentafluorocyclotriphosphonitrile, phenoxy pentafluorocyclotriphosphonitrile. Further optionally, the cyclic phosphonitrile compound may include methoxy pentafluorocyclotriphosphonitrile, ethoxy pentafluorocyclotriphosphonitrile, or a combination thereof.

### (e) Aromatic hydrocarbon and halogenated aromatic hydrocarbon compound

The aromatic hydrocarbon and halogenated aromatic hydrocarbon compound may include one or more of cyclohexylbenzene, fluorinated cyclohexylbenzene compounds (e.g., 1-fluoro-2-cyclohexylbenzene, 1-fluoro-3-cyclohexylbenzene, 1-fluoro-4-cyclohexylbenzene), t-butylbenzene, t-amylbenzene, 1-fluoro-4-t-butylbenzene, biphenyl, terphenyl (ortho-, meta-, para-), diphenyl ether, fluorobenzene, difluorobenzene (ortho-, meta-, para-), anisole, 2,4-difluoroanisole, partial hydrides of terphenyl (e.g., 1,2-dicyclohexylbenzene, 2-phenylbicyclohexyl, 1,2-diphenylcyclohexane, o-cyclohexylbiphenyl). Optionally, the aromatic hydrocarbon and halogenated aromatic hydrocarbon compound may include one or more of biphenyl, terphenyl (ortho-, meta-, para-), fluorobenzene, cyclohexylbenzene, t-butylbenzene, t-amylbenzene. Further optionally, the aromatic hydrocarbon and halogenated aromatic hydrocarbon compound may include one or more of biphenyl, o-terphenyl, fluorobenzene, cyclohexylbenzene, and tert-pentylbenzene.

### (f) Isocyanate compounds

The isocyanate compound may include one or more of methyl isocyanate, ethyl isocyanate, butyl isocyanate, phenyl isocyanate, tetramethylene diisocyanate, hexamethylene diisocyanate, octamethylene diisocyanate, 1,4-phenylene diisocyanate, 2-isocyanatoethyl acrylate, and 2-isocyanatoethyl methacrylate. Optionally, the isocyanate compound may comprise one or more of hexamethylene diisocyanate, octamethylene diisocyanate, 2-isocyanatoethyl acrylate, and 2-isocyanatoethyl methacrylate.

### (g) Acid anhydride compound

The acid anhydride compound may be a chain acid anhydride or a cyclic acid anhydride. Specifically, the acid anhydride compound may include one or more of acetic anhydride, propionic anhydride, succinic anhydride, maleic anhydride, 2-allyl succinic anhydride, glutaric anhydride, itaconic anhydride, and 3-sulfo-propionic anhydride. Optionally, the acid anhydride compound may include one or more of succinic anhydride, maleic anhydride, and 2-allyl succinic anhydride. Further optionally, the acid anhydride compound may include succinic anhydride, 2-allyl succinic anhydride, or a combination thereof.

### (h) Phosphite compound

The phosphite compound may be a silane phosphite compound, and specifically may include one or more of the compounds shown in Formulae 3-5, with R₃₃ to R41 each independently representing halogen-substituted or unsubstituted C1-C6 alkyl.

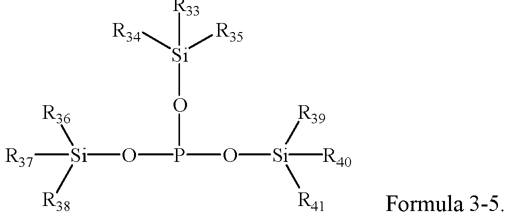

Optionally, the silane phosphite compound may include, but is not limited to, one or more of the following compounds.

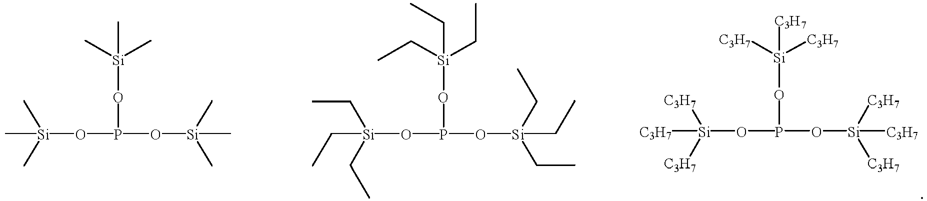

### (i) Phosphate ester compound

The phosphate ester compound may be a silane phosphate ester compound, and specifically may include one or more of the compounds shown in Formula 3-6, with R₄₂ to R₅₀ each independently representing halogen-substituted or unsubstituted C1-C6 alkyl.

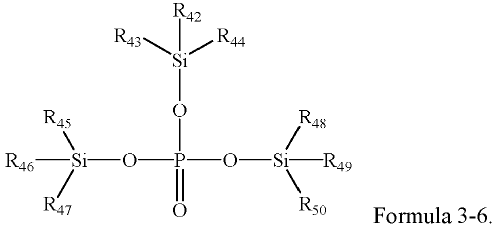

Optionally, the silane phosphate compound may include, but is not limited to, one or more of the following compounds.

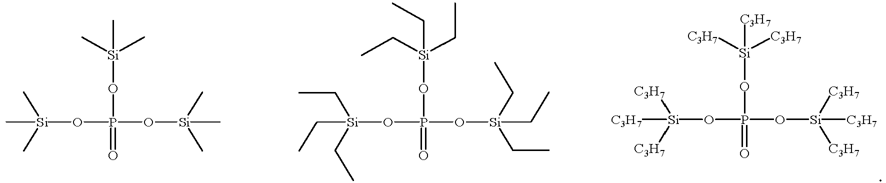

### (j) Sulfite compound

The sulfite compound may be a cyclic sulfite compound, and specifically may include one or more of the compounds shown in Formula 3-7. R₅₁ represents substituted or unsubstituted C1-C6 alkylene, substituted or unsubstituted C2-C6 alkenylene, wherein the substituent is one or more selected from a halogen atom, C1-C3 alkylene, and C2-C4 alkenylene.

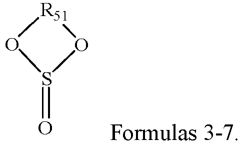

Optionally, the sulfite compound compound may include one or more of ethylene sulfite (ES), propylene sulfite (PS), and butylene sulfite (BS).

### (k) Methylene disulfonate compound

The methylene disulfonate compound may include one or more of the compounds shown in Formula 3-8. R₅₂ to R₅₅ each independently represent a hydrogen atom, a halogen atom, substituted or unsubstituted C1-C10 alkyl group, and substituted or unsubstituted C2-C10 alkenyl, wherein the substituent is one or more selected from a halogen atom, C1-C3 alkyl, and C2-C4 alkenyl.

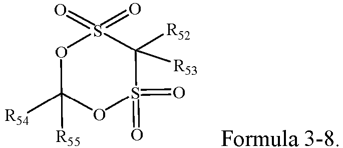

Optionally, the methylene disulfonate compound may include, but is not limited to, one or more of the following compounds.

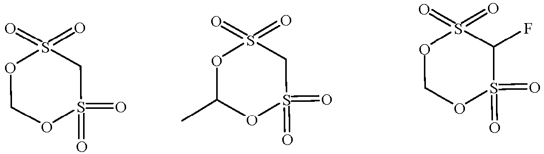

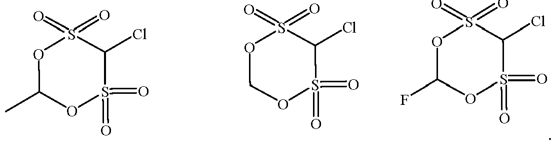

In some embodiments, the third additive may comprise one or more of vinylidene carbonate (VC), vinyl ethylene carbonate (VEC), fluorinated ethylene carbonate (FEC). These third additives are electrochemical reduction additives, and their reduction potential is higher than that of an organic solvent, so that electrochemical reduction can preferentially occur on the surface of the negative electrode active materials to form an interfacial membrane with excellent performances, thereby reducing destruction of the interfacial membrane by the organic solvent, and thus the secondary battery adopting the same can have better electrochemical performance and safety performance.

The non-aqueous electrolytic solution further comprises a lithium salt and an organic solvent. The present application has no particular limitation on the types of the lithium salt and the organic solvent, which may be selected according to actual needs.

As an example, the organic solvent may include one or more of chain carbonate, cyclic carbonate, carboxylic acid ester. Among them, the present application has no specific limitation on the types of the chain carbonate, the cyclic carbonate, and the carboxylic acid ester, which can be selected according to actual needs. Optionally, the organic solvent includes one or more of dimethyl carbonate (DMC), diethyl carbonate (DEC), dipropyl carbonate (DPC), methyl ethyl carbonate (EMC), methyl propyl carbonate (MPC), ethyl propyl carbonate (EPC), ethylene carbonate (EC), propylene carbonate (PC), butylene carbonate (BC), gamma-butyrolactone (GBL), methyl formate (MF), ethyl formate (EF), methyl acetate (MA), ethyl acetate (EA), propyl acetate (PA), methyl propionate (MP), ethyl propionate (EP), propyl propionate (PP), and tetrahydrofuran (THF).

As an example, the lithium salt may include one or more of LiN(CₘF₂ₘ₊₁SO₂)(CₙF₂ₙ₊₁SO₂), LiPF₆, LiBF₄, LiBOB, LiAsF₆, Li(FSO₂)₂N, LiCF₃SO₃, and LiClO₄, with m and n being natural numbers. When the non-aqueous electrolytic solution includes the lithium salt described above, this helps to form a dense, stable and low-impedance interface film on the surface of the positive electrode and/or negative electrode active materials, and effectively improves at least one of cycling performance, storage performance and rate performance of the secondary battery.

The cyclic carbonate has a higher dielectric constant, which facilitates dissociation of lithium salts. In some embodiments, the cyclic carbonate may be present in a content of 20 wt% or more, optionally from 20 wt% to 80 wt%, more optionally from 20 wt% to 50 wt%, based on the total weight of the organic solvent. Optionally, the cyclic carbonate includes one or more of ethylene carbonate (EC), propylene carbonate (PC), and butylene carbonate (BC).

The chain carbonate has a smaller dielectric constant and a weaker ability to dissociate lithium salts, but has a low viscosity and good fluidity and thus it can increase migration rate of lithium ions. In some embodiments, the chain carbonate may be present in a content of 10 wt% or more, optionally from 10 wt% to 80 wt%, based on the total weight of the organic solvent. Optionally, the chain carbonate comprises one or more of dimethyl carbonate (DMC), diethyl carbonate (DEC), dipropyl carbonate (DPC), methyl ethyl carbonate (EMC), methyl propyl carbonate (MPC), and ethyl propyl carbonate (EPC).

Carboxylic acid esters have advantages of low viscosity and high dielectric constant and thus it may enhance conductivity of a non-aqueous electrolytic solution. In some embodiments, the carboxylic acid ester may be present in a content of from 0 wt% to 70 wt%, optionally from 0 wt% to 60 wt%, based on the total weight of the organic solvent. Optionally, the carboxylic acid ester comprises one or more of methyl formate (MF), ethyl formate (EF), methyl acetate (MA), ethyl acetate (EA), propyl acetate (PA), methyl propionate (MP), ethyl propionate (EP), and propyl propionate (PP).

With increasing the content of lithium salts, the total number of migratable lithium ions increases, but at the same time, viscosity of the non-aqueous electrolytic solution will also increase, and migration rate of lithium ions slows down instead. Thus, an optimal value for the content of lithium salts occurs. In some embodiments, the lithium salt may be present in a content of from 6 wt% to 39 wt%, optionally from 10 wt% to 31 wt%, more optionally from 11 wt% to 24 wt%, based on the total weight of the non-aqueous electrolytic solution, and further optionally from 12 wt% to 20 wt%, based on the total weight of the non-aqueous electrolytic solution.

The non-aqueous electrolytic solution of the present application can be prepared in accordance with conventional methods in the field. For example, the additives, the organic solvent, the lithium salt and the like can be mixed well to obtain a non-aqueous electrolytic solution. There is no particular limitation on the order of addition of each material. For example, the additive, the lithium salt and the like can be added to the organic solvent and mixed homogeneously to obtain a non-aqueous electrolytic solution.

**In** the present application, components and their contents in the non-aqueous electrolytic solution can be determined according to methods conventional in the art. For example, they can be determined by gas chromatography-mass spectrometry (GC-MS), ion chromatography (IC), liquid chromatography (LC), nuclear magnetic resonance spectrometry (NMR) and the like.

It should be noted that the non-aqueous electrolytic solution of the present application can also be obtained from a secondary battery. An exemplary method of obtaining a non-aqueous electrolytic solution from a secondary battery comprises the step of discharging the secondary battery to a discharge cut-off voltage and then centrifuging it, after which an appropriate amount of liquid obtained from centrifugation is taken for testing.

### [Outer package]

In some embodiments, the secondary battery may include an outer package. The outer package may be used to encapsulate the electrode assembly and a non-aqueous electrolytic solution.

In some embodiments, the outer package of the secondary battery may be a hard case, such as a hard plastic case, an aluminum case, a steel case, and the like. The outer package of the secondary battery may also be a soft package, such as a pouch-type soft package. Material of the soft bag can be plastic, such as at least one of polypropylene (PP), polybutylene terephthalate (PBT), and polybutylene succinate (PBS).

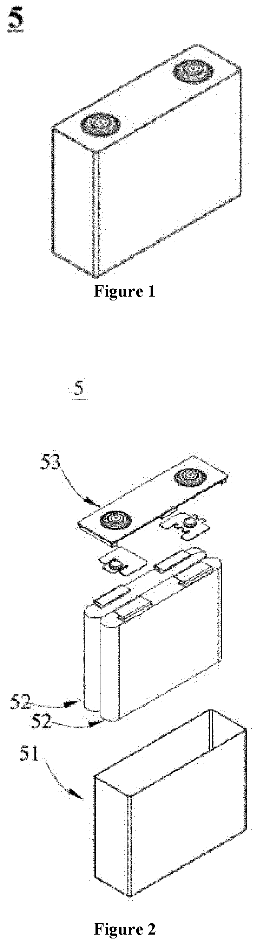

The shape of the secondary battery is not particularly limited in the present application, and it may be cylindrical, square, rectangular or any other shape. FIG. 1 is a schematic diagram of a secondary battery 5 of a rectangular structure as an example.

In some embodiments, as shown in FIG. 2, the outer package may include a casing 51 and a cover plate 53, wherein the casing 51 may include a bottom plate and side plates connected to the bottom plate, and the bottom plate and the side plates enclose an accommodating cavity. The casing 51 has an opening communicating with the accommodating cavity, and the cover plate 53 is used to cover the opening to close the accommodating cavity. A positive electrode plate, a separator, and a negative electrode plate may be made into an electrode assembly 52 by a winding process or a stacking process. The electrode assembly 52 is packaged in the accommodating cavity, and the electrolytic solution is infiltrated in the electrode assembly 52. The number of electrode assemblies 52 contained in the secondary battery 5 may be one or more, and may be adjusted according to requirements.

A process for preparing the secondary battery of the present application is well known in the art. In some embodiments, a positive electrode plate, a separator, a negative electrode plate and a non-aqueous electrolytic solution may be assembled into a secondary battery. As an example, a positive electrode plate, a separator, and a negative electrode plate can be made into an electrode assembly by a winding process or a stacking process, and the electrode assembly can be placed in an outer package and is subjected to drying, to which a non-aqueous electrolytic solution is injected. After vacuum encapsulation, resting, formation, and shaping process, a secondary battery can be obtained.

### Battery Module

**In** some embodiments, the secondary batteries of the present application can be assembled into a battery module, and the number of secondary batteries contained in the battery module can be more than one, and the specific number can be adjusted according to application and capacity of the battery module.

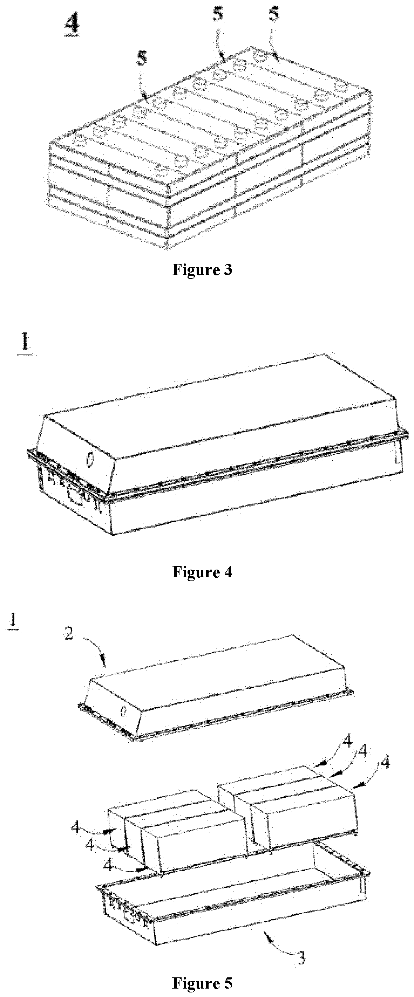

FIG. 3 is a schematic diagram of the battery module 4 as an example. As shown in FIG. 3, in the battery module 4, a plurality of secondary batteries 5 may be arranged in sequence along the longitudinal direction of the battery module 4. Certainly, they can also be arranged in any other manner. Furthermore, a plurality of secondary batteries 5 can be fixed with fasteners.

Optionally, the battery module 4 may further include a housing having an accommodating space in which a plurality of secondary batteries 5 are accommodated.

### Battery pack

**In** some embodiments, the above-mentioned battery modules can also be assembled into a battery pack, and the number of battery modules included in the battery pack can be adjusted according to the application and capacity of the battery pack.

FIGS. 4 and 5 are schematic diagrams of the battery pack 1 as an example. As shown in FIGS. 4 and 5, the battery pack 1 may include a battery case and a plurality of battery modules 4 provided in the battery case. The battery box includes an upper case body 2 and a lower case body 3, and the upper case body 2 is used to cover the lower case body 3 to form a closed space for accommodating the battery modules 4. A plurality of battery modules 4 may be arranged in the battery case in any manner.

### Electrical device

The present application further provides an electrical device comprising at least one of the secondary battery, battery module, and battery pack of the present application. The secondary battery, battery module or battery pack can be used as a power source of the electrical device, and can also be used as an energy storage unit of the electrical device. The electrical device can be, but is not limited to, a mobile device (e.g., a mobile phone, a notebook computer, and the like), an electric vehicle (e.g., a pure electric vehicle, a hybrid electric vehicle, a plug-in hybrid electric vehicle, an electric bicycle, an electric scooter, an electric golf vehicle, an electric truck and the like), an electric train, a ship, a satellite, an energy storage system, and the like.

The electrical device can select a secondary battery, a battery module or a battery pack according to its usage requirements.

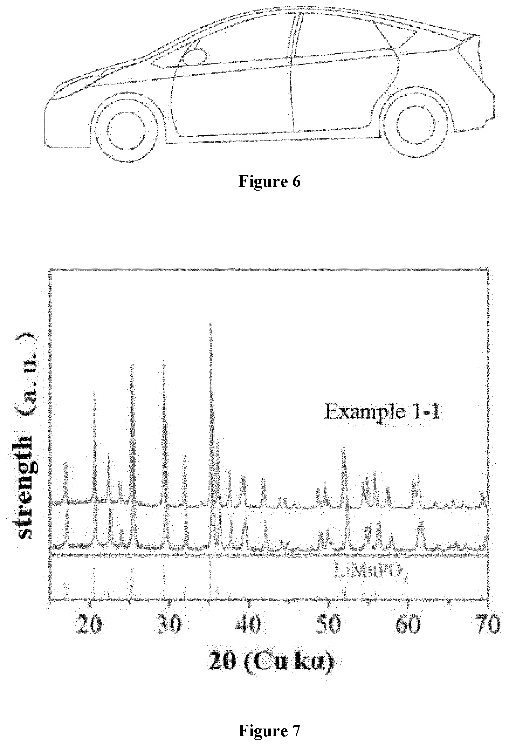

FIG. 6 is a schematic diagrams of an electrical device as an example. The electrical device is a pure electric vehicle, a hybrid electric vehicle, or a plug-in hybrid electric vehicle. In order to meet high power and high energy density requirements of the electrical device, a battery pack or a battery module can be used.

As another example, the electrical device may be a mobile phone, a tablet computer, a notebook computer, and the like. The electric device is generally required to be light and thin, and a secondary battery can be used as a power source.

### Examples

The following examples more specifically describe the content disclosed in the present application, and these examples are only used for explanatory description, because various modifications and changes within the scope of the present disclosure are apparent to those skilled in the art. Unless otherwise stated, all parts, percentages, and ratios described in the following examples are based on mass, and all reagents used in the examples are commercially available or synthesized according to conventional methods and can be directly used without further treatment, and all instruments used in the examples are commercially available.

The sources of raw materials involved in examples of the present application are as follows:

| Name | Chemical Formula | Supplier | Remarkably |
|---|---|---|---|
| Manganese Carbonate | MnCO₃ | Shandong West Asia Chemical Industry Co. | 1Kg |
| Lithium Carbonate | Li₂CO₃ | Shandong West Asia Chemical Industry Co. | 1Kg |
| Magnesium carbonate | MgCO₃ | Shandong West Asia Chemical Industry Co. | 1Kg |
| Zinc Carbonate | ZnCO₃ | Wuhan Xinru Chemical Co. | 25Kg |
| Ferrous Carbonate | FeCO₃ | Xi'an Lanzhiguang Fine Material Co. | 1Kg |
| Nickel Sulfate | NiCO₃ | Shandong West Asia Chemical Industry Co. | 1Kg |
| Titanium Sulfate | Ti(SO₄)₂ | Shandong West Asia Chemical Industry Co. | 1Kg |
| Cobalt Sulfate | CoSO₄ | Xiamen Zhixin Chemical Co. | 500g |
| Vanadium Dichloride | VCl₂ | Shanghai Jin Jin Le Industry Co. | 1Kg |
| Oxalic acid dihydrate | C₂H₂O₄•2H₂O | Shanghai Jin Jin Le Industry Co. | 1Kg |
| Ammonium dihydrogen phosphate | NH₄H₂PO₄ | Shanghai Chengshao Biotechnology Co. | 500g |
| Sucrose | C₁₂H₂₂O₁₁ | Shanghai Yuanye Biotechnology Co. | 100g |
| Dilute sulfuric acid | H₂SO₄ | Shenzhen Haisian Biotechnology Co. | In a mass percentage of 60% |
| Dilute nitric acid | HNO₃ | Anhui Lingtian Fine Chemical Co. | In a mass percentage of 60% |
| Metasilicic acid | H₂SiO₃ | Shanghai Yuanye Biotechnology Co. | 100g |
| Boric acid | H₃BO₃ | Changzhou Qidi Chemical Co., Ltd | 1 Kg |

### I. Preparation of battery

### Example 1-1

### Preparation of positive electrode active material

### (1) Preparation of Co-doped Lithium Manganese Phosphate Core

Preparation of Fe, Co, and V co-doped manganese oxalate: 689.5g of manganese carbonate (calculated as Mn₂O₃, the same below), 455.2g of ferrous carbonate (calculated as FeCO₃, the same below), 4.6g of cobalt sulfate (calculated as CoSO₄, the same below), and 4.9g of vanadium dichloride (calculated as VCl₂, the same below) were thoroughly mixed in a mixer for 6 hours. The mixture was transferred into a reactor, and 5 liters of deionized water and 1260.6g of oxalic acid dihydrate (calculated as C₂H₂O₄.2H₂O, the same below) were added. The reaction kettle was heated to 80°C, stirred at a speed of 600rpm for 6 hours until the reaction terminated (no bubbles generated), giving a Fe, Co, V, and S co-doped manganese oxalate suspension. Then the suspension was filterer, the filter cake was dried at 120°C and ground, giving a Fe, Co, and V co-doped manganese oxalate dihydrate particles with a median particle size Dv50 of 100nm.

Preparation of Fe, Co, V, and S co-doped lithium manganese phosphate: The manganese oxalate dihydrate particles (1793.4g) obtained in the previous step, 369.0g of lithium carbonate (calculated as Li₂CO₃, the same below), 1.6g of dilute sulfuric acid (calculated as 60% H₂SO₄, the same below) with a concentration of 60%, and 1148.9g of ammonium dihydrogen phosphate (calculated as NH₄H₂PO₄, the same below) were added into 20 liters of deionized water, and then the mixture was stirred for 10 hours to give a slurry. The slurry was transferred into a spray drying equipment for spray drying granulation under the drying temperature of 250°C for 4 hours, then the powders were given. Under a protective atmosphere of nitrogen (90 volume%) and hydrogen (10 volume%), the above powder was sintered at 700°C for 4 hours to give 1572.1g of Fe, Co, V, and S co-doped lithium manganese phosphate.

### (2) Preparation of lithium iron pyrophosphate and lithium iron phosphate

Preparation of lithium iron pyrophosphate powder: 4.77g of lithium carbonate, 7.47g of ferrous carbonate, 14.84g of ammonium dihydrogen phosphate, and 1.3g of oxalic acid dihydrate were dissolved in 50mL of deionized water. The mixture having a pH of 5 was stirred for 2 hours to fully react. Then, the reacted solution was heated to 80°C and maintained at this temperature for 4 hours to give a suspension containing Li₂FeP₂O₇. The suspension was filtered, washed with deionized water, and dried at 120°C for 4 hours to give powders. The powders were sintered at 650°C for 8 hours in a nitrogen atmosphere, and then were naturally cooled to room temperature and ground to give Li₂FeP₂O₇ powder.

Preparation of lithium iron phosphate suspension: 11.1g of lithium carbonate, 34.8g of ferrous carbonate, 34.5g of ammonium dihydrogen phosphate, 1.3g of oxalic acid dihydrate, and 74.6g of sucrose (calculated as C₁₂H₂₂O₁₁, the same below) were dissolved in 150mL of deionized water to give a mixture, and then the mixture is stirred for 6 hours to fully react. Then, the reacted solution was heated to 120°C and maintained at this temperature for 6 hours to give a suspension containing LiFePO₄.

### (3) Coating

1572.1g of Fe, Co, V, and S co-doped lithium manganese phosphate and 15.72g of lithium iron pyrophosphate (Li₂FeP₂O₇) powders were added to the lithium iron phosphate (LiFePO₄) suspension prepared in the previous step, then were stirred and mixed, and then were transferred into a vacuum oven to dry at 150°C for 6 hours. Then, the product was dispersed by sand-grinding. After dispersion, the obtained product was sintered in a nitrogen atmosphere at 700°C for 6 hours to give the target product double layered lithium manganese phosphate.

### Preparation of positive electrode plate

The above-prepared double layered lithium manganese phosphate as a positive electrode active material, acetylene black as a conductive agent, and polyvinylidene fluoride (PVDF) as a binder at a weight ratio of 92:2.5:5.5 were added to N-methylpyrrolidone (NMP) under stirring until reaching even mixing, giving a positive electrode slurry. The positive electrode slurry was then applied to an aluminum foil at 0.280 g/1540.25 mm², and then dried, cold pressed, and slit, to give a positive electrode plate.

### Preparation of negative electrode plate

Artificial graphite as a negative active material, hard carbon, acetylene black as a conductive agent, styrene butadiene rubber (SBR) as a binder, sodium carboxymethyl cellulose (CMC) as a thickener at a weight ratio of 90:5:2:2:1 were dissolved in a solvent deionized water, then were stirred and mixed to, give a negative electrode slurry. The negative electrode slurry was applied to a copper foil as a current collector at 0.117 g/1540.25 mm², and then dried, cold pressed, and slit, to give a negative electrode plate.

### Preparation of non-aqueous electrolytic solution

In an argon atmosphere glove box (H₂O<0.1 ppm, O₂<0.1 ppm), ethylene carbonate (EC), diethyl carbonate (DEC) and dimethyl carbonate (DMC) were mixed homogeneously at a volume ratio of 1:1:1 to give an organic solvent, then 12.5 wt% (based on the total weight of the non-aqueous electrolytic solution) of LiPF₆ and 2 wt% (based on the total weight of the non-aqueous electrolytic solution) of compound H3 were dissolved into the organic solvent, after stirring, a non-aqueous electrolytic solution is given.

### Preparation of separator

**A** commercially available PP-PE copolymer microporous film with a thickness of 20 µm and an average pore size of 80 nm was used (available from Trico Electronic Technology, model 20).

### Preparation of full battery

The positive electrode plate, separator and negative electrode plate as prepared above were stacked in order, so that the separator was sandwiched between the positive and negative electrodes for separating, and then they were wound to form an electrode assembly. The electrode assembly was placed in an outer package, which was filled with the above electrolytic solution and was encapsulated, giving a full battery (hereinafter also referred to as "the full"). The full battery had a liquid injection coefficient of 3.5 g/Ah.

### Preparation of button battery

The above-prepared double layered lithium manganese phosphate as a positive electrode active material, PVDF, and acetylene black at a weight ratio of 90: 5: 5 were added to NMP and stirred in a drying room to give a slurry. The above slurry was then applied to an aluminum foil, dried and cold pressed to form a positive electrode plate. The coating amount was 0.2g/cm² and the compaction density was 2.0g/cm³.

Lithium sheet as a negative electrode, the above prepared positive electrode plate, and the non-aqueous electrolytic solution were assembled in a button battery box to form a button battery (hereinafter also referred to as "the button").

### Examples 1-2 to 1-33

Examples 1-2 to 1-33 were conducted according to Example 1-1, except for the preparation of the positive electrode active material, the preparation of the positive electrode plate, the preparation of the negative electrode plate, the preparation of non-aqueous electrolytic solution, the preparation of the separator, and the preparation of the battery.

### Examples 1-2 to 1-6

In Examples 1-2 to 1-6, the co-doped lithium manganese phosphate core was prepared according to Example 1-1, except that vanadium dichloride and cobalt sulfate were not used, and 463.4g of ferrous carbonate, 1.6g of 60% dilute sulfuric acid, 1148.9g of ammonium dihydrogen phosphate, and 369.0g of lithium carbonate were used.

In addition, the lithium iron pyrophosphate and lithium iron phosphate were prepared and the first and second coating layer were applied according to Example 1-1, except that the raw materials as used were adjusted according to the coating amount as show in Table 1, the amounts of Li₂FeP₂O₇/LiFePO₄ in Examples 1-2 to 1-6 are 12.6g/37.7g, 15.7g/47.1g, 18.8g/56.5g, 22.0/66.0g, and 25.1g/75.4g respectively, and the dosage of sucrose in Examples 1-2 to 1-6 was 37.3g.

### Examples 1-7 to 1-10

Examples 1-7 to 1-10 were conducted according to Example 1-3, except that the amounts of sucrose as used in Examples 1-7 to 1-10 were 74.6g, 149.1g, 186.4g, and 223.7g respectively, to achieve corresponding coating amounts of 31.4g, 62.9g, 78.6g, and 94.3g for the carbon layer as the second coating layer.

### Examples 1-11 to 1-14

Examples 1-11 to 1-14 were conducted according to Example 1-7, except that the amounts of raw materials were adjusted according to the coating amount as shown in Table 1 during the preparation of lithium iron pyrophosphate and lithium iron phosphate, so that the dosage of Li₂FeP₂O₇/LiFePO₄ in Examples 1-11 to 1-14 was 23.6g/39.3g, 31.4g/31.4g, 39.3g/23.6g, and 47.2g/15.7g respectively.

### Examples 1-15

Examples 1-15 was conducted according to Example 1-14, except that 492.80g of zinc carbonate was used instead of ferrous carbonate in the preparation of the co-doped lithium manganese phosphate core.

### Examples 1-16 to 1-18

Examples 1-16 to 1-18 were conducted according to Example 1-7, except that in Example 1-16, 466.4g of nickel carbonate, 5.0g of zinc carbonate, and 7.2g of titanium sulfate instead of ferrous carbonate were used in the preparation of co-doped lithium manganese phosphate core, in Example 1-17, 455.2g of ferrous carbonate and 8.5g of vanadium dichloride were used in the preparation of co-doped lithium manganese phosphate core, and Example 1-18, 455.2g of ferrous carbonate, 4.9g of vanadium dichloride and 2.5g of magnesium carbonate were used in the preparation of co-doped lithium manganese phosphate core.

### Examples 1-19 to 1-20

Examples 1-19 to 1-20 were conducted according to Example 1-18, except that in Example 1-19, 369.4g of lithium carbonate and 1.05g of dilute nitric acid instead of dilute sulfuric acid were used in the preparation process of co-doped lithium manganese phosphate core, and in Example 1-20, 369.7g of lithium carbonate and 0.78g of silicic acid instead of dilute sulfuric acid were used in the preparation process of co-doped lithium manganese phosphate core.

### Examples 1-21 to 1-22

Examples 1-21 to 1-22 were conducted according to Example 1-20, except that in Example 1-21, 632.0g of manganese carbonate, 463.30g of ferrous carbonate, 30.5g of vanadium dichloride, 21.0g of magnesium carbonate, and 0.78g of silicic acid were used in the preparation process of co-doped lithium manganese phosphate core, and in Example 1-22, 746.9g of manganese carbonate, 289.6g of ferrous carbonate, 60.9g of vanadium dichloride, 42.1g of magnesium carbonate, and 0.78g of siliceous acid ware used in the preparation process of co-doped lithium manganese phosphate core.

### Examples 1-23 to 1-24

Examples 1-23 to 1-24 were conducted according to Example 1-22, except that in Example 1-23, 804.6g of manganese carbonate, 231.7g of ferrous carbonate, 1156.2g of ammonium dihydrogen phosphate, 1.2g of boric acid (mass fraction 99.5%), and 370.8g of lithium carbonate were used in the preparation process of co-doped lithium manganese phosphate core, and Example 1-24, 862.1g of manganese carbonate, 173.8g of ferrous carbonate, 1155.1g of ammonium dihydrogen phosphate, 1.86g of boric acid (mass fraction 99.5%), and 371.6g of lithium carbonate were used in the preparation process of co-doped lithium manganese phosphate core.

### Examples 1-25

Example 1-25 was conducted according to Example 1-20, except that 370.1g of lithium carbonate, 1.56g of silicic acid, and 1147.7g of ammonium dihydrogen phosphate were used in the preparation of co-doped lithium manganese phosphate core in Examples 1-25.

### Examples 1-26

Example 1-26 was conducted according to Example 1-20, except that 368.3g of lithium carbonate, 4.9g of dilute sulfuric acid with a mass fraction of 60%, 919.6g of manganese carbonate, 224.8g of ferrous carbonate, 3.7g of vanadium dichloride, 2.5g of magnesium carbonate, and 1146.8g of ammonium dihydrogen phosphate were used in the preparation of co-doped lithium manganese phosphate core in Example 1-26.

### Examples 1-27

Example 1-27 was conducted according to Example 1-20, except that 367.9g of lithium carbonate, 6.5g of dilute sulfuric acid with a concentration of 60%, and 1145.4g of ammonium dihydrogen phosphate were used in the preparation of co-doped lithium manganese phosphate core in Example 1-27.

### Examples 1-28 to 1-33

Examples 1-28 to 1-33 were conducted according to Example 1-20, except that 1034.5g of manganese carbonate, 108.9g of ferrous carbonate, 3.7g of vanadium dichloride, and 2.5g of magnesium carbonate were used in the preparation of co-doped lithium manganese phosphate core, the amounts of lithium carbonate in Examples 1-28 to 1-33 were 367.6g, 367.2g, 366.8g, 366.4g, 366.0g, and 332.4g respectively, the amounts of ammonium dihydrogen phosphate in Examples 1-28 to 1-33 were 1144.5g, 1143.4g, 1142.2g, 1141.1g, 1139.9g, and 1138.8g respectively, and the amounts of dilute sulfuric acid with a concentration of 60% in Examples 1-28 to 1-33 were 8.2g, 9.8g, 11.4g, 13.1g, 14.7g, and 16.3g respectively.

### Examples 2-1 to 2-3

Examples 2-1 to 2-3 were conducted according to Example 1-1 in terms of the preparation of the positive electrode plate, the preparation of the negative electrode plate, the preparation of non-aqueous electrolytic solution, the preparation of the separator, and the preparation of the battery, except that the preparation of the positive electrode active material was different.

### Examples 2-1

Example 2-1 was conducted according to Example 1-1, except that in the preparation of lithium iron pyrophosphate (Li₂FeP₂O₇), the sintering temperature was 550°C and the sintering time was 1 hour in the powder sintering step to control the crystallinity of Li₂FeP₂O₇ as 30%, and in the preparation of lithium iron phosphate (LiFePO₄), the sintering temperature was 650°Cand the sintering time of 2 hours in the coating sintering step to control the crystallinity of LiFePO₄ as 30%.

### Examples 2-2

Example 2-2 was conducted according to Example 1-1, except that in the preparation of lithium iron pyrophosphate (Li₂FeP₂O₇), the sintering temperature was 550°Cand the sintering time was 2 hour in the powder sintering step to control the crystallinity of Li₂FeP₂O₇ as 50%, and in the preparation of lithium iron phosphate (LiFePO₄), the sintering temperature was 650°Cand the sintering time of 3 hours in the coating sintering step to control the crystallinity of LiFePO₄ as 50%.

### Examples 2-3

Example 2-3 was conducted according to Example 1-1, except that in the preparation of lithium iron pyrophosphate (Li₂FeP₂O₇), the sintering temperature was 600°Cand the sintering time was 3 hour in the powder sintering step to control the crystallinity of Li₂FeP₂O₇ as 70%, and in the preparation of lithium iron phosphate (LiFePO₄), the sintering temperature was 650°Cand the sintering time of 4 hours in the coating sintering step to control the crystallinity of LiFePO₄ as 70%.

### Examples 3-1 to 3-32

Examples 3-1 to 3-32 were conducted according to Example 1-1 in terms of the preparation of the positive electrode active material, the preparation of the positive electrode plate, the preparation of the negative electrode plate, the preparation of the separator, and the preparation of the battery, except that the preparation process of the aqueous electrolytic solution was different.

### Comparative Examples 1 to 8

Comparative Examples 1 to 8 were conducted according to Example 1-1 in terms of the preparation of the positive electrode plate, the preparation of the negative electrode plate, the preparation of the separator, and the preparation of the battery, except that the preparation of the positive electrode active materials and the preparation of non-aqueous electrolytic solutions were different.

### Comparative Example 1

Preparation of manganese oxalate: 1149.3g of manganese carbonate was added into the reactor, and 5 liters of deionized water and 1260.6g of oxalic acid dihydrate (calculated as C₂H₂O₄ • 2H₂O, the same below) were added. The reactor was heated to 80°C under stirring at a speed of 600rpm for 6 hours until the reaction terminated (no bubbles generated) to give a manganese oxalate suspension. Then the suspension was filtered, the filter cake was dried at 120°C, and then was ground, to give manganese oxalate dihydrate particles with a median particle size Dv50 of 100nm.

Preparation of carbon coated lithium manganese phosphate: 1789.6g of the prepared manganese oxalate dihydrate particles, 369.4g of lithium carbonate (calculated as Li₂CO₃, the same below), 1150.1g of ammonium dihydrogen phosphate (calculated as NH₄H₂PO₄, the same below), and 31g of sucrose (calculated as C₁₂H₂₂O₁₁, the same below) were added into 20 liters of deionized water. The mixture was stirred for 10 hours to give a slurry. The slurry were then transferred into a spray drying equipment for spray drying granulation, the drying was conducted under a temperature of 250°Cfor 4 hours, then the powders were give. Under a protective atmosphere of nitrogen (90 volume%) and hydrogen (10 volume%), the above powders were sintered at 700°C for 4 hours to give carbon-coated lithium manganese phosphate.

Preparation of non-aqueous electrolytic solution: In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 wt% of LiPF₆ (based on the total weight of the non-aqueous electrolytic solution) was added to dissolve in the above organic solvent, after stirring, the non-aqueous electrolytic solution was given.

### Comparative Example 2

Comparative Example 2 was conducted according to Comparative Example 1, except that 689.5g of manganese carbonate was used and an additional 463.3g of ferrous carbonate was added.

### Comparative Example 3

Comparative Example 3 was conducted according to Comparative Example 1, except that 1148.9g of ammonium dihydrogen phosphate and 369.0g of lithium carbonate were used, and an additional of 1.6g of 60% dilute sulfuric acid was added.

### Comparative Example 4

Comparative Example 4 was conducted according to Comparative Example 1, except that 689.5g of manganese carbonate, 1148.9g of ammonium dihydrogen phosphate, and 369.0g of lithium carbonate were used, and an additional 463.3g of ferrous carbonate and 1.6g of 60% dilute sulfuric acid were added.

### Comparative Example 5

Comparative Example 5 was conducted according to Comparative Example 4, except that the following steps was added: the preparation of lithium iron pyrophosphate powders: 9.52g of lithium carbonate, 29.9g of ferrous carbonate, 29.6g of ammonium dihydrogen phosphate, and 32.5g of oxalic acid dehydrate were dissolved into 50mL of deionized water; the mixture has a pH of 5, and was stirred for 2 hours to fully react; then, the reacted solution was heated to 80°C and maintained at this temperature for 4 hours to give a suspension containing Li₂FeP₂O₇. The suspension was filtered, washed with deionized water, and dried at 120°C for 4 hours to give powders. The powders were sintered at 500°C under a nitrogen atmosphere for 4 hours and naturally cooled to room temperature before grinding. The crystallinity of Li₂FeP₂O₇ was controlled to be 5%; and except that when preparing carbon-coated materials, the amount of Li₂FeP₂O₇ was 62.8g.

### Comparative Example 6

Comparative Example 6 was conducted according to Comparative Example 4, except that the following steps was added: the preparation of a lithium iron phosphate suspension: 14.7g of lithium carbonate, 46.1g of ferrous carbonate, 45.8g of ammonium dihydrogen phosphate, and 50.2g of oxalic acid dehydrate were dissolved into 500mL of deionized water, and then the mixture were stirred for 6 hours to fully react; then, the reacted solution was heated to 120°C and maintained at this temperature for 6 hours to give a suspension containing LiFePO₄; in the preparation of lithium iron phosphate (LiFePO₄), the sintering temperature was 600°C and the sintering time was 4 hours in the coating sintering step, to control the crystallinity of LiFePO₄ as 8%; and except that when preparing carbon-coated materials, the amount of LiFePO₄ was 62.8g.

### Comparative Example 7

Preparation of lithium iron pyrophosphate powders: 2.38g of lithium carbonate, 7.5g of ferrous carbonate, 7.4g of ammonium dihydrogen phosphate, and 8.1g of oxalic acid dihydrate were dissolved into 50mL of deionized water; the mixture has a pH of 5, and was stirred for 2 hours to fully react; then, the reacted solution was heated to 80°C and maintained at this temperature for 4 hours to give a suspension containing Li₂FeP₂O₇. The suspension was filtered, washed with deionized water, and dried at 120°C for 4 hours to give powders. The powders were sintered at 500°C under a nitrogen atmosphere for 4 hours and naturally cooled to room temperature before grinding. The crystallinity of Li₂FeP₂O₇ was controlled to be 5%.

Preparation of lithium iron phosphate suspension: 11.1g of lithium carbonate, 34.7g of ferrous carbonate, 34.4g of ammonium dihydrogen phosphate, 37.7g of oxalic acid dihydrate, and 37.3g of sucrose (calculated as C₁₂H₂₂O₁₁, the same below) were dissolved into 1500mL of deionized water, and then the mixture was stirred for 6 hours to fully react; the reacted solution was heated to 120°C and maintained at this temperature for 6 hours to give a suspension containing LiFePO₄.

Next, Comparative Example 7 was conducted according to Comparative Example 4 to give amorphous lithium iron pyrophosphate, amorphous lithium iron phosphate, and carbon-coated positive electrode active materials, excepted that 15.7g of the prepared lithium iron pyrophosphate powders were added into the suspension of the above lithium iron phosphate (LiFePO₄) and sucrose. During preparation, the sintering temperature was 600°C and the sintering time was 4 hours in the coating sintering step to control the crystallinity of LiFePO₄ as 8%.

### Comparative Example 8

Comparative Example 8 was conducted according to Comparative Example 1-3, except that H3 is not added into the non-aqueous electrolytic solution.

### Parameters Testing

### 1. Determination of the chemical formula of the core and the composition of different coating layers

The internal microstructure and surface structure of the positive electrode active material were characterized with high spatial resolution by the spherical Aberration Corrected Scanning Transmission Electron Microscope (ACSTEM). In combination with the three-dimensional reconstruction technology, the chemical formula of the core and the compositions of the first and second coating layers of the positive electrode active material were obtained.

### 2. Test for initial specific capacity of button battery

A button battery prepared in each of the above examples and comparative examples was charged to 4.3V at 0.1C, then charged at the constant voltage of 4.3V until the current was less than or equal to 0.05 mA, then was stood for 5 minutes. Afterwards, the button battery was discharged at 0.1C to 2.0V. The discharge capacity measured at this moment was the specific gram capacity, denoted as D0.

### 3. Test of average discharging voltage (V) of button battery

The button battery as prepared above was placed under a constant temperature environment of 25 °C and was allowed to stand for 5 minutes; afterwards, it was discharged to 2.5V at 0.1C and was allowed to stand for 5 minutes, and then it was charged to 4.3V at 0.1C and was charged at the constant voltage of 4.3V until the current is less than or equal to 0.05mA, allowing it to stand for 5 minutes; then it was discharged to 2.5V at 0.1C; the discharge capacity at that moment was the initial specific capacity, denoted as D0, and the discharge energy at that moment was the initial energy, denoted as E0. The average discharging voltage V of the button was denoted as E0/D0.

### 4. Test of gas expansion after of full battery

The full battery as prepared above at 100% state of charge (SOC) was stored at 60°C. Before, during, and after storing, the open circuit voltage (OCV) and AC internal impedance (IMP) of the battery were tested to monitor SOC, and the volume of the battery was measured. After every 48 hours of storage, the full battery was taken out and was allowed to stand for 1 hour, and then the open circuit voltage (OCV) and internal impedance (IMP) were tested. After cooling to room temperature, the volume of the battery was measured using the buoyant method. The buoyant method was conducted as follows: measuring the gravity F₁ of each battery with a balance having a function of automatically converting units, and then immersing the battery in deionized water (with the density of 1g/cm³), measuring the gravity F₂ of the battery at this moment, the buoyant force F_{b} on the battery was F₁-F₂, and then F_{b} = ρ× g × V was calculated according to the Archimedes principle; the volume of battery was calculated as V=(F₁-F₂)/(ρ×g).

From the OCV and IMP test results, it can be seen that the battery maintains a SOC of 99% or higher until the end of storing during the testing.

After storing for 30 days, the volume of battery was measured, and the percentage increase of the battery volume after storing as compared to the battery volume before string was calculated.

### 5. Test of cycling performance at 45°C of full battery

Under a constant temperature of 45 °C , the full battery as prepared above was charged to 4.3V at 1C, and then was charged at the constant voltage of 4.3V to the current of less than or equal to 0.05mA. After standing for 5 minutes, the full battery was discharged to 2.5V at 1C; the discharging capacity at that moment was recorded as D0. The above charging and discharging cycle was repeated until the discharge capacity decreased to 80% of D0. The number of cycles of the battery were recorded.

### 6. Measurement method for lattice change rate

Under a constant temperature environment of 25°C, the positive electrode active material sample as prepared above was placed in an X-ray diffractometer (Bruker D8 Discover model) and tested at 1°/minute. The test data was organized and analyzed. Referring to the standard PDF card, the lattice constants a0, b0, c0, and v0 (a0, b0, and c0 represent the length size in different directions of a lattice cell, respectively, and v0 represents the volume of lattice cell, which can be directly obtained through XRD refinement results) were calculated.

Using the above preparation method of the button battery, the positive electrode active material sample was prepared into a button battery. The button battery was charged at a small rate of 0.05C until the current decreased to 0.01C. Then the positive electrode plate was taken out of the button battery and soaked in DMC for 8 hours. After drying and scraping powder, the particles with a particle size less than 500 nm were obtained by sieving. A sample was taken and the volume of lattice cell v1 was calculated in the same way as the fresh sample was tested above. The lattice change rate (or the change rate of the lattice cell volume) after complete deintercalation and intercalation of lithium, represented by (v0-v1)/v0 × 100%, was shown in the table.

### 7. Test of Li/Mn antisite defect concentration

The XRD results tested in the "lattice change rate measurement method" was compared with the PDF (Powder Diffusion File) card of the standard crystal to give the Li/Mn inverse defect concentration. Specifically, the XRD results tested in the "lattice change rate measurement method" are imported into the General Structural Analysis System (GSAS) software to automatically obtain refined results, including the occupancies of different atoms. The Li/Mn anti defect concentration is obtained by reading the refined results.

### 8. Test of transition metal leaching-out

The full battery, which was cycled at 45 °C until its capacity decays to 80%, was discharged at a rate of 0.1C to a cut-off voltage of 2.0V. Then the battery was disassembled to take the negative electrode plate; and on the negative electrode plate, 30 circular plates were randomly selected per unit area (1540.25mm²). Agilent ICP-OES730 was used to obtain inductively coupled plasma emission spectroscopy (ICP). According to the ICP results, the amounts of Fe (if Fe was doped at the Mn position of the positive electrode active material) and Mn were calculated; then the leaching-out of Mn (and Mn doped Fe) after cycling was calculated. The test was conducted according to EPA-6010D-2014.

### 9. Test of surface oxygen valence

5g of the positive electrode active material sample as prepared above was taken and was prepared into a button battery according to the preparation method of a button battery described in the above Examples. The button battery was charged at a low rate of 0.05C until the current decreased to 0.01C. Then the positive electrode plate was taken out of the button battery and was soaked in DMC for 8 hours. After drying and scraping powder, the particles with a particle size less than 500 nm were obtained by sieving. The obtained particles were measured with Electron Energy Loss Spectroscopy (EELS, Talos F200S model) to give the energy loss near edge structure (ELNES), which could reflect the state density and energy level distribution of elements. Based on the state density and energy level distribution, the number of occupied electrons was calculated by integrating the state density of valence band, thereby calculating the surface oxygen valence after charging.

### 10. Test of compaction density

5g of positive electrode active material powder were taken and placed in a special mold for compaction (CARVER mold in the United States, model 13mm), and then the mold was placed on a compaction density instrument. 3T (tons) of pressure was applied and the thickness of the powders under the pressure was read on the equipment (the thickness after pressure relief, the area of the container used for testing being 1540.25mm²). The compaction density was calculated via ρ= m/v.

### 11. Test of specific surface area

5 g of the positive electrode active material powders prepared above was taken and tested by using the Tri-Star 3020 specific surface area pore size analysis tester from Micromeritics, US. Then, the specific surface area was calculated using the BET (Brunauer Emmett Teller) method. The testing was conducted according to GB/T 19587-2017.

### 12. Test of crystallinity of pyrophosphate and phosphate via X-ray diffraction method

5g of the positive electrode active material powder as prepared above was take and was measured the total scattering intensity via X-ray. The total scattering intensity, as the sum of the scattering intensities of the entire space substance, was only related to the intensity of the primary ray, chemical structure, and the total number of electrons having diffraction, i.e. the mass, but was not related to the order state of the sample. Then, the crystalline scattering and amorphous scattering on the diffraction pattern were separated; the crystallinity was the ratio of the scattering intensity of the crystalline to the total scattering intensity.

### 13. Test of interplanar spacing and included angle

1g of each positive electrode active material powder as prepared above was added into a 50mL test tube, and the into the test tube, 10mL of alcohol with a mass fraction of 75% was added; after stirring and dispersing for 30 minutes, an appropriate amount of the above solution was taken with a clean disposable plastic pipette and was dropped on a 300-mesh copper mesh; some of the copper powder left on the mesh; then the mesh carrying the sample was transferred to a TEM (Talos F200s G2) sample chamber, to give the original images of TEM test.

The original images from the above TEM test were opened in DigitalMicrograph software and was Fourier transformed (automatically done by the software after clicking on the operation) to obtain the diffraction pattern; the distance from the diffracted spot to the center of the diffraction pattern was measured to obtain the interplanar spacing, and the included angle was calculated according to the Bragg equation.
Table 1 shows the composition of the positive electrode active material of Examples 1-1 to 1-33 and in Comparative Examples 1 to 8.

**Table 1**

| No. | Core | First coating layer | Second coating layer |
|---|---|---|---|
| Example 1-1 | Li_{0.999}Mn_{0.60}Fe_{0.393}V_{0.004}Co_{0.003}P_{0.999}S_{0.001}O₄ | 1% Li₂FeP₂O₇/3% LiFePO₄ | 2%Carbon |
| Example 1-2 | Li_{0.999}Mn_{0.60}Fe_{0.40}P_{0.999}S_{0.001}O₄ | 0.8% Li₂FeP₂O₇/2.4% LiFePO₄ | 1%Carbon |
| Example 1-3 | Li_{0.999}Mn_{0.60}Fe_{0.40}P_{0.999}S_{0.001}O₄ | 1% Li₂FeP₂O₇/3% LiFePO₄ | 1%Carbon |
| Example 1-4 | Li_{0.999}Mn_{0.60}Fe_{0.40}P_{0.996}S_{0.001}O₄ | 1.2% Li₂FeP₂O₇/3.6% LiFePO₄ | 1%Carbon |
| Example 1-5 | Li_{0.999}Mn_{0.60}Fe_{0.40}P_{0.999}S_{0.001}O₄ | 1.4% Li₂FeP₂O₇/4.2% LiFePO₄ | 1%Carbon |
| Example 1-6 | Li_{0.999}Mn_{0.60}Fe_{0.40}P_{0.999}S_{0.001}O₄ | 1.6% Li₂FeP₂O₇/4.8% LiFePO₄ | 1%Carbon |
| Example 1-7 | Li_{0.999}Mn_{0.60}Fe_{0.40}P_{0.999}S_{0.001}O₄ | 1% Li₂FeP₂O₇/3% LiFePO₄ | 2%Carbon |
| Example 1-8 | Li_{0.999}Mn_{0.60}Fe_{0.40}P_{0.999}S_{0.001}O₄ | 1% Li₂FeP₂O₇/3% LiFePO₄ | 4%Carbon |
| Example 1-9 | Li_{0.999}Mn_{0.60}Fe_{0.40}P_{0.999}S_{0.001}O₄ | 1% Li₂FeP₂O₇/3% LiFePO₄ | 5%Carbon |
| Example 1-10 | Li_{0.999}Mn_{0.60}Fe_{0.40}P_{0.999}S_{0.001}O₄ | 1% Li₂FeP₂O₇/3% LiFePO₄ | 6%Carbon |
| Example 1-11 | Li_{0.999}Mn_{0.60}Fe_{0.40}P_{0.999}S_{0.001}O₄ | 1.5% Li₂FeP₂O₇/2.5% LiFePO₄ | 2%Carbon |
| Example 1-12 | Li_{0.999}Mn_{0.60}Fe_{0.40}P_{0.999}S_{0.001}O₄ | 2% Li₂FeP₂O₇/2% LiFePO₄ | 2%Carbon |
| Example 1-13 | Li_{0.999}Mn_{0.60}Fe_{0.40}P_{0.999}S_{0.001}O₄ | 2.5% Li₂FeP₂O₇/1.5% LiFePO₄ | 2%Carbon |
| Example 1-14 | Li_{0.999}Mn_{0.60}Fe_{0.40}P_{0.999}S_{0.001}O₄ | 3% Li₂FeP₂O₇/1% LiFePO₄ | 2%Carbon |
| Example 1-15 | Li_{0.999}Mn_{0.60}Zn_{0.40}P_{0.999}S_{0.001}O₄ | 3% Li₂FeP₂O₇/1% LiFePO₄ | 2%Carbon |
| Example 1-16 | Li_{0.993}Mn_{0.6}Ni_{0.393}Zn_{0.004}Ti_{0.003}P_{0.999}S_{0.001}O₄ | 1% Li₂FeP₂O₇/3% LiFePO₄ | 2%Carbon |
| Example 1-17 | Li_{0.999}Mn_{0.60}Fe_{0.393}V_{0.007}P_{0.999}S_{0.001}O₄ | 1% Li₂FeP₂O₇/3% LiFePO₄ | 2%Carbon |
| Example 1-18 | Li_{0.999}Mn_{0.60}Fe_{0.393}V_{0.004}Mg_{0.003}P_{0.999}S_{0.001}O₄ | 1% Li₂FeP₂O₇/3% LiFePO₄ | 2%Carbon |
| Example 1-19 | LiMn_{0.60}Fe_{0.393}V_{0.004}Mg_{0.003}P_{0.999}N_{0.001}O₄ | 1% Li₂FeP₂O₇/3% LiFePO₄ | 2%Carbon |
| Example 1-20 | Li_{1.001}Mn_{0.60}Fe_{0.393}V_{0.004}Mg_{0.003}P_{0.999}Si_{0.001}O₄ | 1% Li₂FeP₂O₇/3% LiFePO₄ | 2%Carbon |
| Example 1-21 | Li_{1.001}Mn_{0.55}Fe_{0.40}V_{0.025}Mg_{0.025}P_{0.999}Si_{0.001}O₄ | 1% Li₂FeP₂O₇/3% LiFePO₄ | 2%Carbon |
| Example 1-22 | Li₁₀₀₁Mn_{0.65}Fe_{0.25}V_{0.05}Mg_{0.05}P_{0.999}Si_{0.001}O₄ | 1% Li₂FeP₂O₇/3% LiFePO₄ | 2%Carbon |
| Example 1-23 | Li_{1.004}Mn_{0.7}Fe_{0.2}V_{0.05}Mg_{0.05}P_{0.998}B_{0.002}O4 | 1% Li₂FeP₂O₇/3% LiFePO₄ | 2%Carbon |
| Example 1-24 | Li_{1.006}Mn_{0.75}Fe_{0.15}V_{0.05}Mg_{0.05}P_{0.997}B_{0.003}O₄ | 1% Li₂FeP₂O₇/3% LiFePO₄ | 2%Carbon |
| Example 1-25 | Li_{1.002}Mn_{0.60}Fe_{0.393}V_{0.004}Mg_{0.003}P_{0.998}Si_{0.002}O₄ | 1% Li₂FeP₂O₇/3% LiFePO₄ | 2%Carbon |
| Example 1-26 | Li_{0.997}Mn_{0.80}Fe_{0.194}V_{0.003}Mg_{0.003}P_{0.997}S_{0.003}O₄ | 1% Li₂FeP₂O₇/3% LiFePO₄ | 2%Carbon |
| Example 1-27 | Li_{0.996}Mn_{0.60}Fe_{0.393}V_{0.004}Mg_{0.003}P_{0.996}S_{0.004}O₄ | 1% Li₂FeP₂O₇/3% LiFePO₄ | 2%Carbon |
| Example 1-28 | Li_{0.995}Mn_{0.9}Fe_{0.094}V_{0.003}Mg_{0.003}P_{0.995}S_{0.005}O₄ | 1% Li₂FeP₂O₇/3% LiFePO₄ | 2%Carbon |
| Example 1-29 | Li_{0.994}Mn_{0.90}Fe_{0.094}V_{0.003}Mg_{0.003}P_{0.994}S_{0.006}O₄ | 1% Li₂FeP₂O₇/3% LiFePO₄ | 2%Carbon |
| Example 1-30 | Li_{0.993}Mn_{0.90}Fe_{0.094}V_{0.003}Mg_{0.003}P_{0.993}S_{0.007}O₄ | 1% Li₂FeP₂O₇/3% LiFePO₄ | 2%Carbon |
| Example 1-31 | Li_{0.992}Mn_{0.90}Fe_{0.094}V_{0.003}Mg_{0.003}P_{0.992}S_{0.008}O₄ | 1% Li₂FeP₂O₇/3% LiFePO₄ | 2%Carbon |
| Example 1-32 | Li_{0.991}Mn_{0.90}Fe_{0.094}V_{0.003}Mg_{0.003}P_{0.991}S_{0.009}O₄ | 1% Li₂FeP₂O₇/3% LiFePO₄ | 2%Carbon |
| Example 1-33 | Li_{0.9}Mn_{0.90}Fe_{0.094}V_{0.003}Mg_{0.003}P_{0.9}S_{0.1}O₄ | 1% Li₂FeP₂O₇/3% LiFePO₄ | 2%Carbon |
| Comparative Example 1 | LiMnPO₄ | - | 1%Carbon |
| Comparative Example 2 | LiMn_{0.60}Fe_{0.40}PO₄ | - | 1%Carbon |
| Comparative Example 3 | Li_{0.999}MnP_{0.999}S_{0.001}O₄ | - | 1%Carbon |
| Comparative Example 4 | Li_{0.999}Mn_{0.60}Fe_{0.40}P_{0.999}S_{0.001}O₄ | - | 1%Carbon |
| Comparative Example 5 | Li_{0.999}Mn_{0.60}Fe_{0.40}P_{0.999}S_{0.001}O₄ | 4% amorphous Li₂FeP₂O₇ | 1%Carbon |
| Comparative Example 6 | Li_{0.999}Mn_{0.60}Fe_{0.40}P_{0.999}S_{0.001}O₄ | 4% amorphous LiFePO₄ | 1%Carbon |
| Comparative Example 7 | Li_{0.999}Mn_{0.60}Fe_{0.40}P_{0.999}S_{0.001}O₄ | 1% amorphous Li₂FeP₂O₇+3% amorphous LiFePO₄ | 1%Carbon |
| Comparative Example 8 | Li_{0.999}Mn_{0.60}Fe_{0.40}P_{0.999}S_{0.001}O₄ | 1% Li₂FeP₂O₇/3% LiFePO₄ | 1%Carbon |
| Note: 1) The crystallinity of Li₂FeP₂O₇ and LiFePO₄ in Examples 1-1-33 and Comparative Example 8 was 100%; 2) In Comparative Examples 5 to 7, the crystallinity of Li₂FeP₂O₇ was 5%, and the crystallinity of LiFePO₄ was 8%. | | | |

**Table 2**

| No. | Preparation of non-aqueous electrolytic solution |
|---|---|
| Comparative Examples 1-8 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ was dissolved in the above organic solvent, after stirring, the non-aqueous electrolytic solution was obtained. |
| Examples 1-1 to 1-33 and Examples 2-1to 2-3 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ and 1 weight% (based on the total weight of the non-aqueous electrolytic solution) of compound H3 were dissolved in the above organic solvent, after stirring, the non-aqueous electrolytic solution was obtained. |
| Example 3-1 | I In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ and 1 weight% (based on the total weight of the non-aqueous electrolytic solution) of compound H1 were dissolved in the above organic solvent, after stirring, the non-aqueous electrolytic solution was obtained. |
| Example 3-2 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ and 1 weight% (based on the total weight of the non-aqueous electrolytic solution) of compound H2 were dissolved in the above organic solvent, after stirring, the non-aqueous electrolytic solution was obtained. |
| Example 3-3 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ and 1 weight% (based on the total weight of the non-aqueous electrolytic solution) of compound H5 were dissolved in the above organic solvent, after stirring, the non-aqueous electrolytic solution was obtained. |
| Example 3-4 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ and 1 weight% (based on the total weight of the non-aqueous electrolytic solution) of compound H9 were dissolved in the above organic solvent, after stirring, the non-aqueous electrolytic solution was obtained. |
| Example 3-5 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ and 1 weight% (based on the total weight of the non-aqueous electrolytic solution) of compound H11 were dissolved in the above organic solvent, after stirring, the non-aqueous electrolytic solution was obtained. |
| Example 3-6 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ and 1 weight% (based on the total weight of the non-aqueous electrolytic solution) of compound H19 were dissolved in the above organic solvent, after stirring, the non-aqueous electrolytic solution was obtained. |
| Example 3-7 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ and 1 weight% (based on the total weight of the non-aqueous electrolytic solution) of compound H23 were dissolved in the above organic solvent, after stirring, the non-aqueous electrolytic solution was obtained. |
| Example 3-8 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ and 1 weight% (based on the total weight of the non-aqueous electrolytic solution) of compound H28 were dissolved in the above organic solvent, after stirring, the non-aqueous electrolytic solution was obtained. |
| Example 3-9 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ and 1 weight% (based on the total weight of the non-aqueous electrolytic solution) of compound H32 were dissolved in the above organic solvent, after stirring, the non-aqueous electrolytic solution was obtained. |
| Example 3-10 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆ and 1 weight% (based on the total weight of the non-aqueous electrolytic solution) of compound H36 were dissolved in the above organic solvent, after stirring, the non-aqueous electrolytic solution was obtained. |
| Example 3-11 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 1 weight% (based on the total weight of the non-aqueous electrolytic solution) of compound M16 were dissolved in the above organic solvent, after stirring, the non-aqueous electrolytic solution was obtained. |
| Example 3-12 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 1 weight% (based on the total weight of the non-aqueous electrolytic solution) of compound M24 were dissolved in the above organic solvent, after stirring, the non-aqueous electrolytic solution was obtained. |
| Example 3-13 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 1 weight% (based on the total weight of the non-aqueous electrolytic solution) of compound M1 were dissolved in the above organic solvent, after stirring, the non-aqueous electrolytic solution was obtained. |
| Example 3-14 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 1 weight% (based on the total weight of the non-aqueous electrolytic solution) of compound M15 were dissolved in the above organic solvent, after stirring, the non-aqueous electrolytic solution was obtained. |
| Example 3-15 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 1 weight% (based on the total weight of the non-aqueous electrolytic solution) of lithium difluorophosphate were dissolved in the above organic solvent, after stirring, the non-aqueous electrolytic solution was obtained. |
| Example 3-16 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 1 weight% (based on the total weight of the non-aqueous electrolytic solution) of lithium difluorodioxalate phosphate were dissolved in the above organic solvent, after stirring, the non-aqueous electrolytic solution was obtained. |
| Example 3-17 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 1 weight% (based on the total weight of the non-aqueous electrolytic solution) of lithium difluorooxalate borate were dissolved in the above organic solvent, after stirring, the non-aqueous electrolytic solution was obtained. |
| Example 3-18 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, 1 weight% (based on the total weight of the non-aqueous electrolytic solution) of M16, and 1 weight% (based on the total weight of the non-aqueous electrolytic solution) of VC were dissolved in the above organic solvent, after stirring, the non-aqueous electrolytic solution was obtained. |
| Example 3-19 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, 1 weight% (based on the total weight of the non-aqueous electrolytic solution) of M16, and 1 weight% (based on the total weight of the non-aqueous electrolytic solution) of VEC were dissolved in the above organic solvent, after stirring, the non-aqueous electrolytic solution was obtained. |
| Example 3-20 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, 1 weight% (based on the total weight of the non-aqueous electrolytic solution) of M16, and 1 weight% (based on the total weight of the non-aqueous electrolytic solution) of FEC were dissolved in the above organic solvent, after stirring, the non-aqueous electrolytic solution was obtained. |
| Example 3-21 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 0.01 weight% (based on the total weight of the non-aqueous electrolytic solution) of compound H3, and 1 weight% (based on the total weight of the non-aqueous electrolytic solution) of M16 were dissolved in the above organic solvent, after stirring, the non-aqueous electrolytic solution was obtained. |
| Example 3-22 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 0.1 weight% (based on the total weight of the non-aqueous electrolytic solution) of compound H3, and 1 weight% (based on the total weight of the non-aqueous electrolytic solution) of M16 were dissolved in the above organic solvent, after stirring, the non-aqueous electrolytic solution was obtained. |
| Example 3-23 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 0.3 weight% (based on the total weight of the non-aqueous electrolytic solution) of compound H3, and 1 weight% (based on the total weight of the non-aqueous electrolytic solution) of M16 were dissolved in the above organic solvent, after stirring, the non-aqueous electrolytic solution was obtained. |
| Example 3-24 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 5 weight% (based on the total weight of the non-aqueous electrolytic solution) of compound H3, and 1 weight% (based on the total weight of the non-aqueous electrolytic solution) of M16 were dissolved in the above organic solvent, after stirring, the non-aqueous electrolytic solution was obtained. |
| Example 3-25 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 10 weight% (based on the total weight of the non-aqueous electrolytic solution) of compound H3, and 1 weight% (based on the total weight of the non-aqueous electrolytic solution) of M16 were dissolved in the above organic solvent, after stirring, the non-aqueous electrolytic solution was obtained. |
| Example 3-26 | I In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 20 weight% (based on the total weight of the non-aqueous electrolytic solution) of compound H3, and 1 weight% (based on the total weight of the non-aqueous electrolytic solution) of M16 were dissolved in the above organic solvent, after stirring, the non-aqueous electrolytic solution was obtained. |
| Example 3-27 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 0.01 weight% (based on the total weight of the non-aqueous electrolytic solution) of M16 were dissolved in the above organic solvent, after stirring, the non-aqueous electrolytic solution was obtained. |
| Example 3-28 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 0.1 weight% (based on the total weight of the non-aqueous electrolytic solution) of M16 were dissolved in the above organic solvent, after stirring, the non-aqueous electrolytic solution was obtained. |
| Example 3-29 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 0.3 weight% (based on the total weight of the non-aqueous electrolytic solution) of M16 were dissolved in the above organic solvent, after stirring, the non-aqueous electrolytic solution was obtained. |
| Example 3-30 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 5 weight% (based on the total weight of the non-aqueous electrolytic solution) of M16 were dissolved in the above organic solvent, after stirring, the non-aqueous electrolytic solution was obtained. |
| Example 3-31 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 10 weight% (based on the total weight of the non-aqueous electrolytic solution) of M16 were dissolved in the above organic solvent, after stirring, the non-aqueous electrolytic solution was obtained. |
| Example 3-32 | In an argon atmosphere glove box (H₂O<0.1ppm, O₂<0.1ppm), ethyl carbonate (EC), diethyl carbonate (DEC), and dimethyl carbonate (DMC) in a volume ratio of 1:1:1 were mixed to give an organic solvent. Afterwards, 12.5 weight% (based on the total weight of the non-aqueous electrolytic solution) of LiPF₆, 1 weight% (based on the total weight of the non-aqueous electrolytic solution) of compound H3, and 30 weight% (based on the total weight of the non-aqueous electrolytic solution) of M16 were dissolved in the above organic solvent, after stirring, the non-aqueous electrolytic solution was obtained. |

Table 3 shows the performance data of the positive electrode active materials, positive electrode plates, the button, and the full of Comparative Examples 1 to 8 and Examples 1-1 to 1-33; the date were obtained in the above performance test.

Table 4 shows the performance data of the positive electrode active materials, positive electrode plates, the button, and the full of Examples 2-1 to 2-3; the date were obtained in the above performance test.

**Table 3**

| Nos. | Lattice change rate (%) | Li/Mn antisite defect concentration (%) | Compaction density (g/cm3) | Surface oxygen valence | Specific surface area of positive electrode material (m²/g) | Leaching-out of Mn and Fe after cycling (ppm) | Capacity of button battery at 0.1C mAh/g | Average discharging voltage of button battery (V) | Expansion rate of battery at 60°C after storing for 30 days (%) | Number of cycles at capacity retention of 80% at 45 °C |
|---|---|---|---|---|---|---|---|---|---|---|
| Example 1-1 | 2.4 | 0.4 | 2.41 | -1.98 | 15 | 6 | 160.6 | 3.82 | 0.8 | 1482 |
| Example 1-2 | 6.6 | 1.2 | 2.43 | -1.97 | 15 | 48 | 151.1 | 3.79 | 2.6 | 897 |
| Example 1-3 | 6.5 | 1.1 | 2.45 | -1.97 | 15 | 37 | 150.7 | 3.78 | 2.1 | 1024 |
| Example 1-4 | 6.5 | 0.8 | 2.44 | -1.97 | 15 | 24 | 149.5 | 3.77 | 1.9 | 1080 |
| Example 1-5 | 6.5 | 0.7 | 2.45 | -1.98 | 15 | 15 | 149.0 | 3.77 | 1.4 | 1187 |
| Example 1-6 | 6.6 | 0.6 | 2.45 | -1.98 | 15 | 11 | 148.1 | 3.76 | 1.1 | 1327 |
| Example 1-7 | 6.5 | 1.0 | 2.46 | -1.97 | 15 | 32 | 149.7 | 3.77 | 1.9 | 1080 |
| Example 1-8 | 6.5 | 1.0 | 2.47 | -1.97 | 15 | 29 | 148.6 | 3.77 | 1.7 | 1129 |
| Example 1-9 | 6.4 | 1.1 | 2.44 | -1.98 | 15 | 22 | 146.5 | 3.77 | 1.5 | 1237 |
| Example 1-10 | 6.4 | 1.1 | 2.41 | -1.98 | 15 | 14 | 144.2 | 3.77 | 1.1 | 1360 |
| Example 1-11 | 6.5 | 1.1 | 2.44 | -1.97 | 15 | 26 | 150.0 | 3.78 | 2.1 | 1035 |
| Example 1-12 | 6.6 | 1.0 | 2.47 | -1.96 | 15 | 17 | 149.8 | 3.78 | 2.4 | 1001 |
| Example 1-13 | 6.7 | 1.2 | 2.46 | -1.96 | 15 | 14 | 149.4 | 3.78 | 2.8 | 911 |
| Example 1-14 | 6.7 | 1.1 | 2.45 | -1.97 | 15 | 7 | 149.2 | 3.79 | 3.0 | 853 |
| Example 1-15 | 7.5 | 2.5 | 2.45 | -1.97 | 15 | 14 | 140.8 | 3.89 | 3.4 | 956 |
| Example 1-16 | 5.4 | 0.8 | 2.44 | -1.97 | 15 | 11 | 141.5 | 3.89 | 1.8 | 1087 |
| Example 1-17 | 4.2 | 0.6 | 2.45 | -1.97 | 15 | 10 | 155.5 | 3.82 | 1.3 | 1385 |
| Example 1-18 | 2.6 | 0.5 | 2.45 | -1.97 | 15 | 8 | 157.9 | 3.84 | 1.1 | 1389 |
| Example 1-19 | 2.3 | 0.5 | 2.45 | -1.98 | 15 | 7 | 160.0 | 3.84 | 0.8 | 1505 |
| Example 1-20 | 2.4 | 0.7 | 2.44 | -1.98 | 15 | 8 | 159.8 | 3.84 | 1.0 | 1527 |
| Example 1-21 | 2.2 | 0.5 | 2.43 | -1.98 | 15 | 6 | 160.8 | 3.76 | 0.8 | 1628 |
| Example 1-22 | 2.5 | 0.8 | 2.42 | -1.98 | 15 | 9 | 159.3 | 3.87 | 1.1 | 1432 |
| Example 1-23 | 2.6 | 0.8 | 2.43 | -1.98 | 15 | 8 | 159.5 | 3.87 | 1.0 | 1415 |
| Example 1-24 | 2.6 | 0.8 | 2.44 | -1.98 | 15 | 8 | 159.8 | 3.87 | 1.0 | 1483 |
| Example 1-25 | 2.3 | 0.7 | 2.45 | -1.98 | 15 | 8 | 159.7 | 3.82 | 1.0 | 1528 |
| Example 1-26 | 2.8 | 0.9 | 2.45 | -1.98 | 15 | 9 | 158.4 | 3.89 | 1.2 | 1259 |
| Example 1-27 | 2.2 | 0.6 | 2.46 | -1.98 | 15 | 8 | 159.9 | 3.82 | 1.0 | 1556 |
| Example 1-28 | 3.2 | 1.1 | 2.45 | -1.96 | 15 | 10 | 159.2 | 3.90 | 1.3 | 1215 |
| Example 1-29 | 3.0 | 1.2 | 2.44 | -1.95 | 15 | 11 | 158.4 | 3.90 | 1.3 | 1158 |
| Example 1-30 | 2.8 | 1.4 | 2.45 | -1.95 | 15 | 12 | 158.1 | 3.90 | 1.2 | 1058 |
| Example 1-31 | 2.6 | 1.4 | 2.44 | -1.94 | 15 | 13 | 157.7 | 3.90 | 1.2 | 1023 |
| Example 1-32 | 2.4 | 1.2 | 2.45 | -1.94 | 15 | 14 | 157.1 | 3.90 | 1.1 | 1001 |
| Example 1-33 | 2.1 | 0.9 | 2.44 | -1.94 | 15 | 15 | 156.8 | 3.90 | 1.1 | 981 |
| Comparative Example 1 | 11.4 | 3.2 | 1.81 | -1.55 | 15 | 2060 | 125.6 | 4.02 | 48.6 | 185 |
| Comparative Example 2 | 8.7 | 2.8 | 1.92 | -1.76 | 15 | 1597 | 134.8 | 3.76 | 42.5 | 358 |
| Comparative Example 3 | 9.8 | 2.5 | 1.88 | -1.66 | 15 | 1895 | 128.6 | 4.05 | 45.5 | 267 |
| Comparative Example 4 | 6.7 | 1.8 | 1.82 | -1.83 | 15 | 1279 | 140.5 | 3.78 | 38.5 | 417 |
| Comparative Example 5 | 6.5 | 1.8 | 1.79 | -1.90 | 15 | 208 | 140.3 | 3.73 | 12.5 | 519 |
| Comparative Example 6 | 6.6 | 1.8 | 1.83 | -1.91 | 15 | 318 | 140.2 | 3.74 | 11.5 | 528 |
| Comparative Example 7 | 6.6 | 1.8 | 1.84 | -1.90 | 15 | 174 | 140.1 | 3.75 | 8.6 | 682 |
| Comparative Example 8 | 6.5 | 1.1 | 2.45 | -1.97 | 15 | 48 | 148.5 | 3.74 | 5.3 | 918 |

**Table 4**

| Nos. | Pyrophosphate and phosphate crystallinity | Lattice change rate (%) | Li/Mn antisite defect concentration (%) | Compaction density (g/cm3) | Surface oxygen valence | Specific surface area of positive electrode material (m²/g) | Leaching-out of Mn and Fe after cycling (ppm) | Capacity of button battery at 0.1C mAh/g | Average discharging voltage of button battery (V) | Expansion rate of battery at 60°C after storing for 30 days (%) | Number of cycles at capacity retention of 80% at 45°C |
|---|---|---|---|---|---|---|---|---|---|---|---|
| Example 2-1 | 30% | 6.3 | 1.9 | 2.1 | -1.88 | 15 | 112 | 147.4 | 3.76 | 2.2 | 779 |
| Example 2-2 | 50% | 4.7 | 1.2 | 2.25 | -1.89 | 15 | 74 | 151.4 | 3.78 | 1.9 | 970 |
| Example 2-3 | 70% | 3.5 | 0.8 | 2.33 | -1.91 | 15 | 22 | 153.6 | 3.79 | 1.5 | 1146 |
| Example 1-1 | 100% | 2.4 | 0.4 | 2.41 | -1.98 | 15 | 6 | 160.6 | 3.82 | 0.8 | 1482 |

As can be seen from Examples 1-1 to 1-33 and Comparative Examples 1 to 8, the first coating layer is beneficial for reducing the Li/Mn antisite defect concentration and the Fe and Mn leaching-out after cycling of the obtained materials, thereby increasing the specific capacity of the battery and improving the cycling and storage performance of the battery. When other elements are doped at the Mn and phosphorus sites, the lattice change rate, antisite defect concentration, and Fe and Mn leaching-out of the obtained materials can be significantly reduced, thereby increasing the specific capacity of the battery and improving the cycling and storage performance of the battery. The first additive in the non-aqueous electrolytic solution is beneficial for reducing the leaching-out of the first coating layer and reducing the catalytic oxidation effect of the second coating layer, thereby further improving the cycling and storage performance of the battery.

As can be seen from Examples 1-2 to 1-6, with the amount of the first coating layer increasing from 3.2% to 6.4%, the Li/Mn antisite defect concentration of the obtained materials gradually decreases, and the Fe and Mn leaching-out gradually decreases after cycling. The safety performance and the cycling performance at 45 °C of the corresponding battery are improved, but the specific capacity slightly decreases. Optionally, under the condition that the total amount of the first coating layer is 4-5.6 wt%, the corresponding battery has the best overall performance.

As can be seen from Examples 1-3 and Examples 1-7 to 1-10, with the amount of the second coating layer increasing from 1% to 6%, the Fe and Mn leaching-out gradually decreases after cycling. The safety performance and the cycling performance at 45 °C of the corresponding battery also improved, but the specific capacity slightly decreases. Optionally, under the condition that the total amount of the second coating layer is 3-5wt%, the corresponding battery has the best overall performance.

As can be seen from Examples 1-11 to 1-15 and the Comparative Examples 5-6, under the condition that Li₂FeP₂O₇ and LiFePO₄ were present in the first coating layer, especially under the condition that the weight ratio of Li₂FeP₂O₇ to LiFePO₄ is from 1:3 to 3:1 and especially from 1:3 to 1:1, the performance improvement of the battery is more significant.

Figure 7 shows a comparison between the XRD spectrum of the core of the positive electrode active material prepared in Example 1-1 and the standard XRD spectrum of lithium manganese phosphate (00-033-0804). As shown in Figure 7, the positions of the main characteristic peaks of the core of the positive electrode active material of the present application are basically consistent with those before being doped with lithium manganese phosphate, indicating that there is no impurity phase in the core of the positive electrode active material of the present application; the improvement of battery performance mainly results from element doping rather than impurity phase.

From Table 3, it can be seen that with the crystallinity of pyrophosphate and phosphate in the first coating layer gradually increasing, the lattice change rate, Li/Mn antisite defect concentration, and Fe and Mn leaching-out of the corresponding material decrease gradually; the specific capacity of the battery increases gradually, and the safety and cycling performance also improve gradually.

Examples 3-1 to 3-32 are the same as Examples 1-1 except for the preparation of the non-aqueous electrolytic solution.

Table 5 shows the performance data of the full battery or button battery of Examples 3-1 to 3-20, as tested above.

Table 6 shows the performance data of the full battery or button battery of Examples 3-21 to 3-32, as tested above.

**Table 5**

| Nos. | First additive | | Second additive | | Third additive | | Leaching-out of Mn and Fe after cycling (ppm) | Capacity of button battery at 0.1C mAh/g | Average discharging voltage of button battery (V) | Expansion rate of battery at 60°C after storing for 30 days (%) | Number of cycles at capacity retention of 80% at 45°C |
|---|---|---|---|---|---|---|---|---|---|---|---|
| | Type | W1 (wt%) | Type | W2 (wt%) | Type | W3 (wt%) | | | | | |
| Example 3-1 | H1 | 1 | - | - | - | - | 7 | 159.6 | 3.81 | 1.5 | 1385 |
| Example 3-2 | H2 | 1 | - | - | - | - | 6 | 160.2 | 3.82 | 1.2 | 1423 |
| Example 3-3 | H5 | 1 | - | - | - | - | 6 | 160.5 | 3.82 | 1.1 | 1445 |
| Example 3-4 | H9 | 1 | - | - | - | - | 7 | 159.4 | 3.80 | 1.4 | 1367 |
| Example 3-5 | H11 | 1 | - | - | - | - | 7 | 158.3 | 3.79 | 1.8 | 1338 |
| Example 3-6 | H19 | 1 | - | - | - | - | 7 | 158.3 | 3.79 | 1.7 | 1340 |
| Example 3-7 | H23 | 1 | - | - | - | - | 7 | 159.5 | 3.80 | 1.7 | 1356 |
| Example 3-8 | H28 | 1 | - | - | - | - | 7 | 158.4 | 3.80 | 1.6 | 1344 |
| Example 3-9 | H32 | 1 | - | - | - | - | 6 | 160.4 | 3.80 | 1.1 | 1401 |
| Example 3-10 | H36 | 1 | - | - | - | - | 7 | 158.3 | 3.79 | 1.8 | 1343 |
| Example 3-11 | H3 | 1 | M16 | 1 | - | - | 5 | 162.5 | 3.90 | 0.4 | 1792 |
| Example 3-12 | H3 | 1 | M24 | 1 | - | - | 5 | 162.3 | 3.89 | 0.5 | 1756 |
| Example 3-13 | H3 | 1 | M1 | 1 | - | - | 5 | 161.3 | 3.88 | 0.5 | 1667 |
| Example 3-14 | H3 | 1 | M15 | 1 | - | - | 5 | 161.0 | 3.87 | 0.7 | 1601 |
| Example 3-15 | H3 | 1 | Lithium difluorophosphate | 1 | - | - | 5 | 162.4 | 3.90 | 0.6 | 1767 |
| Example 3-16 | H3 | 1 | Lithium difluorodioxalate phosphate | 1 | - | - | 6 | 161.3 | 3.89 | 0.7 | 1645 |
| Example 3-17 | H3 | 1 | Lithium difluorooxalate borate | 1 | - | - | 6 | 161.1 | 3.88 | 0.8 | 1589 |
| Example 3-18 | H3 | 1 | M16 | 1 | VC | 1 | 3 | 162.8 | 3.90 | 0.2 | 2067 |
| Example 3-19 | H3 | 1 | M16 | 1 | VEC | 1 | 4 | 162.5 | 3.90 | 0.3 | 1943 |
| Example 3-20 | H3 | 1 | M16 | 1 | FEC | 1 | 3 | 162.7 | 3.90 | 0.2 | 2050 |

As can be seen from Example 1-1 and Examples 3-10 to 3-10, different the first additives have slightly different effects on improving battery performance.

As can be seen from Example 3-1 and Examples 3-11 to 3-20, under the condition that the non-aqueous electrolytic solution further contains an appropriate amount of second and/or third additives, it helps to further improve the specific capacity, rate performance, high-temperature cycling performance, and high-temperature storage performance of the battery.

As can be seen from Example 3-11 and Examples 3-21 to 3-32, under the condition that the coating amount of the first coating layer denoted as C1 wt%, the coating amount of the second coating layer denoted as C2 wt%, the amount of the first additive denoted W1 wt %, and the amount of the second additive denoted as W2 wt % satisfy W1/W2 is from 0.01 to 20 and optionally from 0.01 to 10, and/or, satisfy (W1+W2)/(C1+C2+C3) is from 0.001 to 2 and optionally from 0.01 to 1, it helps to further improve specific capacity, rate performance, cycling performance, and storage performance of the battery.

It should be noted that this application is not limited to the above embodiments. The above embodiments are only provided as examples, and within the technical solution of the present application, embodiments having substantially the same configuration as the technical idea and exerting the same effects are all included within the technical scope of the present application. In addition, various modifications to the embodiments that can be conceived by those skilled in the art without departing from the scope of the spirit of the present application and other embodiments constructed by combining some constituent elements in the embodiments are also included in the scope of the present application.

## Claims

1. A secondary battery, comprising a positive electrode plate and a non-aqueous electrolytic solution, wherein
the positive electrode plate comprises a positive electrode active material having a core-shell structure, the positive electrode active material comprising a core and a shell covering the core,
the core comprises Li₁₊ₓMn_{1-y}A_{y}P_{1-z}R_{z}O₄, wherein
x is from -0.100 to 0.100,
y is from 0.001 to 0.500,
z is from 0.001 to 0.100,
A is one or more selected from the group consisting of Zn, Al, Na, K, Mg, Mo, W, Ti, V, Zr, Fe, Ni, Co, Ga, Sn, Sb, Nb, and Ge, and optionally is one or more selected from the group consisting of Fe, Ti, V, Ni, Co, and Mg,
R is one or more element(s) selected from the group consisting of B, Si, N, and S;
the shell comprises a first coating covering the core, and a second coating covering the first coating, wherein
the first coating layer comprises pyrophosphate MP₂O₇ and phosphate XPO₄, wherein M and X are each independently one or more selected from Li, Fe, Ni, Mg, Co, Cu, Zn, Ti, Ag, Zr, Nb, and Al,
the second coating comprises carbon;
the non-aqueous electrolytic solution comprises a first additive one or more selected from the compounds shown in formulae 1-A to 1-D, 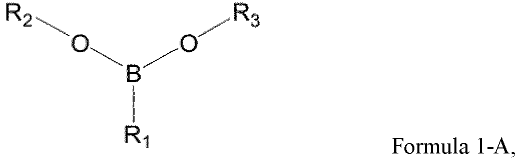 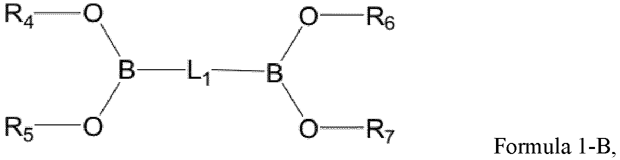 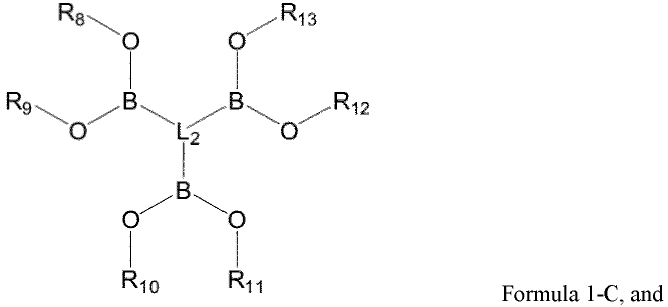 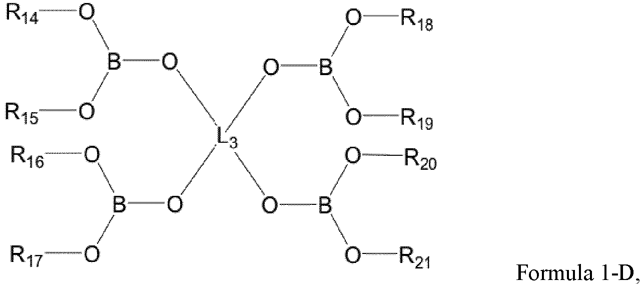
in which
R₁ represents a hydrogen atom or at least one selected from the group consisting of hydroxyl, C1-C18 monovalent alkyl, C1-C18 monovalent alkoxy, C2-C18 monovalent alkoxy alkyl, C3-C18 monovalent cycloalkyl, C2-C18 monovalent heterocyclic alkyl, C6-C18 monovalent aryl, C7-C18 monovalent aryl alkyl, C7-C18 monovalent alkylaryl, C6-C18 monovalent aryloxy, C7-C18 monovalent aryloxy alkyl, C12-C18 monovalent aryl ether group, C2-C18 monovalent heteroaryl, C3-C18 monovalent heteroaryl alkyl, C3-C18 monovalent alkyl heteroaryl, and C1-C18 monovalent silyl;
R₂ to R₂₁ each independently represent a hydrogen atom or at least one selected from the group consisting of C1-C18 monovalent alkyl, C2-C18 monovalent alkoxyalkyl, C3-C18 monovalent cycloalkyl, C2-C18 monovalent oxocycloalkyl, C6-C18 monovalent aryl, C7-C18 monovalent aryl alkyl, C7-C18 monovalent alkylaryl, C7-C18 monovalent aryloxyalkyl, C12-C18 monovalent aryl ether group, C2-C18 monovalent heteroaryl, C3-C18 monovalent heteroaryl alkyl, C3-C18 monovalent alkyl heteroaryl, and C1-C18 monovalent silyl, and when R₂ and R₃ both are hydrogen atoms, R₁ is not hydroxyl;
optionally, R₂ and R₃ can further bond with each other to form a ring structure, R₄ and R₅ can further bond with each other to form a ring structure, R₆ and R₇ can further bond with each other to form a ring structure, R₈ and R₉ can further bond with each other to form a ring structure, R₁₀ and R₁₁ can further bond with each other to form a ring structure, R₁₂ and R₁₃ can further bond with each other to form a ring structure, R₁₄ and R₁₅ can further bond with each other to form a ring structure, R₁₆ and R₁₇ can further bond with each other to form a ring structure, R₁₈ and R₁₉ can further bond with each other to form a ring structure, and R₂₀ and R₂₁ can further bond with each other to form a ring structure;
L₁ represents an oxygen atom or at least one selected from the group consisting of C1-C18 divalent alkyl, C1-C18 oxodialkyl, C6-C18 divalent cycloalkyl, C6-C18 divalent oxo cycloalkyl, C6-C18 divalent aryl, C7-C18 divalent arylalkyl, C7-C18 divalent alkyl aryl, C6-C18 divalent aryloxy, C7-C18 divalent aryloxyalkyl, C12-C18 divalent aryl ether group, C2-C18 divalent heteroaryl, C3-C18 divalent heteroaryl alkyl, and C3-C18 divalent alkyl heteroaryl;
L₂ represents at least one selected from the group consisting of C1-C18 trivalent alkyl, C1-C18 oxo trivalent alkyl, C6-C18 trivalent cycloalkyl, C6-C18 trivalent oxocycloalkyl, C6-C18 trivalent aryl, C7-C18 trivalent arylalkyl, C7-C18 trivalent alkylaryl, C6-C18 trivalent aryloxy, C7-C18 trivalent aryloxyalkyl, C12-C18 trivalent aryl ether group, C3-C18 trivalent heteroaryl, C3-C18 trivalent heteroaryl alkyl, and C3-C18 trivalent alkyl heteroaryl; and
L₃ represents at least one selected from the group consisting of C1-C18 tetravalent alkyl, C1-C18 oxo tetravalent alkyl, C6-C18 tetravalent cycloalkyl, C6-C18 tetravalent oxo cycloalkyl, C6-C18 tetravalent aryl, C7-C18 tetravalent arylalkyl, C7-C18 tetravalent alkylaryl, C7-C18 tetravalent aryloxyalkyl, C12-C18 tetravalent aryl ether group, C4-C18 tetravalent heteroaryl, C4-C18 tetravalent heteroaryl, and C4-C18 tetravalent alkyl heteroaryl.

2. The secondary battery according to claim 1, wherein the first additive satisfies at least one of the following conditions from (1) to (9):
(1) R₂ and R₃ bond with each other to form a ring structure;
(2) at least one of R₁ to R₃ represents C3-C18 monovalent cycloalkyl, C2-C18 monovalent oxocycloalkyl, C6-C18 monovalent aryl, C7-C18 monovalent arylalkyl, C7-C18 monovalent alkylaryl, C7-C18 monovalent aryloxyalkyl, C12-C18 monovalent aryl ether group, C2-C18 monovalent heteroaryl, C3-C18 monovalent heteroarylalkyl, or C3-C18 monovalent alkyl heteroaryl;
(3) R₁ to R3 independently represent C6-C18 monovalent aryl, C7-C18 monovalent arylalkyl, C7-C18 monovalent alkylaryl, C7-C18 monovalent aryloxyalkyl, C12-C18 monovalent aryl ether group, C2-C18 monovalent heteroaryl group, C3-C18 monovalent heteroaryl group, or C3-C18 monovalent alkyl heteroaryl group;
(4) R₄ and R₅, and R₆ and R₇, at least one pair of them, bond with each other to form a ring structure;
(5) L₁ represents an oxygen atom, and R₄ to R₇ independently represent C6-C18 monovalent aryl, C7-C18 monovalent arylalkyl, C7-C18 monovalent alkylaryl, C7-C18 monovalent aryloxyalkyl, C12-C18 monovalent aryl ether group, C2-C18 monovalent heteroaryl, C3-C18 monovalent heteroarylalkyl, or C3-C18 monovalent alkyl heteroaryl;
(6) R₈ and R₉, R₁₀ and R₁₁, and R₁₂ and R₁₃, at least one pair of them, bond to form a ring structure;
(7) L₂ represents C6-C18 trivalent aryl, C7-C18 trivalent arylalkyl, C7-C18 trivalent alkylaryl, C6-C18 trivalent aryloxy, C7-C18 trivalent aryloxyalkyl, C12-C18 trivalent aryl ether group, C3-C18 trivalent heteroaryl group, C3-C18 trivalent heteroaryl alkyl, or C3-C18 trivalent alkyl heteroaryl;
(8) R₁₄ and R₁₅, R₁₆ and R₁₇, R₁₈ and R₁₉, R₂₀ and R₂₁, at least on pair of them, bond to form a ring structure; and
(9) L₃ represents C6-C18 tetravalent aryl, C7-C18 tetravalent arylalkyl, C7-C18 tetravalent alkylaryl, C7-C18 tetravalent aryloxyalkyl, C12-C18 tetravalent aryl ether group, C4-C18 tetravalent heteroaryl group, C4-C18 tetravalent heteroaryl, or C4-C18 tetravalent alkyl heteroaryl.

3. The secondary battery according to claim 1 or 2, wherein the first additive comprises at least one of the following compounds: 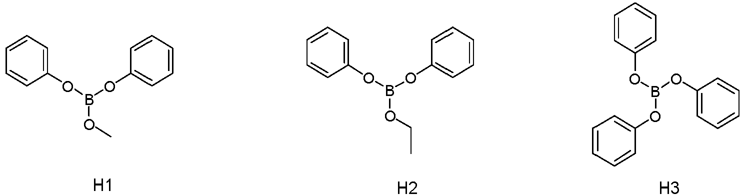 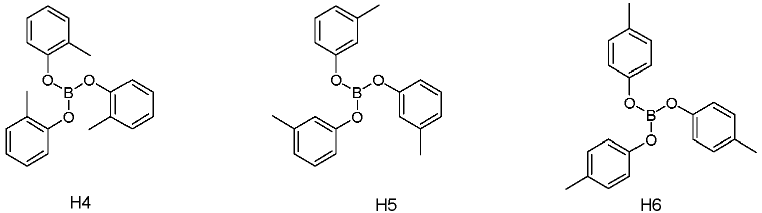 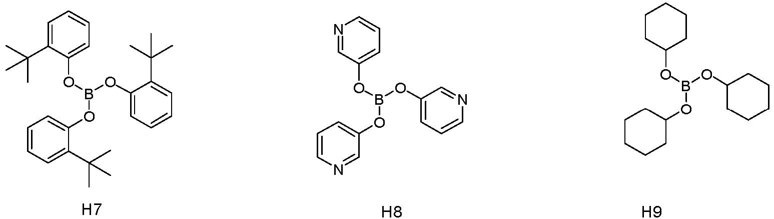 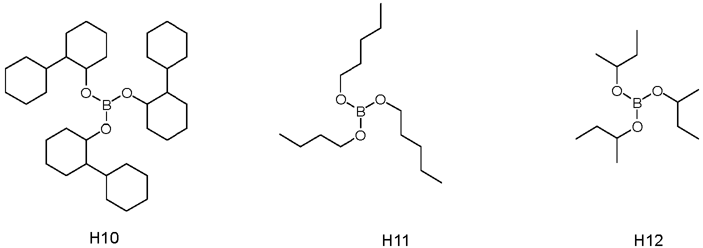 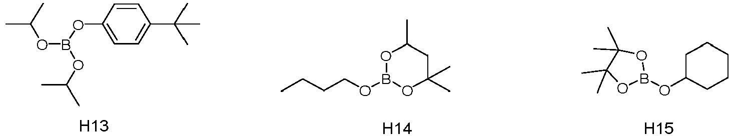 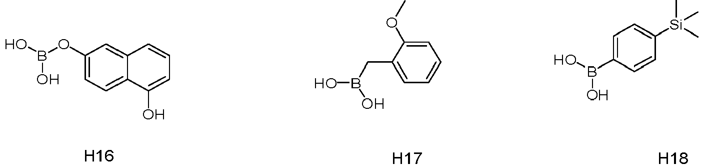 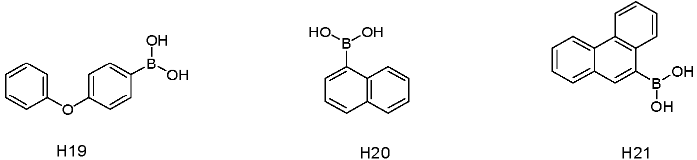 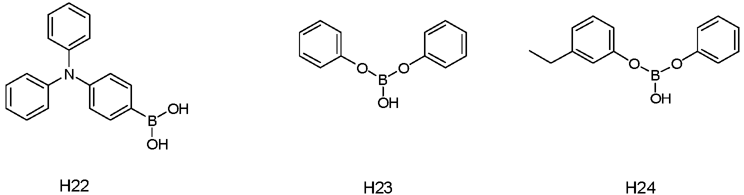 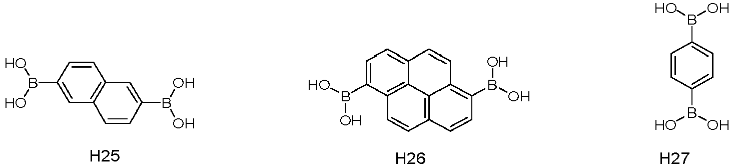 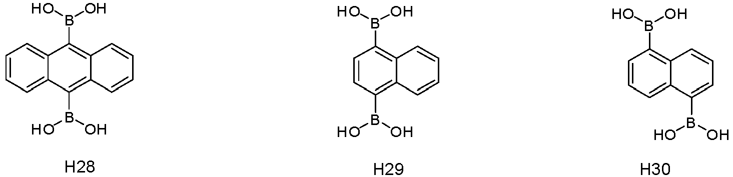 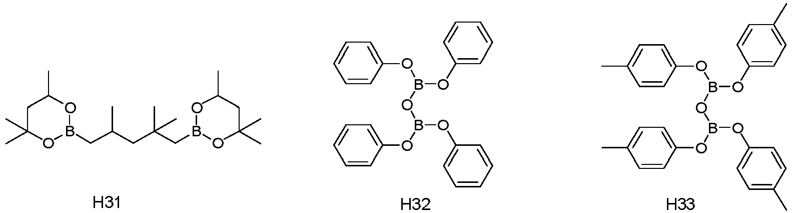 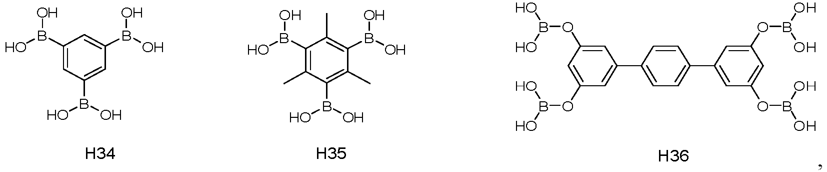
optionally, the first additive comprises at least one selected from the group consisting of H1 to H10, H13 to H15, H31 to H33, and
more optionally, the first additive comprises at least one selected from the group consisting of H1 to H8 and H32 to H33.

4. The secondary battery according to any one of claims 1-3, wherein the non-aqueous electrolytic solution further comprises a second additive comprising one or more selected from the group consisting of sulfonic acid lactones, cyclic sulfate, lithium difluorophosphate, lithium difluorooxalate phosphate, and lithium difluorooxalate borate.

5. The secondary battery according to claim 4, wherein
the sulfonic acid lactones comprise at least one of the compounds as represented by Formula 2-A, 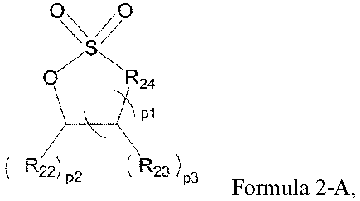
in which
p1 represents 1, 2, or 3, p2 represents 1 or 2, and p3 represents 1 or 2,
R₂₂, at each occurrence, independently represents a hydrogen atom, halogen atom, carboxylic ester group, sulfonic ester group, C1-C6 monovalent alkyl, C1-C6 monovalent haloalkyl, C1-C6 monovalent alkoxy, C1-C6 monovalent haloalkoxy, C2-C6 monovalent alkenyl, or a chain structure or a ring structure formed from more than one of the aforementioned groups linked together with single bonds, and when R₂₂ represents the ring structure, it is bonded to the ring of Formula 2-A via a single bond or shares a carbon atom with the ring of Formula 2-A to form a spiral-ring compound,
R₂₃, at each occurrence, independently represents a hydrogen atom, halogen atom, carboxylic ester group, sulfonic ester group, C1-C6 monovalent alkyl, C1-C6 monovalent haloalkyl, C1-C6 monovalent alkoxy, C1-C6 monovalent haloalkoxy, C2-C6 monovalent alkenyl, or a chain structure or a ring structure formed from more than one of the aforementioned groups linked together with single bonds, and when R₂₃ represents the ring structure, it is bonded to the ring of Formula 2-A via a single bond or shares a carbon atom with the ring of Formula 2-A to form a spiral-ring compound,
R₂₄ represents carbonyl or C(Y¹)₂, wherein Y₁ at each occurrence independently represents a hydrogen atom, halogen atom, carboxylic ester group, sulfonic ester group, C1-C6 monovalent alkyl group, C1-C6 monovalent haloalkyl group, C1-C6 monovalent alkoxy group, C1-C6 monovalent haloalkoxy group, C2-C6 monovalent alkenyl group, C6-C12 monovalent aryl group, or a combination thereof, and
optionally, R₂₂ and R₂₃ can further bond with each other to form a ring structure,
optionally, the sulfonic acid lactone comprises at least one of the following compounds: 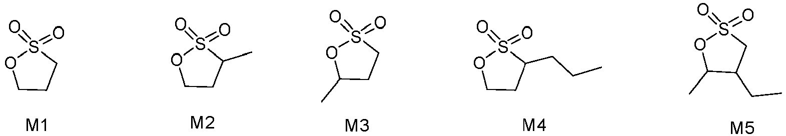 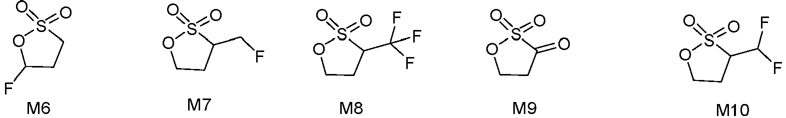 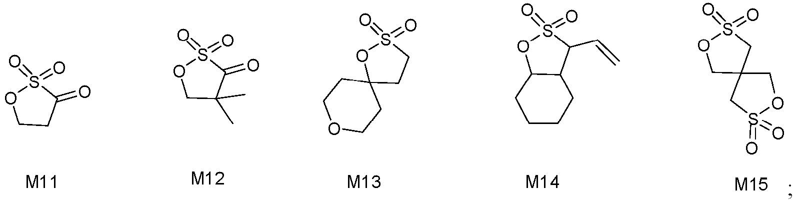 and/or,
the cyclic sulfate comprises at least one of the compounds represented by Formula 2-B, 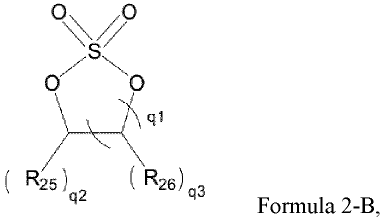
in which
q1 represents 1, 2, or 3, q2 represents 1 or 2, q3 represents 1 or 2,
R₂₅, at each occurrence, independently represents a hydrogen atom, halogen atom, carbonyl oxygen atom, carboxylate ester group, sulfate ester group, C1-C6 monovalent alkyl, C1-C6 monovalent haloalkyl, C1-C6 monovalent alkoxy, C1-C6 monovalent haloalkoxy, C2-C6 monovalent alkenyl, or a chain structure or a ring structure formed from more than one of the aforementioned groups linked together with single bonds, and when R₂₅ represents the ring structure, it is bonded to the ring of Formula 2-B via a single bond or shares a carbon atom with the ring of Formula 2-B to form a spiral-ring compound,
R₂₆, at each occurrence, independently represents a hydrogen atom, halogen atom, carboxylic ester group, sulfate group, C1-C6 monovalent alkyl group, C1-C6 monovalent haloalkyl, C1-C6 monovalent alkoxy, C1-C6 monovalent haloalkoxy, C2-C6 monovalent alkenyl, or a chain structure or a ring structure formed from more than one of the aforementioned groups linked together with single bonds, and when R₂₆ represents the ring structure, it is bonded to the ring of Formula 2-B via a single bond or shares a carbon atom with the ring of Formula 2-B to form a spiral-ring compound,
optionally, R₂₅ and R₂₆ can further bond with each other to form a ring structure,
optionally, the cyclic sulfate comprises at least one of the following compounds:

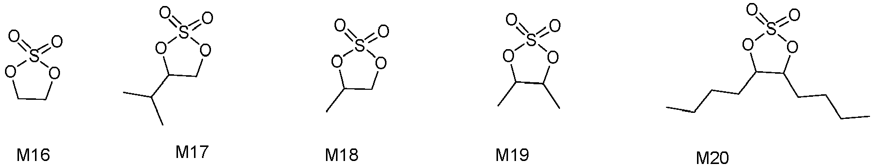

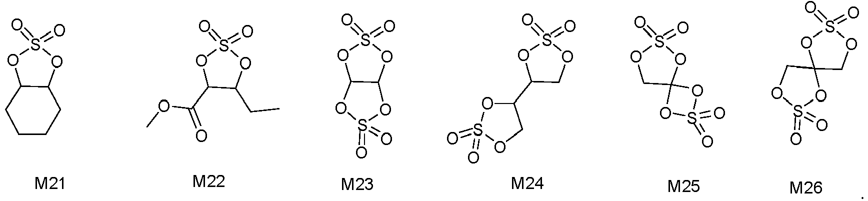

6. The secondary battery according to any one of claims 1-5, wherein,
the first additive has an amount of W1 % by weight of from 0.01 to 20, optionally from 0.1 to 10, and more optionally from 0.3 to 5, based on the total weight of the non-aqueous electrolytic solution; and/or,
the second additive an amount of W2 % by weight of from 0.01 to 20, optionally from 0.1 to 10, and more optionally from 0.3 to 5, based on the total weight of the non-aqueous electrolytic solution; and/or,
the first coating layer has a coating amount of C1 % by weight of from greater than 0 to less than or equal to 7, and optionally from 4 to 5.6, based on the weight of the core; and/or,
the second coating layer has a coating amount of C2 % by weight of from greater than 0 to less than or equal to 6, and optionally from 3 to 5, based on the weight of the core.

7. The secondary battery according to claim 6, wherein
W1/W2 is from 0.01 to 20, and optionally from 0.01 to 10; and/or,
(W1+W2)/(C1+C2) is from 0.001 to 2, and optionally from 0.01 to 1.

8. The secondary battery according to any one of claims 1-7, wherein the non-aqueous electrolytic solution further comprises a third additive comprising one or more selected from the group consisting of cyclic carbonate compounds having unsaturated bond(s), halogenated cyclic carbonate compounds, nitrile compounds, phosphoronitrile compounds, aromatic and halogenated aromatic compounds, isocyanate compounds, anhydride compounds, phosphite compounds, phosphate ester compounds, sulfite compounds or dimethyl disulfonate compounds.

9. The secondary battery according to any one of claims 1-8, wherein,
in the core, y and 1-y has a ratio of from 1:10 to 10:1, and optionally from 1:4 to 1:1; and/or,
in the core, z and 1-z has a ratio of from 1:9 to 1:999, and optionally from 1:499 to 1:249; and/or
the A is at least two selected from Fe, Ti, V, Ni, Co, and Mg.

10. The secondary battery according to any one of claims 1-9, wherein the first coating layer satisfies at least one of the following conditions from (1) to (4):
(1) the first coating layer has a interplanar spacing of the phosphate of 0.345-0.358nm, and has a included angle in the crystal direction (111) of 24.25°-26.45°;
(2) the first coating layer has a interplanar spacing of pyrophosphate of 0.293-0.326nm, and has a included angle of the crystal direction (111) of 26.41°-32.57°;
(3) the first coating layer has a weight ratio of pyrophosphate to phosphate of from 1:3 to 3:1, and optionally from 1:3 to 1:1; and
(4) The the first coating layer has a crystallinity of the pyrophosphate and phosphate independently of 10% to 100%, and optionally from 50% to 100%.

11. The secondary battery according to any one of claims 1-10, wherein the positive electrode active material satisfies at least one of the following conditions from (1) to (5):
(1) the positive electrode active material has a Li/Mn antisite defect concentration of 4% or less, and optionally 2% or less;
(2) the positive electrode active material has a lattice change rate of 6% or less, and optionally 4% or less;
(3) the positive electrode active material has a surface oxygen valence of -1.88 or less, and optionally from -1.98 to -1.88;
(4) the positive electrode active material has a compaction density under 3 tons of 2.0g/cm³ or more, and optionally 2.2g/cm³ or above; and
(5) the positive electrode active material has a specific surface area of B m²/g, with B being from 7 to 18, and optionally from 10 to 15.

12. A battery module, comprising a secondary battery according to any one of claims 1-11.

13. A battery pack, comprising the battery module according to claim 12.

14. A powder device, comprising at least one of the secondary battery according to any one of claims 1-11, the battery module according to claim 12, or the battery pack according to claim 13.
